# EUROPEAN PATENT SPECIFICATION

(11) **EP 4 248 866 B1**
(45) Date of publication and mention of the grant of the patent: **09.12.2026**
(21) Application number: 23190032.5
(22) Date of filing: 24.03.2011
(51) Int. Cl.: A61B 5/157

(54) **MEDICAL DEVICE INSERTERS**
EINSETZVORRICHTUNGEN FÜR MEDIZINISCHE VORRICHTUNGEN UND VERFAHREN ZUM EINSETZEN UND VERWENDEN VON MEDIZINISCHEN VORRICHTUNGEN
DISPOSITIFS D'INSERTION DE DISPOSITIFS MÉDICAUX ET PROCÉDÉS D'INSERTION ET D'UTILISATION DE DISPOSITIFS MÉDICAUX

(30) Priority: 24.03.2010 US 31724310 P; 17.05.2010 US 34556210 P; 02.07.2010 US 36137410 P; 08.11.2010 US 41126210 P
(43) Date of publication of application: 27.09.2023
(62) Document number(s) of the earlier application(s) in accordance with Art. 76 EPC: 23166498.8 / 4 245 220; 22169853.3 / 4 066 731; 20195922.8 / 3 766 408; 19184881.1 / 3 622 883; 16176370.1 / 3 123 934; 11760268.0 / 2 549 918
(73) Proprietor: Abbott Diabetes Care, Inc., Alameda, CA 94502 (US)
(72) Inventor: DONNAY, Manuel Luis Miguel, San Francisco, CA 94133 (US); PACE, Louis, San Carlos, CA 94070 (US); ROBINSON, Peter, Alamo, CA 94507 (US); NGUYEN, Tuan, San Jose, CA 95138 (US)
(74) Representative: Mathys & Squire

(56) References cited:
- EP-A1- 1 704 889
- WO-A1-2009/039013
- WO-A2-2006/110742
- US-A- 5 536 259
- US-A1- 2004 072 357
- US-A1- 2005 101 912
- US-A1- 2006 161 194
- US-A1- 2007 060 814
- US-A1- 2008 064 941
- US-A1- 2008 194 935

## Description

### BACKGROUND

The detection and/or monitoring of glucose levels or other analytes, such as lactate, oxygen, AIC, or the like, in certain individuals is vitally important to their health. For example, the monitoring of glucose is particularly important to individuals with diabetes. Diabetics generally monitor glucose levels to determine if their glucose levels are being maintained within a clinically safe range, and may also use this information to determine if and/or when insulin is needed to reduce glucose levels in their bodies or when additional glucose is needed to raise the level of glucose in their bodies.

Growing clinical data demonstrates a strong correlation between the frequency of glucose monitoring and glycemic control. Despite such correlation, many individuals diagnosed with a diabetic condition do not monitor their glucose levels as frequently as they should due to a combination of factors including convenience, testing discretion, pain associated with glucose testing, and cost.

Devices have been developed for the automatic monitoring of analyte(s), such as glucose, in bodily fluid such as in the blood stream or in interstitial fluid ("ISF"), or other biological fluid. Some of these analyte measuring devices are configured so that at least a portion of the devices are positioned below a skin surface of a user, e.g., in a blood vessel or in the subcutaneous tissue of a user, so that the monitoring is accomplished in vivo.

With the continued development of analyte monitoring devices and systems, there is a need for such analyte monitoring devices, systems, and methods, as well as for processes for manufacturing analyte monitoring devices and systems that are cost effective, convenient, and with reduced pain, provide discreet monitoring to encourage frequent analyte monitoring to improve glycemic control.

WO 2009/039013 A1, US 2005/101912 A1, WO 2006/110742 A2, US 2008/064941 A1, EP 1704889A1, and US 2008/194935 A1 all disclose methods of insertion and/or inserters for inserting devices into the skin of a subject.

### SUMMARY

The scope of the invention is defined by the appended independent claims. The aspects and/or embodiments and/or examples disclosed in the following description but that are not covered by the appended claims are considered as not being part of the present invention and are disclosed by way of example only. **In** certain embodiments, there is provided, an apparatus for inserting a medical device into the skin of a subject is provided, which includes a sheath defining a distal surface for placement on the skin of the subject; a device support movable between a proximal and distal position, and adapted to support the medical device; a sharp support movable between a proximal and a distal position and adapted to support a sharp for inserting the medical device into the skin of the subject and extending through a portion of said device support, the device support comprising a first engagement member for releasably coupling the sharp support to the device support and a second engagement member for engaging the medical device; a handle movable between a proximal position and a distal position relative to the sheath and adapted to urge the device support and the sharp support from a proximal to a distal position to insert the sharp into the skin of the subject; and a driver for advancing the sharp support towards the proximal position when the sharp support reaches the distal position.

**In** some embodiments, the handle and sheath define an interlocking configuration which prevents relative movement of the handle with respect to the sheath which is overcome by a force applied to the handle. **In** some embodiment, the second engagement member includes one or more movable arms for engaging the device. The one or more movable arms are normally biased in a position spaced apart from the medical device in some embodiments. The one or more movable arms may be maintained in engagement with the medical device when the device support is in the proximal position. **In** some embodiments, the one or more movable arms return to the configuration space apart from the medical device when the device support is in the distal position.

**In** some embodiments, the engagement member is released from the sharp support when the device support reaches a distal position. **In** some embodiments, the engagement member is maintained in engagement with the device support by a portion of the sheath.

In some embodiments, a stop is provided to maintain the device support in the proximal position.

In some embodiments, the handle includes a button disposed within an outer housing. The handle may be flush with the top of the outer housing in an initial configuration when the medical device is supported in the device support, and the handle may protrude above the outer housing after the medical device is released from the device support.

In some embodiments, the medical device is an analyte sensor.

A method for using a medical device is provided which includes providing an apparatus comprising a sheath defining a distal surface, a device support adapted to support the medical device, a sharp support adapted to support a sharp extending through a portion of said device support, a handle movable relative to the sheath, and a driver for displacing the sharp support; disposing the distal surface of the sheath on the skin of the subject; and displacing the handle in a first longitudinal direction; displacing the sharp support in the first longitudinal direction, the sharp support displacing the sharp and the medical device. The method further includes inserting the sharp into the skin of the subject; delivering the medical device to the subject; releasing the driver; and displacing the sharp in the second longitudinal direction by the driver.

In some embodiments, the method further includes locking at least a portion of the sheath to the handle.

These and other features, objects, and advantages of the disclosed subject matter will become apparent to those persons skilled in the art upon reading the detailed description as more fully described below.

### BRIEF DESCRIPTION OF THE DRAWINGS

A detailed description of various aspects, features, and embodiments of the subject matter described herein is provided with reference to the accompanying drawings, which are briefly described below. The drawings are illustrative and are not necessarily drawn to scale, with some components and features being exaggerated for clarity. The drawings illustrate various aspects and features of the present subject matter and may illustrate one or more embodiment(s) or example(s) of the present subject matter in whole or in part.
**FIGURE 1** illustrates analyte monitoring system for real time analyte (e.g., glucose) measurement, data acquisition and/or processing in certain embodiments
**FIGURE 2** is a view of an electrochemical sensor in accordance with an embodiment of the disclosed subject matter;
**FIGURE 3** is a view of the electrochemical sensor of FIGURE 2 in a folded configuration in accordance with the disclosed subject matter;
**FIGURE 4** is a perspective view of an embodiment of an inserter in accordance with one embodiment of the disclosed subject matter;
**FIGURES 5-6** are perspective views of the inserter of FIGURE 4 in accordance with the disclosed subject matter;
**FIGURES 7-8** are sectional, perspective views of the inserter of FIGURE 4 in accordance with the disclosed subject matter;
**FIGURES 9-10** are schematic views of a needle hub in accordance with one embodiment of the disclosed subject matter;
**FIGURE 11** is a distal end view of a sharp in accordance with one embodiment of the disclosed subject matter;
**FIGURE 12** is a side view of a sharp in accordance with one embodiment of the disclosed subject matter;
**FIGURE 13** is a side view of a sharp in accordance with one embodiment of the disclosed subject matter;
**FIGURE 14** is a perspective view with parts separated of an inserter in accordance with one embodiment of the disclosed subject matter;
FIGURES 14A**-B** are top views of a sharp in accordance with one embodiment of the disclosed subject matter;
**FIGURE 14C** is a side view of a sharp in accordance with one embodiment of the disclosed subject matter;
**FIGURE 15** is a schematic view of an alternate embodiment for forming a sharp to be used in an inserter in accordance with one embodiment of the disclosed subject matter;
**FIGURE 16** is a perspective view of an inserter in accordance with one embodiment of the disclosed subject matter;
**FIGURE 17** is a perspective view with parts separated of an inserter in accordance with one embodiment of the disclosed subject matter;
**FIGURE 17A** is an enlarged perspective view of a portion of an inserter in accordance with one embodiment of the disclosed subject matter.
**FIGURE 18** is an enlarged sectional view with parts separated of an inserter in accordance with one embodiment of the disclosed subject matter;
**FIGURES 19-21** depict an alternative method for retaining a sharp and sensor within the on body housing;
**FIGURE 22** is a sectional, perspective views of the inserter of FIGURE 4 in accordance with the disclosed subject matter;
**FIGURES 23-24** are perspective views of the inserter of FIGURE 4 in accordance with the disclosed subject matter;
**FIGURES 25-26** are perspective views of another embodiment of an inserter in accordance with the disclosed subject matter;
**FIGURES 27-32** are perspective views of components of the inserter of FIGURE 25 in accordance with the disclosed subject matter;
**FIGURE 33** is a sectional view of the inserter of FIGURE 25 in accordance with the disclosed subject matter;
**FIGURE 34** is a sectional, perspective view of the inserter of Figure 25 in accordance with the disclosed subject matter;
**FIGURE 35** is a perspective view of the inserter of FIGURE 25 in accordance with the disclosed subject matter;
**FIGURES 36-37** are sectional, perspective views of the inserter of FIGURE 25 in accordance with the disclosed subject matter;
**FIGURES 38-39** are perspective views of the inserter of FIGURE 25 in accordance with the disclosed subject matter;
**FIGURES 40-41** are perspective views of another embodiment of an inserter in accordance with the disclosed subject matter;
**FIGURES 42-46** are perspective views of components of the inserter of FIGURE 40 in accordance with the disclosed subject matter;
**FIGURE 47** is a sectional views of the inserter of FIGURE 40 in accordance with the disclosed subject matter;
**FIGURES 48-50** are sectional, perspective views of the inserter of Figure 40 in accordance with the disclosed subject matter;
**FIGURE 51** is a cross-sectional view of another inserter in accordance with the disclosed subject matter;
**FIGURE 52** is an exploded perspective view of the inserter of FIGURE 51 in accordance with the disclosed subject matter;
**FIGURES 53 and 54** are side views of the inserter of FIGURE 51 showing the assembly of various components in accordance with the disclosed subject matter;
**FIGURES 55-60** are perspective views of the inserter of FIGURE 51 showing the assembly of various components in accordance with the disclosed subject matter;
**FIGURES 61-65** are cross-sectional views of the inserter of FIGURE 51 in accordance with the disclosed subject matter;
**FIGURES 66-68** illustrate a process for utilizing a sterilized versions of the inserter of FIGURE 51 in accordance with the disclosed subject matter;
**FIGURE 69-72** illustrates an alternate process for utilizing a sterilized versions of the inserter of FIGURE 51 in accordance with the disclosed subject matter;
**FIGURE 73** is a perspective view of another inserter in accordance with the disclosed subject matter;
**FIGURE 74** is a perspective view of a component of the inserter of FIGURE 73 in accordance with the disclosed subject matter;
**FIGURE 75** **is** a cross-sectional view of a component of the inserter of FIGURE 73 in accordance with the disclosed subject matter;
**FIGURES 76-80** are perspective views of a components of the inserter of FIGURE 73 in accordance with the disclosed subject matter;
**FIGURES 81-87** are cross-sectional views of the inserter of FIGURE 73 in accordance with the disclosed subject matter;
**FIGURES 88-90** illustrate a process for utilizing a sterilized versions of the inserter of FIGURE 73 in accordance with the disclosed subject matter;
**FIGURE 91** is a perspective view of another inserter in accordance with the disclosed subject matter;
**FIGURES 92-99** are additional views of the components of the inserter of FIGURE 91 in accordance with the disclosed subject matter;
**FIGURES 100-106** are cross-sectional views of the inserter of FIGURE 91 in accordance with the disclosed subject matter;
**FIGURES 107-108** are views of an alternate embodiment of the inserter of FIGURE 91 in accordance with the disclosed subject matter; and
**FIGURES 109-134** are views of an alternate embodiment of the inserter of FIGURE 73 in accordance with the disclosed subject matter.
**FIGURES 135-136** illustrate bottom and top views, respectively, of the medical device carrier in accordance with the disclosed subject matter.
**FIGURES 137-138** illustrate the sheath component of an inserter in accordance with the disclosed subject matter.

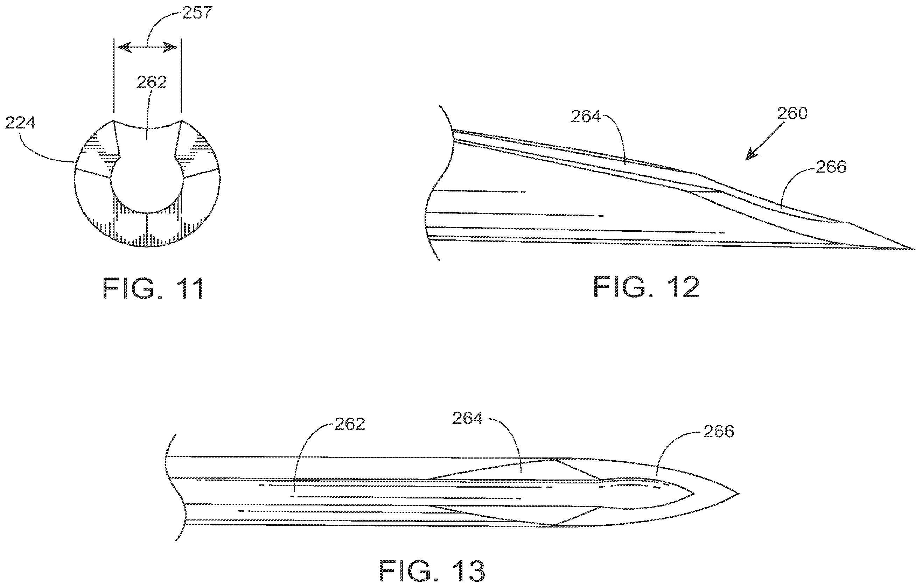

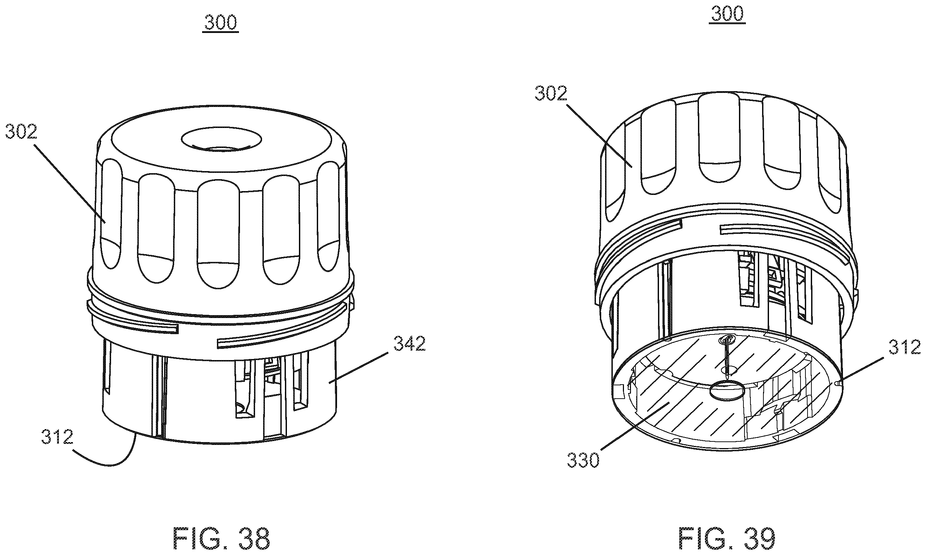

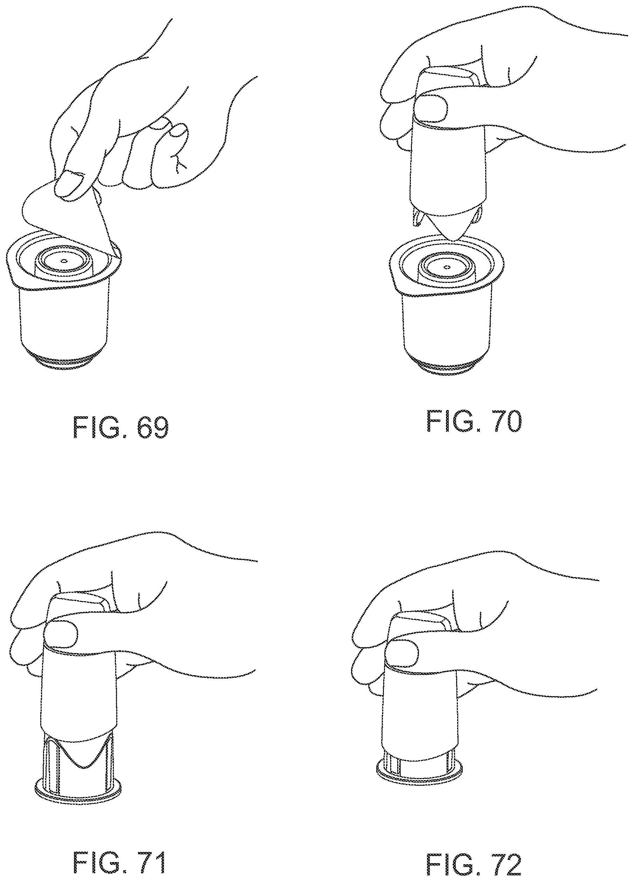

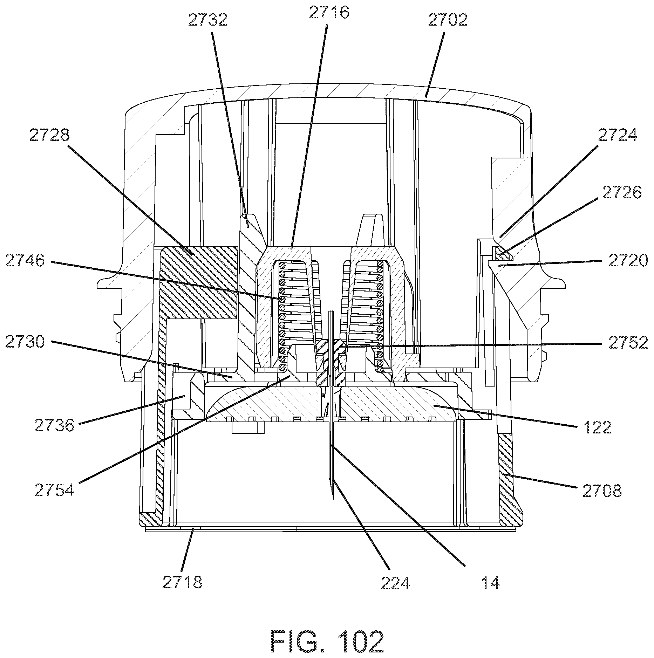

**FIGURES 13 -140** illustrate, in cross section, the progressive advancement of the on body housing in accordance with the disclosed subject matter.

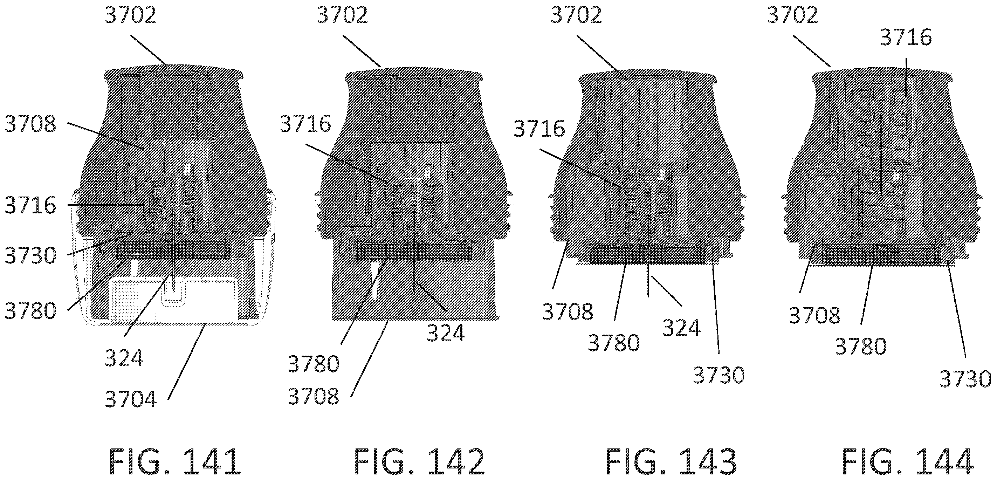

**FIGURES 141-144** illustrate the advancement of the on body housing within an inserter in accordance with the disclosed subject matter.

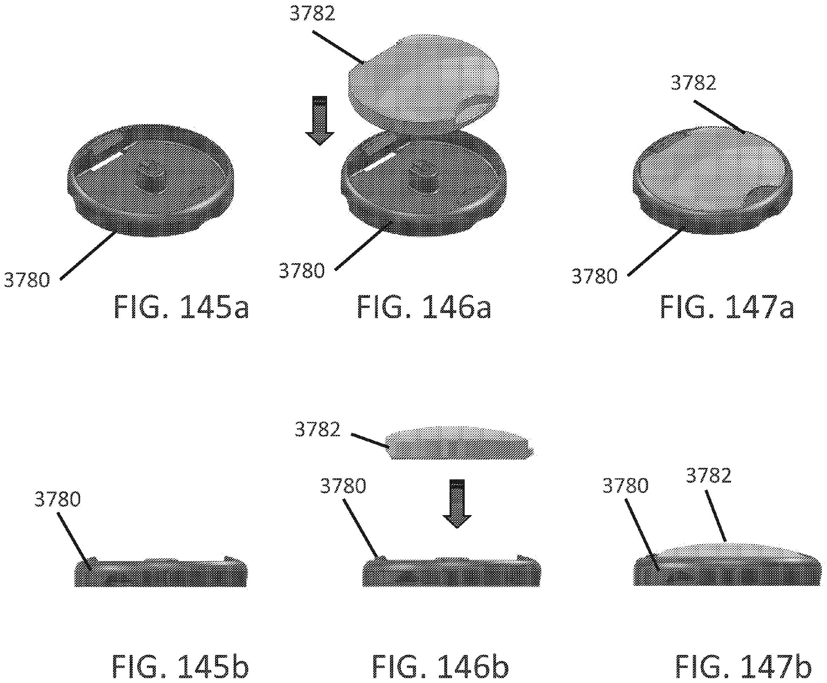

**FIGURES 145a-147b** illustrate the attachment of a two piece on body housing in accordance with the disclosed subject matter.

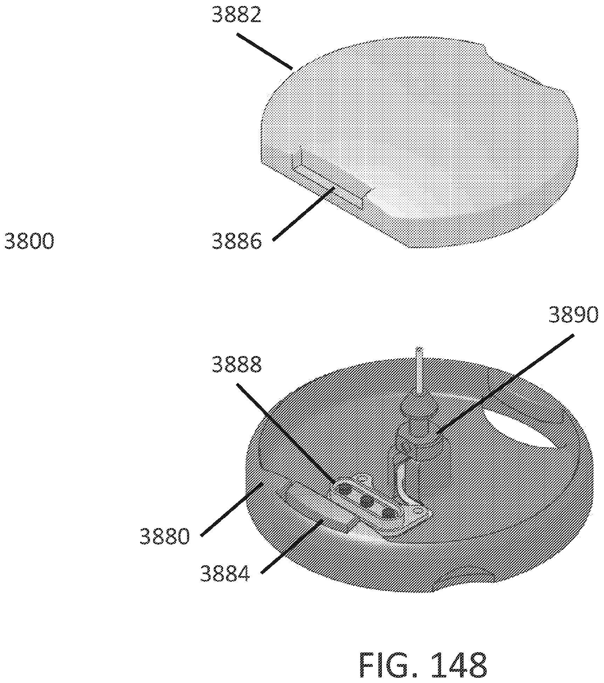

**FIGURE 148** illustrates a two-piece on body housing in accordance with the disclosed subject matter.

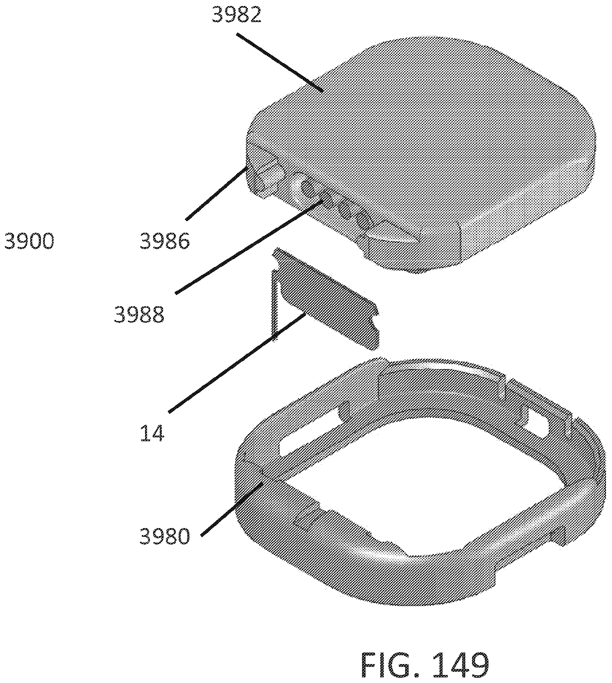

**FIGURE 149** illustrates another two-piece on body housing in accordance with the disclosed subject matter.

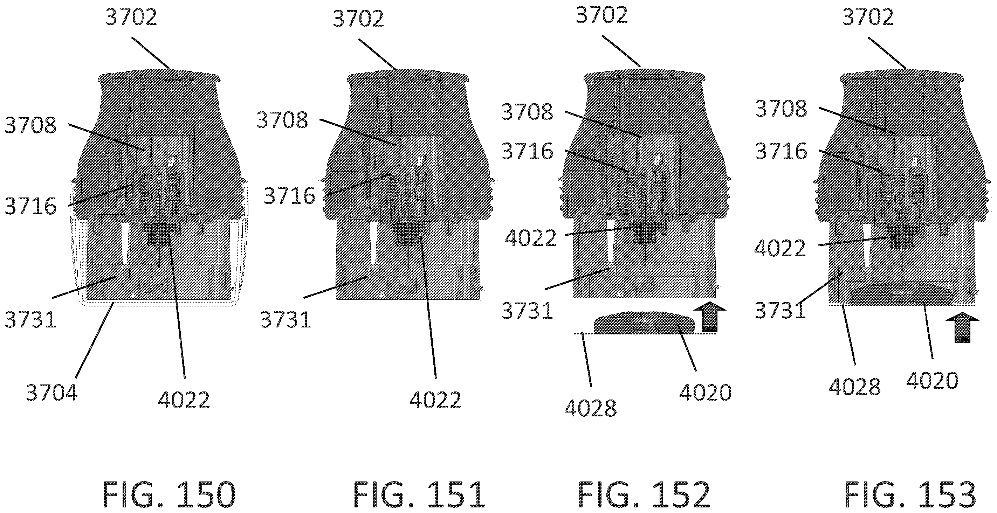

**FIGURES 150-156** illustrate the advancement of a two piece on body housing in accordance with the disclosed subject matter.

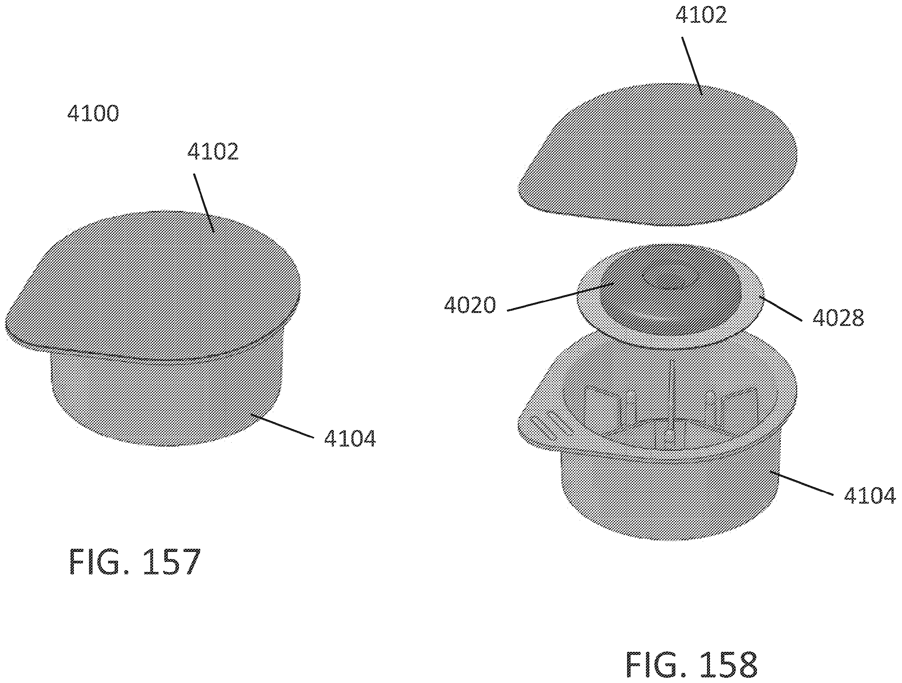

**FIGURES 157-158** illustrate the assembly of the two piece on body housing in accordance with the disclosed subject matter.

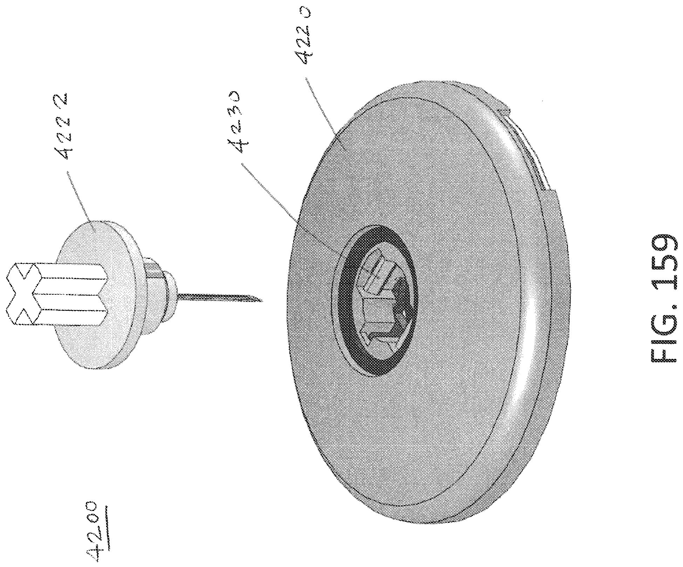

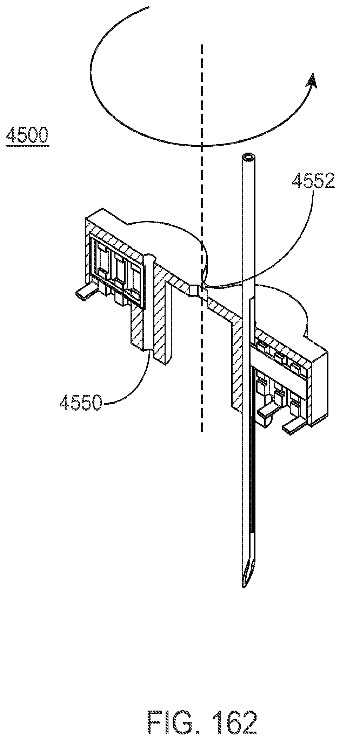

**FIGURES 159-164** illustrate two piece on body housings in accordance with the disclosed subject matter.

### DETAILED DESCRIPTION OF THE EMBODIMENTS

A detailed description of the disclosure is provided herein. It should be understood, in connection with the following description, that the subject matter is not limited to particular embodiments described, as the particular embodiments of the subject matter may, of course, vary. It is also to be understood that the terminology used herein is for the purpose of describing particular embodiments only, and is not intended to be limiting, since the scope of the disclosed subject matter will be limited only by the appended claims.

Where a range of values is provided, it is understood that each intervening value between the upper and lower limit of that range, and any other stated or intervening value in that stated range, is encompassed within the disclosed subject matter. Every range stated is also intended to specifically disclose each and every "subrange" of the stated range. That is, each and every range smaller than the outside range specified by the outside upper and outside lower limits given for a range, whose upper and lower limits are within the range from said outside lower limit to said outside upper limit (unless the context clearly dictates otherwise), is also to be understood as encompassed within the disclosed subject matter, subject to any specifically excluded range or limit within the stated range. Where a range is stated by specifying one or both of an upper and lower limit, ranges excluding either or both of those stated limits, or including one or both of them, are also encompassed within the disclosed subject matter, regardless of whether or not words such as "from," "to," "through," or "including" are or are not used in describing the range.

Unless defined otherwise, all technical and scientific terms used herein have the same meaning as commonly understood by one of ordinary skill in the art to which this disclosed subject matter belongs. Although any methods and materials similar or equivalent to those described herein can also be used in the practice or testing of the present disclosed subject matter, this disclosure may specifically mention certain exemplary methods and materials.

The publications discussed herein are provided solely for their disclosure prior to the filing date of the present application. Nothing herein is to be construed as an admission that the present disclosed subject matter is not entitled to antedate such publication by virtue of prior invention. Further, the dates of publication provided may be different from the actual publication dates, which may need to be independently confirmed.

As used herein and in the appended claims, the singular forms "a," "an," and "the" include plural referents unless the context clearly dictates otherwise.

Nothing contained in the Abstract or the Summary should be understood as limiting the scope of the disclosure. The Abstract and the Summary are provided for bibliographic and convenience purposes and due to their formats and purposes should not be considered comprehensive.

As will be apparent to those of skill in the art upon reading this disclosure, each of the individual embodiments described and illustrated herein has discrete components and features which may be readily separated from or combined with the features of any of the other several embodiments without departing from the scope of the present disclosed subject matter. Any recited method can be carried out in the order of events recited, or in any other order which is logically possible.

Reference to a singular item includes the possibility that there are plural of the same item present. When two or more items (for example, elements or processes) are referenced by an alternative "or," this indicates that either could be present separately or any combination of them could be present together except where the presence of one necessarily excludes the other or others.

Generally, embodiments of the present disclosure relate to apparatus for inserting a medical device at least partially into the skin of the patient. Some embodiments relate to in vivo methods and devices for detecting at least one analyte such as glucose in body fluid. Accordingly, embodiments include in vivo analyte sensors configured so that at least a portion of the sensor is positioned in the body of a user (e.g., within the ISF), to obtain information about at least one analyte of the body, e.g., transcutaneously positioned in user's body. In certain embodiments, an in vivo analyte sensor is coupled to an electronics unit that is maintained on the body of the user to process information obtained from the sensor.

In certain embodiments, analyte information is communicated from a first device such as an on body electronics unit to a second device which may include user interface features, including a display, and/or the like. Information may be communicated from the first device to the second device automatically and/or continuously when the analyte information is available, or may not be communicated automatically and/or continuously, but rather stored or logged in a memory of the first device. Accordingly, in many embodiments of the system, analyte information derived by the sensor/on body electronics (for example, on body electronics) is made available in a user-usable or viewable form only when queried by the user such that the timing of data communication is selected by the user. In some embodiments, the display of information is selected by the user, while the timing of data communication is not.

In this manner, analyte information is only provided or evident to a user (provided at a user interface device) in some embodiments when desired by the user even though an in vivo analyte sensor automatically and/or continuously monitors the analyte level in vivo, i.e., the sensor automatically monitors analyte such as glucose on a predefined time interval over its usage life. For example, an analyte sensor may be positioned in vivo and coupled to on body electronics for a given sensing period, e.g., about 14 days. In certain embodiments, the sensor-derived analyte information is automatically communicated from the sensor electronics assembly to a remote monitor device or display device for output to a user throughout the 14 day period according to a schedule programmed at the on body electronics (e.g., about every 1 minute or about every 5 minutes or about every 10 minutes, or the like). In certain embodiments, sensor-derived analyte information is only communicated from the sensor electronics assembly to a remote monitor device or display device at user-determined times, e.g., whenever a user decides to check analyte information. At such times, a communications system is activated and sensor-derived information is then sent from the on body electronics to the remote device or display device.

In still other embodiments, the information may be communicated from the first device to the second device automatically and/or continuously when the analyte information is available, and the second device stores or logs the received information without presenting or outputting the information to the user. In such embodiments, the information is received by the second device from the first device when the information becomes available (e.g., when the sensor detects the analyte level according to a time schedule). However, the received information is initially stored in the second device and only output to a user interface or an output component of the second device (e.g., display) upon detection of a request for the information on the second device.

Accordingly, in certain embodiments an inserter as described herein is used to place a sensor electronics assembly on the body so that at least a portion of the in vivo sensor is in contact with bodily fluid such as ISF. Once the sensor is electrically coupled to the electronics unit, sensor derived analyte information may be communicated from the on body electronics to a display device on-demand by powering on the display device (or it may be continually powered), and executing a software algorithm stored in and accessed from a memory of the display device, to generate one or more request commands, control signal or data packet to send to the on body electronics. The software algorithm executed under, for example, the control of the microprocessor or application specific integrated circuit (ASIC) of the display device may include routines to detect the position of the on body electronics relative to the display device to initiate the transmission of the generated request command, control signal and/or data packet.

Display devices may also include programming stored in memory for execution by one or more microprocessors and/or ASICs to generate and transmit the one or more request command, control signal or data packet to send to the on body electronics in response to a user activation of an input mechanism on the display device such as depressing a button on the display device, triggering a soft button associated with the data communication function, and so on. The input mechanism may be alternatively or additionally provided on or in the on body electronics which may be configured for user activation. In certain embodiments, voice commands or audible signals may be used to prompt or instruct the microprocessor or ASIC to execute the software routine(s) stored in the memory to generate and transmit the one or more request command, control signal or data packet to the on body device. In the embodiments that are voice activated or responsive to voice commands or audible signals, on body electronics and/or display device includes a microphone, a speaker, and processing routines stored in the respective memories of the on body electronics and/or the display device to process the voice commands and/or audible signals. In certain embodiments, positioning the on body electronics and the display device within a predetermined distance (e.g., close proximity) relative to each other initiates one or more software routines stored in the memory of the display device to generate and transmit a request command, control signal or data packet.

Different types and/or forms and/or amounts of information may be sent for each on demand reading, including but not limited to one or more of current analyte level information (i.e., real time or the most recently obtained analyte level information temporally corresponding to the time the reading is initiated), rate of change of an analyte over a predetermined time period, rate of the rate of change of an analyte (acceleration in the rate of change), historical analyte information corresponding to analyte information obtained prior to a given reading and stored in memory of the assembly. Some or all of real time, historical, rate of change, rate of rate of change (such as acceleration or deceleration) information may be sent to a display device for a given reading. In certain embodiments, the type and/or form and/or amount of information sent to a display device may be preprogrammed and/or unchangeable (e.g., preset at manufacturing), or may not be preprogrammed and/or unchangeable so that it may be selectable and/or changeable in the field one or more times (e.g., by activating a switch of the system, etc). Accordingly, in certain embodiments, for each on demand reading, a display device will output a current (real time) sensor-derived analyte value (e.g., in numerical format), a current rate of analyte change (e.g., in the form of an analyte rate indicator such as a arrow pointing in a direction to indicate the current rate), and analyte trend history data based on sensor readings acquired by and stored in memory of on body electronics (e.g., in the form of a graphical trace). Additionally, the on skin or sensor temperature reading or measurement associated with each on demand reading may be communicated from the on body electronics to the display device. The temperature reading or measurement, however, may not be output or displayed on the display device, but rather, used in conjunction with a software routine executed by the display device to correct or compensate the analyte measurement output to the user on the display device.

As described, embodiments include inserters for in vivo analyte sensors and on body electronics that together provide body wearable sensor electronics assemblies. In certain embodiments, in vivo analyte sensors are fully integrated with on body electronics (fixedly connected during manufacture), while in other embodiments they are separate but connectable post manufacture (e.g., before, during or after sensor insertion into a body). On body electronics may include an in vivo glucose sensor, electronics, battery, and antenna encased (except for the sensor portion that is for in vivo positioning) in a waterproof housing that includes or is attachable to an adhesive pad. In certain embodiments, the housing withstands immersion in about one meter of water for up to at least 30 minutes. In certain embodiments, the housing withstands continuous underwater contact, e.g., for longer than about 30 minutes, and continues to function properly according to its intended use, e.g., without water damage to the housing electronics where the housing is suitable for water submersion.

Embodiments include sensor insertion devices, which also may be referred to herein as sensor delivery units, or the like. Insertion devices may retain on body electronics assemblies completely in an interior compartment, i.e., an insertion device may be "pre-loaded" with on body electronics assemblies during the manufacturing process (e.g., on body electronics may be packaged in a sterile interior compartment of an insertion device). In such embodiments, insertion devices may form sensor assembly packages (including sterile packages) for pre-use or new on body electronics assemblies, and insertion devices configured to apply on body electronics assemblies to recipient bodies.

Embodiments include portable handheld display devices, as separate devices and spaced apart from an on body electronics assembly, that collect information from the assemblies and provide sensor derived analyte readings to users. Such devices may also be referred to as meters, readers, monitors, receivers, human interface devices, companions, or the like. Certain embodiments may include an integrated in vitro analyte meter. In certain embodiments, display devices include one or more wired or wireless communications ports such as USB, serial, parallel, or the like, configured to establish communication between a display device and another unit (e.g., on body electronics, power unit to recharge a battery, a PC, etc). For example, a display device communication port may enable charging a display device battery with a respective charging cable and/or data exchange between a display device and its compatible informatics software.

Compatible informatics software in certain embodiments include, for example, but not limited to stand alone or network connection enabled data management software program, resident or running on a display device, personal computer, a server terminal, for example, to perform data analysis, charting, data storage, data archiving and data communication as well as data synchronization. Informatics software in certain embodiments may also include software for executing field upgradable functions to upgrade firmware of a display device and/or on body electronics unit to upgrade the resident software on the display device and/or the on body electronics unit, e.g., with versions of firmware that include additional features and/or include software bugs or errors fixed, etc. Embodiments may include a haptic feedback feature such as a vibration motor or the like, configured so that corresponding notifications (e.g., a successful on-demand reading received at a display device), may be delivered in the form of haptic feedback.

Embodiments include programming embedded on a computer readable medium, i.e., computer-based application software (may also be referred to herein as informatics software or programming or the like) that processes analyte information obtained from the system and/or user self-reported data. Application software may be installed on a host computer such as a mobile telephone, PC, an Internet-enabled human interface device such as an Internet-enabled phone, personal digital assistant, or the like, by a display device or an on body electronics unit. Informatics programming may transform data acquired and stored on a display device or on body unit for use by a user.

Embodiments of the subject disclosure are described primarily with respect to glucose monitoring devices and systems, and methods of glucose monitoring, for convenience only and such description is in no way intended to limit the scope of the disclosure. It is to be understood that the analyte monitoring system may be configured to monitor a variety of analytes at the same time or at different times.

As described in detail below, embodiments include devices, systems, kits and/or methods to monitor one or more physiological parameters such as, for example, but not limited to, analyte levels, temperature levels, heart rate, user activity level, over a predetermined monitoring time period. Also provided are methods of manufacturing. Predetermined monitoring time periods may be less than about 1 hour, or may include about 1 hour or more, e.g., about a few hours or more, e.g., about a few days of more, e.g., about 3 or more days, e.g., about 5 days or more, e.g., about 7 days or more, e.g., about 10 days or more, e.g., about 14 days or more, e.g., about several weeks, e.g., about 1 month or more. In certain embodiments, after the expiration of the predetermined monitoring time period, one or more features of the system may be automatically deactivated or disabled at the on body electronics assembly and/or display device.

For example, a predetermined monitoring time period may begin with positioning the sensor in vivo and in contact with a body fluid such as ISF, and/or with the initiation (or powering on to full operational mode) of the on body electronics. Initialization of on body electronics may be implemented with a command generated and transmitted by a display device in response to the activation of a switch and/or by placing the display device within a predetermined distance (e.g., close proximity) to the on body electronics, or by user manual activation of a switch on the on body electronics unit, e.g., depressing a button, or such activation may be caused by the insertion device, e.g., as described in U.S. Patent Application No. 12/698,129 filed on February 1, 2010 and U.S. Provisional Application Nos. 61/238,646, 61/246,825, 61/247,516, 61/249,535, 61/317,243, 61/345,562, and 61/361,374.

When initialized in response to a received command from a display device, the on body electronics retrieves and executes from its memory software routine to fully power on the components of the on body electronics, effectively placing the on body electronics in full operational mode in response to receiving the activation command from the display device. For example, prior to the receipt of the command from the display device, a portion of the components in the on body electronics may be powered by its intemal power supply such as a battery while another portion of the components in the on body electronics may be in powered down or maintained in a low power state including no power state, inactive mode, or all components may be in an inactive mode, powered down mode. Upon receipt of the command, the remaining portion (or all) of the components of the on body electronics is switched to active, fully operational mode.

Embodiments of on body electronics may include one or more printed circuit boards with electronics including control logic implemented in ASIC, microprocessors, memory, and the like, and transcutaneously positionable analyte sensors forming a single assembly. On body electronics may be configured to provide one or more signals or data packets associated with a monitored analyte level upon detection of a display device of the analyte monitoring system within a predetermined proximity for a period of time (for example, about 2 minutes, e.g., 1 minute or less, e.g., about 30 seconds or less, e.g., about 10 seconds or less, e.g., about 5 seconds or less, e.g., about 2 seconds or less) and/or until a confirmation, such as an audible and/or visual and/or tactile (e.g., vibratory) notification, is output on the display device indicating successful acquisition of the analyte related signal from the on body electronics. A distinguishing notification may also be output for unsuccessful acquisition in certain embodiments.

In certain embodiments, the monitored analyte level may be correlated and/or converted to glucose levels in blood or other fluids such as ISF. Such conversion may be accomplished with the on body electronics, but in many embodiments will be accomplished with display device electronics. In certain embodiments, glucose level is derived from the monitored analyte level in the ISF.

Analyte sensors may be insertable into a vein, artery, or other portion of the body containing analyte. In certain embodiments, analyte sensors may be positioned in contact with ISF to detect the level of analyte, where the detected analyte level may be used to infer the user's glucose level in blood or interstitial tissue.

Embodiments include transcutaneous sensors and also wholly implantable sensors and wholly implantable assemblies in which a single assembly including the analyte sensor and electronics are provided in a sealed housing (e.g., hermetically sealed biocompatible housing) for implantation in a user's body for monitoring one or more physiological parameters.

Embodiments include analyte monitors that are provided in small, lightweight, battery-powered and electronically-controlled systems. Such systems may be configured to detect physical parameters of subjects, such as signals indicative of *in vivo* analyte levels using an electrochemical sensor, and collect such signals, with or without processing. Any suitable measurement technique may be used to obtain signals from the sensors, e.g., may detect current, may employ potentiometry, etc. Techniques may include, but are not limited to amperometry, coulometry, and voltammetry. In some embodiments, sensing systems may be optical, colorimetric, and the like. In some embodiments, the portion of the system that performs this initial processing may be configured to provide the raw or at least initially processed data to another unit for further collection and/or processing. Such provision of data may be effected, for example, by a wired connection, such as an electrical, or by a wireless connection, such as an IR or RF connection.

**In** certain systems, the analyte sensor is in communication with on body electronics. The on-body unit may include a housing in which the on body electronics and at least a portion of the sensor are received.

Certain embodiments are modular. The on-body unit may be separately provided as a physically distinct assembly from a monitor unit, e.g., which displays or otherwise indicates analyte levels to a user. The on-body unit may be configured to provide the analyte levels detected by the sensor and/or other information (such as temperature, sensor life, etc.) over a communication link to the monitor unit. The monitor unit, in some embodiments, may include, e.g., a mobile telephone device, an in vitro glucose meter, a personal digital assistant, or other consumer electronics such as MP3 device, camera, radio, personal computer, etc., or other communication-enabled data-processing device.

The display unit may perform a variety of functions such as but not limited to data storage and/or processing and/or analysis and/or communication, etc., on the received analyte data to generate information pertaining to the monitored analyte levels and/or process the other information. The monitor unit may incorporate a display screen, which can be used, for example, to display measured analyte levels, and/or an audio component such as a speaker to audibly provide information to a user, and/or a vibration device to provide tactile feedback to a user. It is also useful for a user of an analyte - monitoring system to be able to see trend indications (including the magnitude and direction of any ongoing trend, e.g., the rate of change of an analyte or other parameter, and the amount of time a subject is above and/or below a threshold, such as a hypoglycemic and/or hyperglycemic threshold, etc.); such data may be displayed either numerically, or by a visual indicator such as an arrow that may vary in visual attributes, like size, shape, color, animation, or direction. The monitor unit may further be adapted to receive information from or about an in vitro analyte test strip, which may be manually or automatically entered into the monitor unit. **In** some embodiments a monitor unit may incorporate an in vitro analyte test strip port and related electronics in order to be able to make discrete (e.g., blood glucose) measurements using an in vitro test strip (see, e.g., 6,175,752).

The modularity of these systems may vary where one or more components may be constructed to be single use and one or more may be constructed to be re-useable. In some embodiments the sensor is designed to be attachable and detachable from the on body electronics (and the on-body unit may be reusable), e.g., so that one or more of the components may be reused one or more times, while in other embodiments, the sensor and on body electronics may be provided as an integrated, undetachable package, which may be designed to be disposable after use, *i.e.,* not re-used.

### Embodiments of In Vivo Monitoring Systems

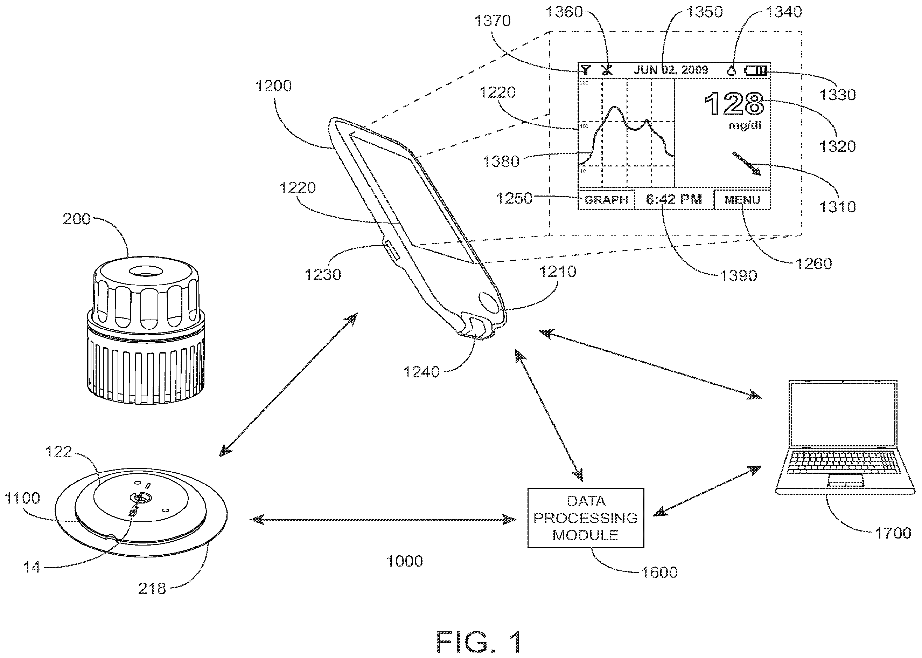

For purpose of illustration, and not limitation, the inserters described herein may be used in connection with an exemplary analyte monitoring system is depicted in FIGURE 1. It is understood that the inserters described herein may be used with any medical device on its own or in connection with a system. FIGURE 1 shows an exemplary in vivo-based analyte monitoring system 100 in accordance with embodiments of the present disclosure. As shown, in certain embodiments, analyte monitoring system 100 includes on body electronics 1100 electrically coupled to in vivo analyte sensor 14 (a proximal portion of which is shown in FIG. 1, and attached to adhesive layer 218 for attachment on a skin surface on the body of a user. On body electronics 1100 includes on body housing 122 that defines an interior compartment.

Also shown in FIGURE 1 is insertion device 200 (or insertion devices 300, 400, 2400, 2500, 2700, 3700 described herein) that, when operated, transcutaneously positions a portion of analyte sensor 14 through a skin surface and in fluid contact with ISF, and positions on body electronics 1100 and adhesive layer 218 on a skin surface, as will be described in greater detail herein. In certain embodiments, on body electronics 1100, analyte sensor 14 and adhesive layer 218 are sealed within the housing of insertion device 200 before use, and in certain embodiments, adhesive layer 218 is also sealed within the housing or the adhesive layer can provide a seal for preserving the sterility of the apparatus. Additional details regarding insertion devices are discussed, e.g., in U.S. Patent Application No. 12/698,129 and U.S. Provisional Application Nos. 61/238,646,

Referring back to the FIGURE 1, analyte monitoring system 100 includes display device 1200 which includes a display 1220 to output information to the user, an input component 1210 such as a button, actuator, a touch sensitive switch, a capacitive switch, pressure sensitive switch, jog wheel or the like, to input data or command to display device 1200 or otherwise control the operation of display device 1200. It is noted that some embodiments may include display-less devices or devices without any user interface components. These devices may be functionalized to store data as a data logger and/or provide a conduit to transfer data from on body electronics and/or a display-less device to another device and/or location. Embodiments will be described herein as display devices for exemplary purposes which are in no way intended to limit the embodiments of the present disclosure. It will be apparent that display-less devices may also be used in certain embodiments.

In certain embodiments, on body electronics 1100 may be configured to store some or all of the monitored analyte related data received from analyte sensor 14 in a memory during the monitoring time period, and maintain it in memory until the usage period ends. In such embodiments, stored data is retrieved from on body electronics 1100 at the conclusion of the monitoring time period, for example, after removing analyte sensor 14 from the user by detaching on body electronics 1100 from the skin surface where it was positioned during the monitoring time period. In such data logging configurations, real time monitored analyte level is not communicated to display device 1200 during the monitoring period or otherwise transmitted from on body electronics 1100, but rather, retrieved from on body electronics 1100 after the monitoring time period.

In certain embodiments, input component 1210 of display device 1200 may include a microphone and display device 1200 may include software configured to analyze audio input received from the microphone, such that functions and operation of the display device 1200 may be controlled by voice commands. In certain embodiments,
an output component of display device 1200 includes a speaker for outputting information as audible signals. Similar voice responsive components such as a speaker, microphone and software routines to generate, process and store voice driven signals may be provided to on body electronics 1100.

In certain embodiments, display 1220 and input component 1210 may be integrated into a single component, for example a display that can detect the presence and location of a physical contact touch upon the display such as a touch screen user interface. In such embodiments, the user may control the operation of display device 1200 by utilizing a set of pre-programmed motion commands, including, but not limited to, single or double tapping the display, dragging a finger or instrument across the display, motioning multiple fingers or instruments toward one another, motioning multiple fingers or instruments away from one another, etc. In certain embodiments, a display includes a touch screen having areas of pixels with single or dual function capacitive elements that serve as LCD elements and touch sensors.

Display device 1200 also includes data communication port 1230 for wired data communication with external devices such as remote terminal (personal computer) 1700, for example. Example embodiments of the data communication port 1230 include USB port, mini USB port, RS-232 port, Ethernet port, Firewire port, or other similar data communication ports configured to connect to the compatible data cables. Display device 1200 may also include an integrated in vitro glucose meter, including in vitro test strip port 1240 to receive an in vitro glucose test strip for performing in vitro blood glucose measurements.

Referring still to FIGURE 1, display 1220 in certain embodiments is configured to display a variety of information - some or all of which may be displayed at the same or different time on display 1220. In certain embodiments the displayed information is user-selectable so that a user can customize the information shown on a given display screen. Display 1220 may include but is not limited to graphical display 1380, for example, providing a graphical output of glucose values over a monitored time period (which may show important markers such as meals, exercise, sleep, heart rate, blood pressure, etc, numerical display 1320, for example, providing monitored glucose values (acquired or received in response to the request for the information), and trend or directional arrow display 1310 that indicates a rate of analyte change and/or a rate of the rate of analyte change, e.g., by moving locations on display 1220.

As further shown in FIGURE 1, display 1220 may also include date display 1350 providing for example, date information for the user, time of day information display 1390 providing time of day information to the user, battery level indicator display 1330 which graphically shows the condition of the battery (rechargeable or disposable) of the display device 1200, sensor calibration status icon display 1340 for example, in monitoring systems that require periodic, routine or a predetermined number of user calibration events, notifying the user that the analyte sensor calibration is necessary, audio/vibratory settings icon display 1360 for displaying the status of the audio/vibratory output or alarm state, and wireless connectivity status icon display 1370 that provides indication of wireless communication connection with other devices such as on body electronics, data processing module 1600, and/or remote terminal 1700. As additionally shown in FIGURE 1 , display 1220 may further include simulated touch screen button 1250, 1260 for accessing menus, changing display graph output configurations or otherwise for controlling the operation of display device 1200.

Referring back to FIGURE 1, in certain embodiments, display 1220 of display device 1200 may be additionally, or instead of visual display, configured to output alarms notifications such as alarm and/or alert notifications, glucose values etc, which may be audible, tactile, or any combination thereof. In one aspect, the display device 1200 may include other output components such as a speaker, vibratory output component and the like to provide audible and/or vibratory output indication to the user in addition to the visual output indication provided on display 1220. Further details and other display embodiments can be found in, e.g., U.S. Patent Application No. 12/871,901, U.S. provisional application nos. 61/238,672, 61/247,541, 61/297,625.

After the positioning of on body electronics 1100 on the skin surface and analyte sensor 14 in vivo to establish fluid contact with ISF (or other appropriate body fluid), on body electronics 1100 in certain embodiments is configured to wirelessly communicate analyte related data (such as, for example, data corresponding to monitored analyte level and/or monitored temperature data, and/or stored historical analyte related data) when on body electronics 1100 receives a command or request signal from display device 1200. In certain embodiments, on body electronics 1100 may be configured to at least periodically broadcast real time data associated with monitored analyte level which is received by display device 1200 when display device 1200 is within communication range of the data broadcast from on body electronics 1100, i.e., it does not need a command or request from a display device to send information.

For example, display device 1200 may be configured to transmit one or more commands to on body electronics 1100 to initiate data transfer, and in response, on body electronics 1100 may be configured to wirelessly transmit stored analyte related data collected during the monitoring time period to display device 1200. Display device 1200 may in turn be connected to a remote terminal 1700 such as a personal computer and functions as a data conduit to transfer the stored analyte level information from the on body electronics 1100 to remote terminal 1700. In certain embodiments, the received data from the on body electronics 1100 may be stored (permanently or temporarily) in one or more memory of the display device 1200. In certain other embodiments, display device 1200 is configured as a data conduit to pass the data received from on body electronics 1100 to remote terminal 1700 that is connected to display device 1200.

Referring still to FIGURE 1, also shown in analyte monitoring system 1000 are data processing module 1600 and remote terminal 1700. Remote terminal 1700 may include a personal computer, a server terminal a laptop computer or other suitable data processing devices including software for data management and analysis and communication with the components in the analyte monitoring system 1000. For example, remote terminal 1700 may be connected to a local area network (LAN), a wide area network (WAN), or other data network for uni-directional or bi-directional data communication between remote terminal 1700 and display device 1200 and/or data processing module 1600.

Remote terminal 1700 in certain embodiments may include one or more computer terminals located at a physician's office or a hospital. For example, remote terminal 1700 may be located at a location other than the location of display device 1200. Remote terminal 1700 and display device 1200 could be in different rooms or different buildings. Remote terminal 1700 and display device 1200 could be at least about one mile apart, e.g., at least about 100 miles apart, e.g., at least about 1000 miles apart. For example, remote terminal 1700 could be in the same city as display device 1200, remote terminal 1700 could be in a different city than display device 1200, remote terminal 1700 could be in the same state as display device 1200, remote terminal 1700 could be in a different state than display device 1200, remote terminal 1700 could be in the same country as display device 1200, or remote terminal 1700 could be in a different country than display device 1200, for example.

In certain embodiments, a separate, optional data communication/processing device such as data processing module 1600 may be provided in analyte monitoring system 1000. Data processing module 1600 may include components to communicate using one or more wireless communication protocols such as, for example, but not limited to, infrared (IR) protocol, Bluetooth protocol, Zigbee protocol, and 802.11 wireless LAN protocol. Additional description of communication protocols including those based on Bluetooth protocol and/or Zigbee protocol can be found in U.S. Patent Publication No. 2006/0193375. Data processing module 1600 may further include communication ports, drivers or connectors to establish wired communication with one or more of display device 1200, on body electronics 1100, or remote terminal 1700 including, for example, but not limited to USB connector and/or USB port, Ethernet connector and/or port, FireWire connector and/or port, or RS-232 port and/or connector.

In certain embodiments, data processing module 1600 is programmed to transmit a polling or query signal to on body electronics 1100 at a predetermined time interval (e.g., once every minute, once every five minutes, or the like), and in response, receive the monitored analyte level information from on body electronics 1100. Data processing module 1600 stores in its memory the received analyte level information, and/or relays or retransmits the received information to another device such as display device 1200. More specifically in certain embodiments, data processing module 1600 may be configured as a data relay device to retransmit or pass through the received analyte level data from on body electronics 1100 to display device 1200 or a remote terminal (for example, over a data network such as a cellular or WiFi data network) or both.

In certain embodiments, on body electronics 1100 and data processing module 1600 may be positioned on the skin surface of the user within a predetermined distance of each other (for example, about 2.54-30.48 cm (1-12 inches), or about 2.54-25.40 cm (1-10 inches), or about 2.54-17.78 cm (1-7 inches), or about 2.54-12.70 cm (1-5 inches)) such that periodic communication between on body electronics 1100 and data processing module 1600 is maintained. Alternatively, data processing module 1600 may be worn on a belt or clothing item of the user, such that the desired distance for communication between the on body electronics 1100 and data processing module 1600 for data communication is maintained. In a further aspect, the housing of data processing module 1600 may be configured to couple to or engage with on body electronics 1100 such that the two devices are combined or integrated as a single assembly and positioned on the skin surface. In further embodiments, data processing module 1600 is detachably engaged or connected to on body electronics 1100 providing additional modularity such that data processing module 1600 may be optionally removed or reattached as desired.

Referring again to FIGURE 1, in certain embodiments, data processing module 1600 is programmed to transmit a command or signal to on body electronics 1100 at a predetermined time interval such as once every minute, or once every 5 minutes or once every 30 minutes or any other suitable or desired programmable time interval to request analyte related data from on body electronics 1100. When data processing module 1600 receives the requested analyte related data, it stores the received data. In this manner, analyte monitoring system 1000 may be configured to receive the continuously monitored analyte related information at the programmed or programmable time interval, which is stored and/or displayed to the user. The stored data in data processing module 1600 may be subsequently provided or transmitted to display device 1200, remote terminal 1700 or the like for subsequent data analysis such as identifying frequency of periods of glycemic level excursions over the monitored time period, or the frequency of the alarm event occurrence during the monitored time period, for example, to improve therapy related decisions. Using this information, the doctor, healthcare provider or the user may adjust or recommend modification to the diet, daily habits and routines such as exercise, and the like.

In another embodiment, data processing module 1600 transmits a command or signal to on body electronics 1100 to receive the analyte related data in response to a user activation of a switch provided on data processing module 1600 or a user initiated command received from display device 1200. In further embodiments, data processing module 1600 is configured to transmit a command or signal to on body electronics 1100 in response to receiving a user initiated command only after a predetermined time interval has elapsed. For example, in certain embodiments, if the user does not initiate communication within a programmed time period, such as, for example about 5 hours from last communication (or 10 hours from the last communication, or 24 hours from the last communication), the data processing module 1600 may be programmed to automatically transmit a request command or signal to on body electronics 1100. Alternatively, data processing module 1600 may be programmed to activate an alarm to notify the user that a predetermined time period of time has elapsed since the last communication between the data processing module 1600 and on body electronics 1100. In this manner, users or healthcare providers may program or configure data processing module 1600 to provide certain compliance with analyte monitoring regimen, so that frequent determination of analyte levels is maintained or performed by the user.

In certain embodiments, when a programmed or programmable alarm condition is detected (for example, a detected glucose level monitored by analyte sensor 14 that is outside a predetermined acceptable range indicating a physiological condition which requires attention or intervention for medical treatment or analysis (for example, a hypoglycemic condition, a hyperglycemic condition, an impending hyperglycemic condition or an impending hypoglycemic condition), the one or more output indications may be generated by the control logic or processor of the on body electronics 1 100 and output to the user on a user interface of on body electronics 1100 so that corrective action may be timely taken. In addition to or alternatively, if display device 1200 is within communication range, the output indications or alarm data may be communicated to display device 1200 whose processor, upon detection of the alarm data reception, controls the display 1220 to output one or more notification.

In certain embodiments, control logic or microprocessors of on body electronics 1100 include software programs to determine future or anticipated analyte levels based on information obtained from analyte sensor 14, e.g., the current analyte level, the rate of change of the analyte level, the acceleration of the analyte level change, and/or analyte trend information determined based on stored monitored analyte data providing a historical trend or direction of analyte level fluctuation as function time during monitored time period. Predictive alarm parameters may be programmed or programmable in display device 1200, or the on body electronics 1100, or both, and output to the user in advance of anticipating the user's analyte level reaching the future level. This provides the user an opportunity to take timely corrective action.

Information, such as variation or fluctuation of the monitored analyte level as a function of time over the monitored time period providing analyte trend information, for example, may be determined by one or more control logic or microprocessors of display device 1200, data processing module 1600, and/or remote terminal 1700, and/or on body electronics 1100. Such information may be displayed as, for example, a graph (such as a line graph) to indicate to the user the current and/or historical and/or and predicted future analyte levels as measured and predicted by the analyte monitoring system 1000. Such information may also be displayed as directional arrows (for example, see trend or directional arrow display 1310) or other icon(s), e.g., the position of which on the screen relative to a reference point indicated whether the analyte level is increasing or decreasing as well as the acceleration or deceleration of the increase or decrease in analyte level. This information may be utilized by the user to determine any necessary corrective actions to ensure the analyte level remains within an acceptable and/or clinically safe range. Other visual indicators, including colors, flashing, fading, etc., as well as audio indicators including a change in pitch, volume, or tone of an audio output and/or vibratory or other tactile indicators may also be incorporated into the display of trend data as means of notifying the user of the current level and/or direction and/or rate of change of the monitored analyte level. For example, based on a determined rate of glucose change, programmed clinically significant glucose threshold levels (e.g., hyperglycemic and/or hypoglycemic levels), and current analyte level derived by an in vivo analyte sensor, the system 1000 may include an algorithm stored on computer readable medium to determine the time it will take to reach a clinically significant level and will output notification in advance of reaching the clinically significant level, e.g., 30 minutes before a clinically significant level is anticipated, and/or 20 minutes, and/or 10 minutes, and/or 5 minutes, and/or 3 minutes, and/or 1 minute, and so on, with outputs increasing in intensity or the like.

Referring again back to FIGURE 1, in certain embodiments, software algorithm(s) for execution by data processing module 1600 may be stored in an external memory device such as an SD card, microSD card, compact flash card, XD card, Memory Stick card, Memory Stick Duo card, or USB memory stick/device including executable programs stored in such devices for execution upon connection to the respective one or more of the on body electronics 1100, remote terminal 1700 or display device 1200. In a further aspect, software algorithms for execution by data processing module 1600 may be provided to a communication device such as a mobile telephone including, for example, WiFi or Internet enabled smart phones or personal digital assistants (PDAs) as a downloadable application for execution by the downloading communication device.

Examples of smart phones include Windows^{®}, Android^{™}, iPhone^{®} operating system, Palm^{®} WebOS^{™}, Blackberry^{®} operating system, or Symbian^{®} operating system based mobile telephones with data network connectivity functionality for data communication over an internet connection and/or a local area network (LAN). PDAs as described above include, for example, portable electronic devices including one or more microprocessors and data communication capability with a user interface (e.g., display/output unit and/or input unit, and configured for performing data processing, data upload/download over the internet, for example. In such embodiments, remote terminal 1700 may be configured to provide the executable application software to the one or more of the communication devices described above when communication between the remote terminal 1700 and the devices are established.

In still further embodiments, executable software applications may be provided over-the-air (OTA) as an OTA download such that wired connection to remote terminal 1700 is not necessary. For example, executable applications may be automatically downloaded as software download to the communication device, and depending upon the configuration of the communication device, installed on the device for use automatically, or based on user confirmation or acknowledgement on the communication device to execute the installation of the application. The OTA download and installation of software may include software applications and/or routines that are updates or upgrades to the existing functions or features of data processing module 1600 and/or display device 1200.

Referring back to remote terminal 1700 of FIGURE 1, in certain embodiments, new software and/or software updates such as software patches or fixes, firmware updates or software driver upgrades, among others, for display device 1200 and/or on body electronics 1100 and/or data processing module 1600 may be provided by remote terminal 1700 when communication between the remote terminal 1700 and display device 1200 and/or data processing module 1600 is established. For example, software upgrades, executable programming changes or modification for on body electronics 1100 may be received from remote terminal 1700 by one or more of display device 1200 or data processing module 1600, and thereafter, provided to on body electronics 1100 to update its software or programmable functions. For example, in certain embodiments, software received and installed in on body electronics 1100 may include software bug fixes, modification to the previously stalled software parameters (modification to analyte related data storage time interval, resetting or adjusting time base or information of on body electronics 1100, modification to the transmitted data type, data transmission sequence, or data storage time period, among others). Additional details describing field upgradability of software of portable electronic devices, and data processing are provided in U.S. Application Nos. 12/698,124, 12/794,721, 12/699,653, and 12/699,844, and U.S. Provisional Application Nos. 61,359,265, and 61/325,155.

### The Sensor

The analyte sensor 14 of the analyte measurement system 100 may be used to monitor levels of a wide variety of analytes. Analytes that may be monitored include, for example, acetylcholine, amylase, bilirubin, cholesterol, chorionic gonadotropin, creatine kinase (e.g., CK-MB), creatine, DNA, fructosamine, glucose, glutamine, growth hormones, hormones, ketones, lactate, peroxide, prostate-specific antigen, prothrombin, RNA, thyroid-stimulating hormone, and troponin. The concentration of drugs, such as, for example, antibiotics (e.g., gentamicin, vancomycin, and the like), digitoxin, digoxin, drugs of abuse, theophylline, and warfarin, may also be monitored. One or more analyte may be monitored by a given sensor. In those embodiments that monitor more than one analyte, the analytes may be monitored at the same or different times, which may use the same on body electronics (e.g., simultaneously) or with different on body electronics.

In one embodiment of the present disclosure, sensor 14 is physically positioned in or on the body of a user whose analyte level is being monitored. Sensor 14 may be configured to continuously sample the analyte level of the user and convert the sampled analyte level, e.g., glucose concentration into a corresponding data signal, e.g., a current or voltage, for input into on body electronics. Alternatively, sensor 14 may be configured to sample analyte levels on demand. The on body electronics may amplify, filter, average, and/or otherwise process signal provided by the sensor.

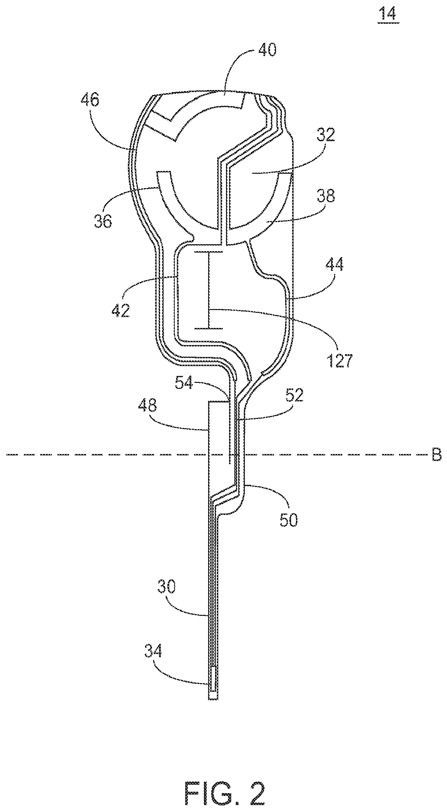

An embodiment of the sensor 14 is illustrated in FIGURE 2. It is understood that the inserters described herein can be used with other medical devices. The shape(s) described herein are exemplary only. Other sensor shapes are contemplated. In some embodiments, sensor 14 includes a substrate which is a dielectric, e.g., a polymer or plastic material, such as polyester or polyamide. In this embodiment, the sensor is constructed so that a portion is positionable beneath skin and a portion is above skin.
Accordingly, sensor 14 includes an insertion or internal portion 30 and an external or electrical contact portion 32. In some embodiments, the contact portion 32 includes several conductive contacts 36, 38, and 40 (herein shown as three contacts) for connection to other electronics, e.g., at the on body electronics 1100. (See Figure 1.) The contacts provided in this embodiment are for a working electrode, a reference electrode, and a counter electrode. In some embodiments, two or more working electrodes are provided. The operative portions of these electrodes, that is, working electrode, reference electrode, and counter electrode (not individually shown), are provided at the insertion portion, e.g., at the distal end of insertion portion 30, e.g., portion 34. In some embodiments, one or more electrodes may be external to the body, e.g., an external counter electrode. The contact and operative portions of the electrodes are connected by circuit traces 42, 44, and 46 running on the surface of the substrate. In some embodiments, the traces are provided in channels, or may be embedded within the substrate, or may traverse different sides of the substrate. The conductive contacts, conductive traces, and electrodes are fabricated from conductive material, such as platinum, palladium, gold, carbon, or the like. More than one material may be used for a given sensor. Further details of sensors are described, e.g., in U.S. Patent Nos. 6,175,572 and 6,103,033.

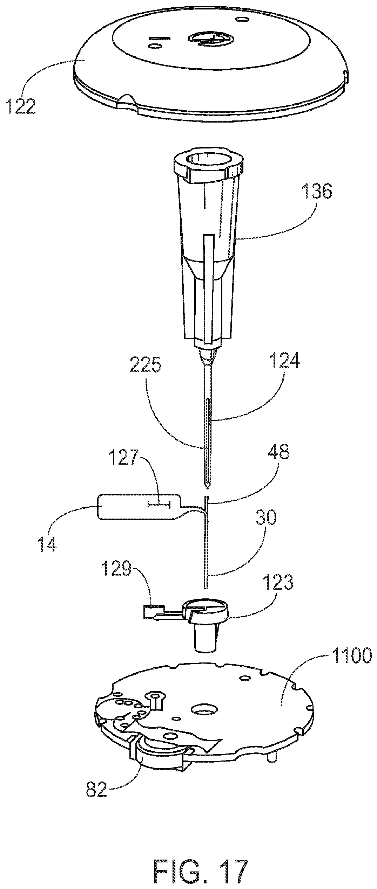

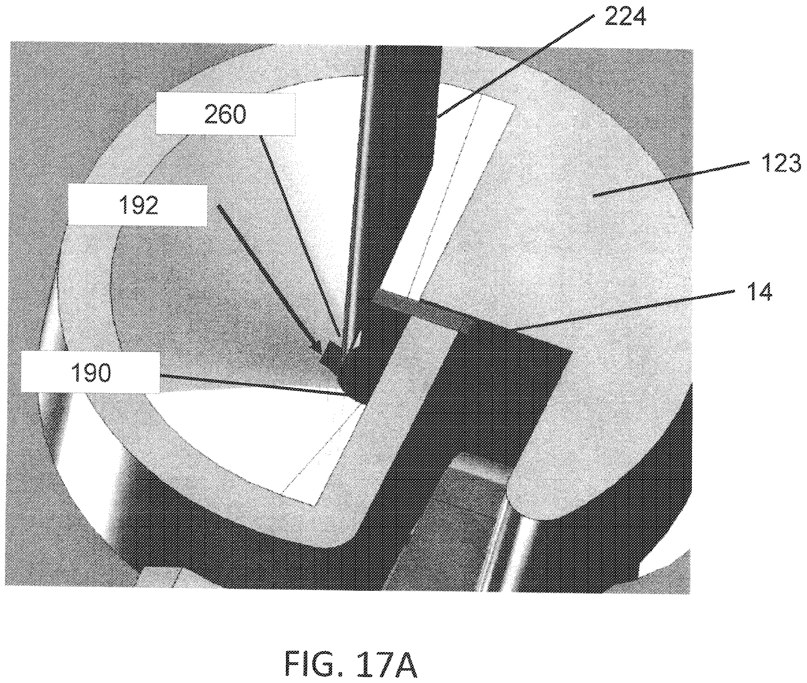

Sensor 14 may include a proximal retention portion 48. The insertion portion 30 and the proximal retention portion 48 are sized and configured to be positioned with a sharp for installation into the skin of a subject, as described herein. In use, the sensor 14 may be configured to bend (e.g., along the line B) and therefore be positioned in two substantially perpendicular, intersecting planes. Such bending may occur prior to or during coupling to the on body electronics as described below. (See FIGURE 17).

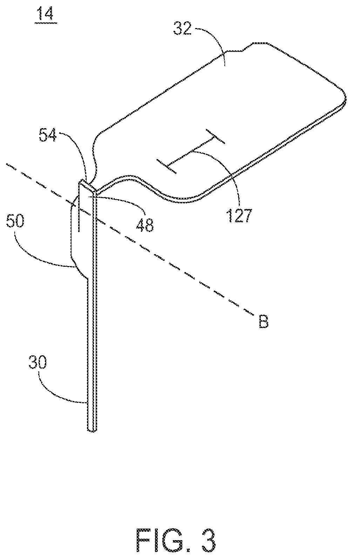

Portions 48 and 52 which provide a path for electrical connections, e.g., the conductive traces, between the proximal and distal portions of the sensor. Sensor 14 is further provided with a notch or cut-out 54. Such configuration facilitates the sensor 14 to bend {e.g., along the line indicated by line B) such that retention portion 48 remains upright and therefore be positioned in two substantially perpendicular, intersecting planes, as illustrated in FIGURE 3. As will be described below, the sensor tab 50 can be
encased in the on body housing 122 to aid in securing and positioning the sensor 14. Proximal retention portion 48 maintains its longitudinal alignment with insertion portion 30 for positioning within an insertion sharp.

Embodiments of analyte sensors have been described herein to operate electrochemically, through an arrangement of electrodes having chemical sensing layers applied thereto, by generating an electrical current proportional to the volume of a redox reaction of the analyte (and indicative of analyte concentration), catalyzed by an analyte-specific oxidizing enzyme. Embodiments exist in which the number of electrodes provided to bring about and detect the level of these reactions is two, three, or a greater number. However, other types of sensors may be employed as described herein.

A portion of sensor 14 may be situated above the surface of the skin, with a distal portion 30 penetrating through the skin and into the subcutaneous space in contact with the user's biofluid, such as ISF. Further details regarding the electrochemistry of sensor 14 is provided in U.S. Patent Nos. 5,264, 104; 5,356,786; 5,262,035; 5,320,725; and 6,990,366.

In some embodiments, the sensor is implantable into a subject's body for a usage period (e.g., a minute or more, at least one day or more, about one to about 30 days or even longer, about three to about fourteen days, about three to about seven days, or in some embodiments, longer periods of up to several weeks) to contact and monitor an analyte present in a biological fluid. In this regard, the sensor can be disposed in a subject at a variety of sites (e.g., abdomen, upper arm, thigh, etc.), including intramuscularly, transcutaneously, intravascularly, or in a body cavity.

In some embodiments, sensor 14 is employed by insertion and/or implantation into a user's body for some usage period. In such embodiments, the substrate may be formed from a relatively flexible material.

While the embodiments illustrated in FIGURES 2-3 have three electrodes, other embodiments can include a fewer or greater number of electrodes. For example, a two-electrode sensor can be utilized. The sensor 14 may be externally-powered and allow a current to pass which current is proportional to the amount of analyte present. Alternatively, the sensor 14 itself may act as a current source in some embodiments. In some two-electrode embodiments, the sensor may be self-biasing and there may be no need for a reference electrode. An exemplary self-powered, two-electrode sensor is described in U.S. Patent Application Serial No. 12/393,921, filed February 26, 2009, and entitled "Self-Powered Analyte Sensor,". The level of current provided by a self-powered sensor may be low, for example, on the order of nanoamperes, in certain embodiments.

### Insertion Assembly

Insertion assemblies are provided, which are used to install a medical device to the subject. In some embodiments, an insertion assembly includes an inserter and the medical device itself. The inserter can be configured to insert various medical devices into the subject, such as for example, an analyte sensor, an infusion set, or a cannula. In some embodiments, the inserter can be configured to install a combination of such devices, e.g., a combined sensor/infusion set, etc., at the same or different times or locations. For example, in certain embodiments a given inserter can be configured to install a first device and a second device at different times. In this regard, the inserter can be reusable. For example, an inserter may be modifiable to be used with more than one medical device, to include more than one type of medical device, e.g., by attaching an adapter and/or removing detaching a portion of an inserter. The inserter can install the medical device in, under, or through the skin of the subject, or place the medical device on the surface of the skin. The medical device can include features or structures, e.g., barbs, tabs, adhesive, etc., to maintain the device in position with respect to the skin after insertion. The inserter device may also be used as a lancet, e.g., to pierce the skin without inserting or installing a medical device.

In some embodiments, an insertion assembly includes an inserter, an analyte sensor, and a power supply. The power supply may be applied to the patient, e.g., to the surface of the skin, simultaneously with the analyte sensor by the inserter. In other embodiments, the battery is installed after or before installation of the analyte sensor. In such case the power supply may be applied by the inserter or separately. The power supply may be used to provide a current or a potential to the sensor and/or to provide power for communication of one or more signals to the monitor unit.

In some embodiments, an insertion assembly includes an inserter, a medical device such as an analyte sensor, and on body electronics. The on body electronics may be deployed and/or installed simultaneously with the analyte sensor by the inserter. In other embodiments, the on body electronics are installed after or before installation of the analyte sensor. For example, the analyte sensor may be installed by the inserter, and the on body electronics may be subsequently installed.

In some embodiments, the on body electronics provide a voltage or current to the analyte sensor. In some embodiments, the on body electronics process signals provided by the analyte sensor. In further embodiments, the on body electronics may include communications functionality for providing signal relating to signal provided by the analyte sensor to a further component, such as, *e.g.,* a monitor unit, a computer, or other component. In some embodiments, communications circuitry, such as an RFID antenna, is provided. The power supply may be used to power some or all of these functions. In some embodiments, power is provided from the monitor unit, e.g., via inductive coupling.

An inserter can include a plurality of different components. For example, an inserter may include one or more components for advancing a sharp towards the skin of the subject. The sensor and on body electronics may be supported by a support structure, such as a carriage. A driver may be provided for advancing the sharp and/or the analyte sensor/support structure towards the skin of the patient. In some embodiments, the actuator is directly or indirectly coupled to the sharp and/or support structure, such that manual force applied by the user to the actuator is transferred to the sharp and/or support structure. In some embodiments, the applied force drives the sharp and/or support structure between a retracted position (disposed within the insertion device) and an advanced position (disposed towards the skin of the patient). In some embodiments, the sensor and on body electronics is maintained in a retracted position prior to installation by contacting projections extending inwardly from a recess in the inserter. In accordance with this embodiment, the sensor and on body electronics are temporarily maintained operatively between the support structure and the projections disposed on the interior wall of the sheath.

An inserter can also include one or more components for retracting the sharp, while allowing the analyte sensor and optional on body electronics to remain on the subject. The components for retracting the sharp can include a retractor. It is understood that the retractor and the actuator may be the same structure or include some common components. In some embodiments, the retractor is directly or indirectly coupled to the sharp such that the manual force applied by the user is transferred from the retractor to the sharp to retract the sharp from the skin. In other embodiments, a drive assembly may be provided to retract the sharp. For example, the drive assembly may include a spring, motor, hydraulic piston, etc., to retract the sharp away from the skin of the subject. The drive assembly may also include a linear drive component.

In some embodiments, the retractor withdraws the sharp upon actuation by the user. In such cases, the user actuates the retractor when it is desired to withdraw the sharp. For example, the retractor may include a release switch. Upon activation of the release switch, the drive assembly, *e.g*., the spring or other driver, retracts the sharp from the skin. In other embodiments, the retractor and the actuator include common components. After activating the actuator to advance the sharp and the analyte sensor, the user releases the actuator, which allows the drive assembly to withdraw the sharp from the skin.

In some embodiments, the retractor withdraws the sharp without further user interaction after actuation of insertion. For example, the inserter may include features or components which automatically retract the sharp upon advancement of the sharp and support structure by a predetermined amount. Inserter devices, in which no further action by the user is required to initiate withdrawal of the sharp after insertion, are referred to herein as having "automatic" withdrawal of the sharp.

### Inserter Devices

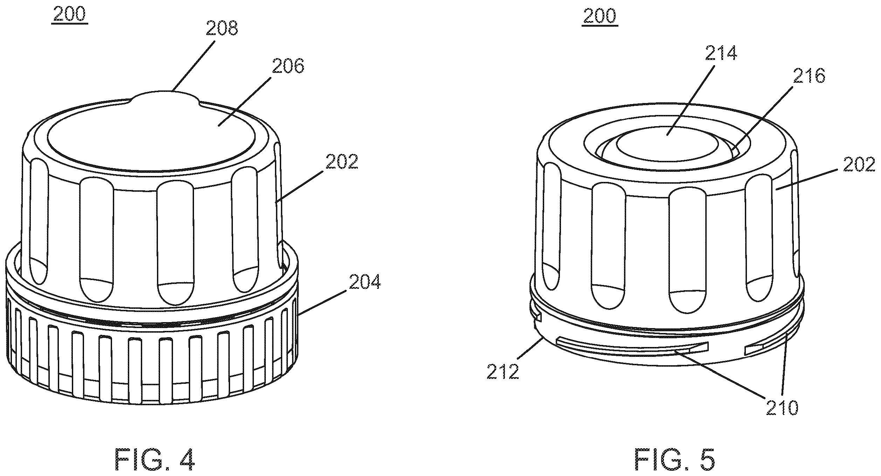

An inserter 200 in accordance with an exemplary embodiment is illustrated in FIGURE 4. Inserter 200 includes a housing 202 and a removable distal cap 204 for maintaining a sterile environment for the medical device and sharp housed therein. In some embodiments, inserter 200 has a maximum diameter, of about 30mm to about 60 mm, e.g., about 40 mm, about 43 mm, about 43.5 mm, about 50.5 mm, about 54.5 mm, etc. In some embodiments, inserter 200 has a maximum height of about 40 mm to about 80 mm, e.g., about 44 mm, about 46 mm, about 50 mm, about 53 mm, about 67 mm, about 71 mm, etc. In some embodiments, inserter 200 has a volume of about 35 cm³ to about 110 cm³, *e.g.,* about 40 cm³, about 41 cm³, about 50 cm³, about 60 cm³, about 61 cm³, about 62 cm³, about 69 cm³, about 70 cm³, about 79 cm³, about 90 cm³, about 106 cm³, etc. In the case of inserter 200, the dimension are defined with respect to the housing 202.

Housing 202 and distal cap 204 may be fabricated from any suitable materials such as metal, plastic, etc. In some embodiments cap 204 may be fabricated from a polymer or plastic material. Also provided is a removable proximal cover 206, which, among other things, prevents accidental deployment of the inserter 200 and maintains a sterile environment. In some embodiments, proximal cover 206 is a sheet of material such as a foil sheet or the like secured to the upper surface of housing 202 using an adhesive, and may include a tab 208 to assist removal of the cover 206. Proximal cover 206 may also be a plastic sheet or member that forms a seal with housing 202. In some embodiments, proximal cover 206 may include a pull tab or a perforated section for easy removal.

As illustrated in FIGURE 5, proximal cover 206 and distal cap 204 are shown removed from inserter 200. Distal cap 204 is secured to housing 202, *e.g.,* by use of threads 210. In some embodiments, distal cap 204 is secured by a friction fit, snap fit, a bayonet mount, an adhesive, etc. The distal portion of cap 204 may include a recess for retaining a desiccant therein. In some embodiments, a silica gel or molecular sieves may be used. Such material can be in granular form (pellets) or pressed into tablets, or otherwise. In some embodiments, silica gel tablets are used. Embodiments may include desiccant and/or packaging as described in U.S. Patent Application Serial No. 12/714,439. Cap 204 may be provided with one or more apertures, which allows for passage of air to the desiccant to remove moisture from the interior of the inserter 200.

Housing 202 includes a distal portion 212 for placement on the skin of a subject. Inserter 200 includes an actuator 214 to advance a medical device into the skin of the subject. In some embodiments, actuator 214 is disposed within an opening 216 in housing 202 and can be longitudinally moveable within housing 202.

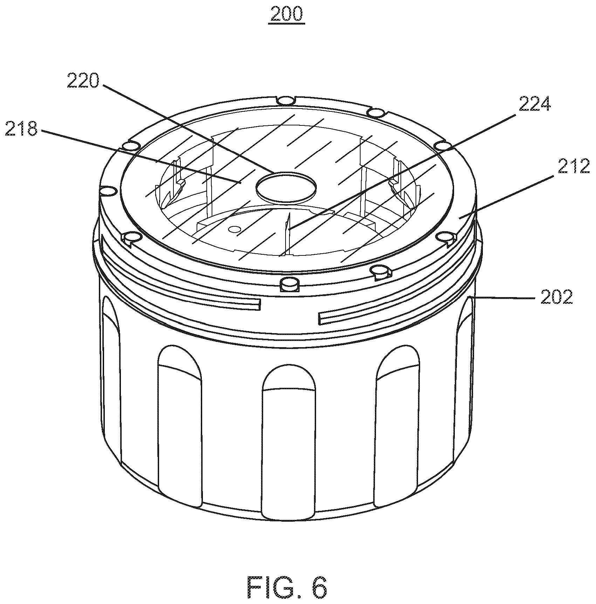

The distal portion of inserter 200 is illustrated in FIGURE 6. In some embodiments, an adhesive pad 218, having adhesive material 218 on both faces, is provided across the distal portion 212 of the housing 202. A central aperture 220 may be provided in adhesive pad 218. As will be described in greater detail herein, inserter 200 supports a medical device, such as on body housing 122 (not shown) and a sharp 224. In some embodiments, on body housing 122, includes an analyte sensor 14. During insertion, sharp 224 passes through aperture 220 and into the skin of the subject carrying at least the sensor 14 with it.

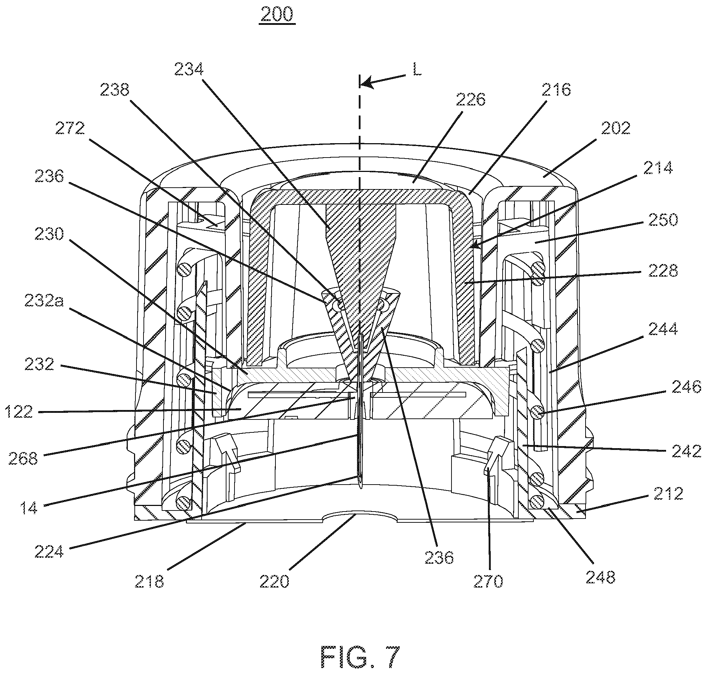

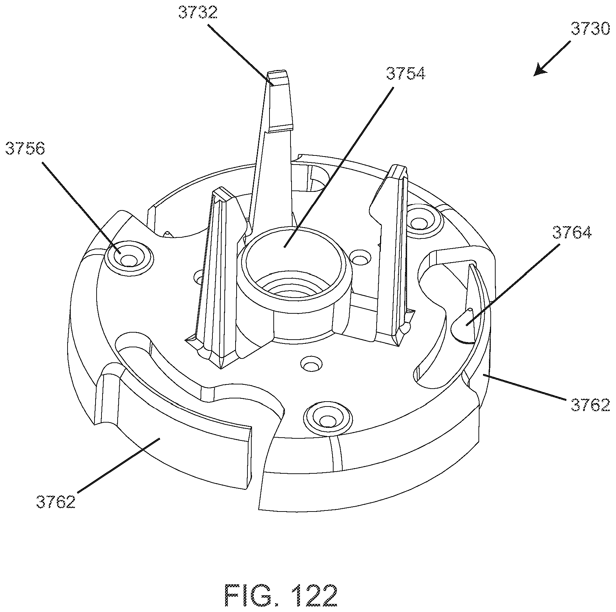

FIGURE 7 illustrates inserter 200 in cross-section, in an initial configuration prior to use, after removal of the distal cap 204. Actuator 214 may be cylindrical in shape (or other shape as appropriate) and, including an upper contact surface 226, capable of being depressed by a user and/or a mechanism, as described herein. Actuator 214 may further include side walls 228 extending downwardly from upper surface 226, and which engage or otherwise contact the upper surface of carriage 230. Carriage 230 provides a support for holding the medical device, such as on body housing 122, prior to and during installation. In some embodiments, carriage 230 includes a distal portion 232, which may be configured to form a substantially concave recess 232a as shown in this embodiment, for supporting the medical device therein. In some embodiments, the on body housing 122 is supported within the recess 232a of carriage 230 in a snap-fit or other relationship. In some embodiments, carriage 230 does not include a recess. In such
embodiments, carriage may include one or more projections which contact and/or advance the on body housing 122. (See, e.g., FIGURES 122, 135-136 herein.)

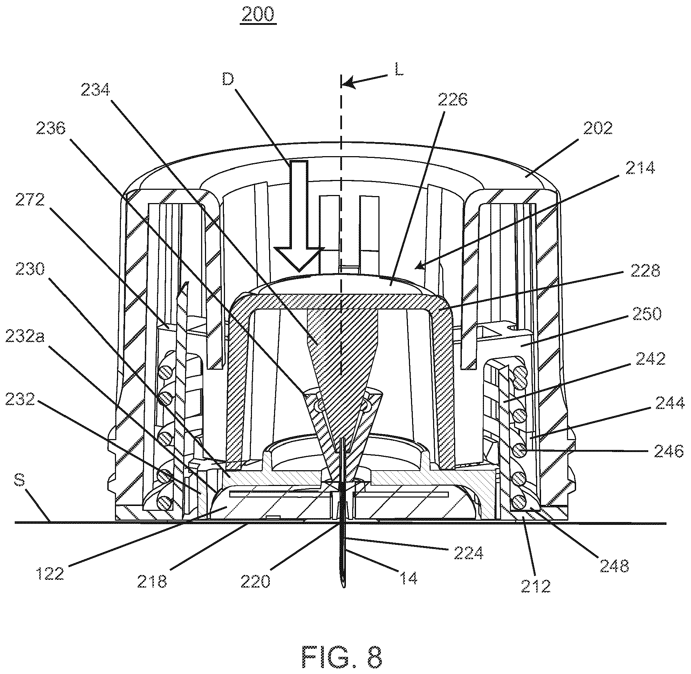

In FIGURE 8 the longitudinal axis L of the inserter 200 is illustrated. Extending distally from the upper surface 226 of actuator 214 and substantially parallel to the longitudinal axis is a support member 234, which may have an elongated configuration. Support member 234 supports needle hub 236, from which sharp 224 extends longitudinally within the inserter 200. In some embodiments, the sharp 224 is supported at an oblique angle, *e.g*., between about 0° and 90° with respect to the skin surface. Needle hub 236 can be secured to support member 234 via an interlocking O-ring configuration, adhesive, or other techniques known in the art. Support member 234 can be omitted and needle hub 236 can be secured to the actuator 214 directly in some embodiments, e.g., by manufacturing needle hub 236 as a single component with actuator 226 or by otherwise adhering needle hub 236 to actuator 226.

In some embodiments, sharp 224 is a solid needle, for example, if inserter 200 is used to insert a cannula. In some embodiments, sharp 224 is provided with a substantially cylindrical configuration defining an interior bore, e.g., a rigid cylindrical member or a hypodermic-style needle. Sharp 224 may also be provided with an elongated longitudinal opening or gap in the wall of the sharp 224 (see, sharp 224 in FIGURES 11-18). In some embodiments, sharp 224 is fabricated from a sheet of metal, and folded into a substantially "V" or "U" or "C" configuration in cross-section to define the longitudinal recess.

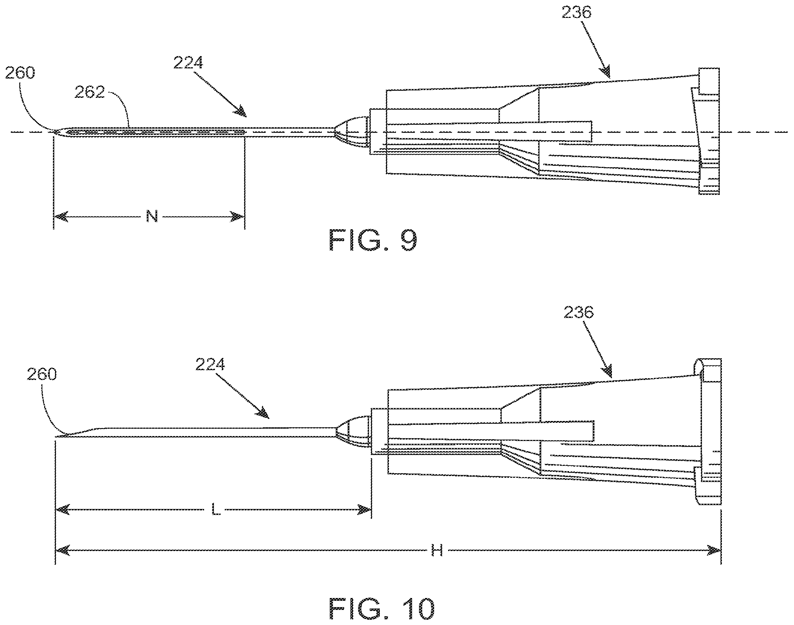

Needle hub 236 is further illustrated in FIGURES 9-10. Needle hub 236 supports sharp 224, having a sharpened distal portion 260. In some embodiments, as discussed herein, a longitudinal wall opening or gap 262 is provided in at least a portion of the wall of the sharp 224. The length N of the gap 262 is selected to be commensurate with the length of the insertion portion 30 through to the proximal retention portion 48 of the sensor 14 where the bend at line B occurs (See FIGURES 2-3), and in certain embodiments may be about 3 mm to about 50 mm, *e.g*., about 5 mm, or about 10 mm, or about 15 mm, or about 20 mm. The length L of the sharp 224 may be about 3 mm to about 50 mm, *e.g*., 5mm or more, or about 10 mm, or about 20 mm, or about 30 mm, or about 50 mm, and is selected based upon the length of the insertion portion 30 of a sensor and the desired depth of the insertion portion 30 of the sensor 14. In some embodiments, the distance or spacing between the two edges of the gap is about 0.2mm to about 0.5mm, e.g., about 0.22mm, about 0.25mm, etc. (See, spacing 257 in FIG. 11).

The distal portion 260 of sharp 224 is illustrated in greater detail in FIGURES 11-13. As illustrated in FIGURE 11, sharp 224 has a substantially "C"- or "U"-shaped profile in this embodiment, but may have other configurations, *e.g*., substantially "V"-shaped. A longitudinal gap 262 is provided in the wall of the sharp 224. FIGURE 12 illustrates distal portion 260 is provided with an angled tip. In some embodiments, the angled tip may be provided with a first angled tip portion 264 and a second steep-angled tip portion 266. The exemplary configuration, which includes multiple edges and faces, provides a sharp point to reduce penetration force, trauma, and bleeding for the subject. The distal section of the sensor body has a width sized to fit within the gap 262 of the insertion sharp 224 having a diameter less than about 20 to about 26 gauge, *e.g*., 21 gauge to about 25 gauge, where in certain embodiments the sharp is 21 gauge or 23 gauge or 25 gauge. Such sharp may be used with a sensor having a width or diameter - at least the portion that is carried by the sharp - of about .20 mm to about .80 mm, e.g., about .25 mm to about .60 mm, where in some embodiments the width or diameter of at least a portion of a sensor is .27 mm or .33 mm or .58 mm. In some embodiments, sharp 224 is fabricated from a sheet of metal and folded into a substantially "V" or "U" or "C" configuration in cross-section.

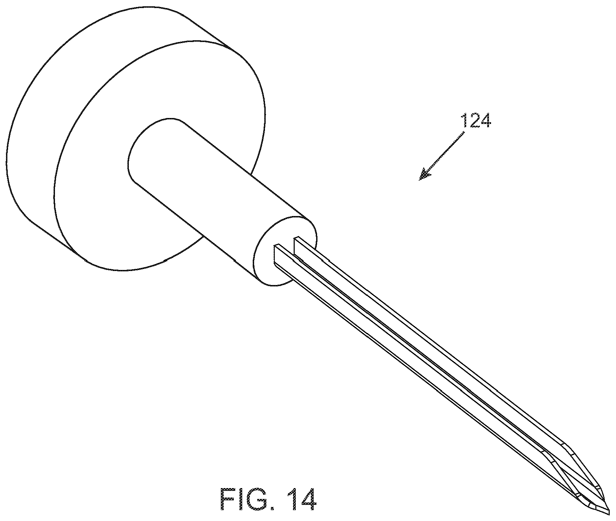

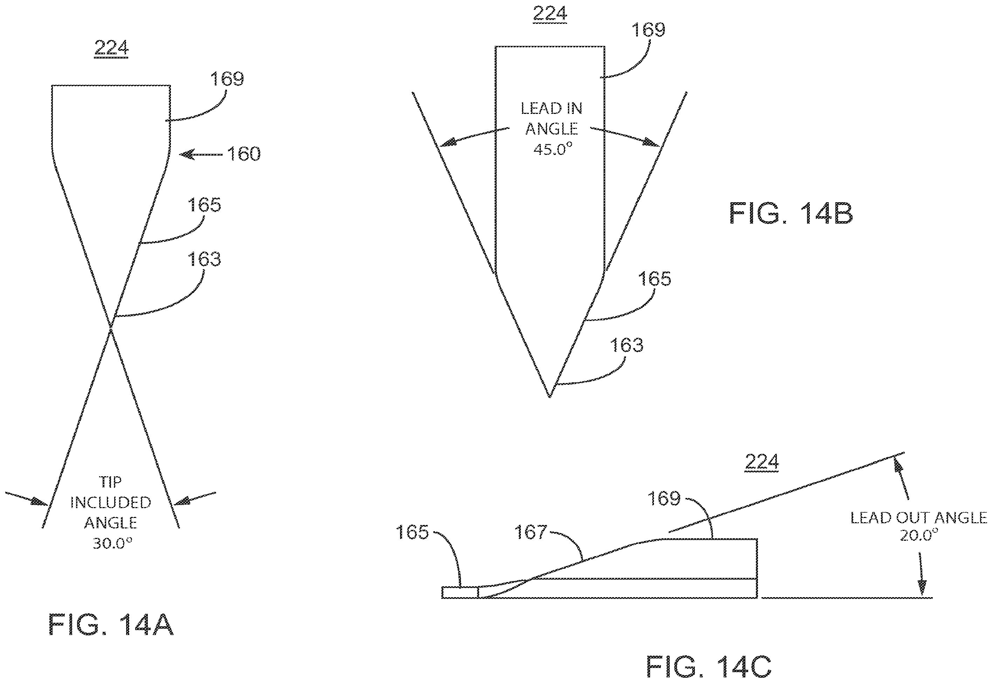

Various technologies can be used to manufacture a folded sheet of metal to form sharp 224. For example, etched-sheet metal technology can be used to form the sharp 224. In this manner, the sharp can be formed having a very sharp edge so that penetration through the skin during insertion is less painful. In other embodiments, a progressive die technology may be utilized to form a complex sheet-metal shape that has a sharp edge as depicted in FIGURE 14. In some embodiments, the sharp 224 can be molded with a plastic cap so that the sharp can be handled during the inserter assembly process. Further, the die cut sharp may be molded with plastic to reinforce the "V," "U," or "C" shaped sheet metal configuration. In some embodiments, a "U" shaped cross-section can be provided with having flat, rather than curved walls. The "U" shaped configuration provides the advantage that they can more securely and closely hold the sensor. Also, the "U" shaped configuration provides the advantage that it has a reduced cross-section when compared with a comparable circular cross section. Further details of the tip of sharp 224 are illustrated in FIGURES 14A-C. As illustrated in FIGURES 14A-B, a top view of the sharp 224 is shown. This represents a flat portion of the sharp, e.g., the bottom of the "U" configuration. A tip is formed by first distal edges 263 closest to the distal tip and second distal edges 265 between the first distal edges 263 and the substantially parallel side walls 269. In some embodiments, the first distal edges 163 form an "included tip" angle of about 15 degrees, about 30 degrees, or about 60 degrees. Such angle is symmetrical, that is, equal angles from the longitudinal axis of the sharp 224. The second distal edges 265 provide a somewhat less acute angle than the first distal edges 263. In some embodiments, the "lead in" angle may be about 20 degree, about 45 degrees, or about 65 degrees. By having a tip defined by two angles, a first, smaller "included angle" and a second, larger "lead in angle," allows the tip to meet several objectives. First, the small included angle allows the tip to pierce the skin with less trauma. Second, by broadening out to a larger angle, the overall length of the tip is reduced and strength of the tip is increased. Figure 14C illustrates a side view of the sharp 224 and illustrates the side walls 269. An additional angle, i.e., the "lead-out" angle is provided by the rising edge 267 of the sharp. The edge 267 provides the ability to separate the tissue to allow placement of the sensor 14. In other embodiments, a laser-cut sharp can be formed. In this manner, the laser can be used to form the wall opening or gap 262 and first-angled tip portion 264 and a second, steep-angled tip portion 266.

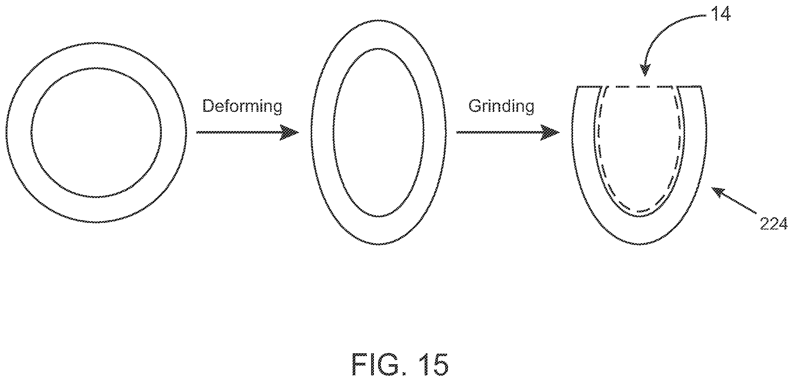

In another embodiment, sharp 224 may be formed from a standard hypodermic needle utilizing the method depicted in FIGURE 15. First, the hypodermic needle (having a circular cross-section) is cut to the desired length for sharp 224. Next, the hypodermic needle is compressed so that its cross-section is permanently deformed from a circular shape to an oval shape. The tip of the hypodermic needle is then ground to a bevel to produce a sharp point to reduce the required penetration force, as previously discussed. Finally, the top section of the needle is removed by appropriate techniques (e.g., grinding, electropolish, etc.). The resulting sharp 224 has a "U"-shaped configuration and provides ample space for the insertion of sensor 14. In some embodiments, the tip-grinding step and the compression step may be carried out in reversed order.

Due to the compression step, a user may initially start with a larger diameter hypodermic needle so that the finished sharp 224 will have similar dimensions to the previously described sharps.

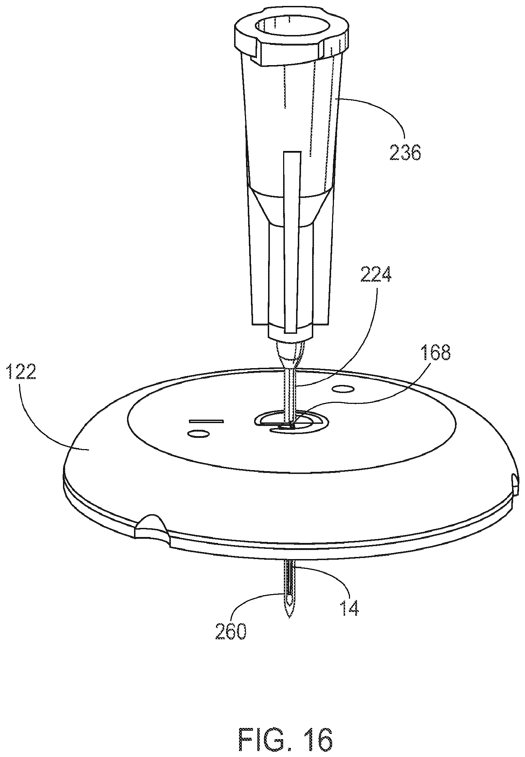

FIGURES 16-18 illustrate the position of on body housing 122 with respect to the needle hub 236 and sharp 224. The on body housing 122 can be configured to hold at least a portion of sensor 14 and on body electronics 1100 (also referred to herein as electronics 80). As illustrated in FIGURE 16, the sharp 224 extends through an aperture 168 in the on body housing 122. Thus, in some embodiments, the sharp 224 is uncoupled to on body housing 122. The distal portion of sensor 14 is positioned within the sharp 224. As further illustrated in FIGURE 17, on body electronics 1100 and sensor hub 123 are positioned within on body housing 122. Sensor 14 may include an optional positioning structure, or slit 127, which receives a positioning member, such as tab 129 of sensor hub 123. A power supply 82, such as a battery, *e.g*., a single use disposable battery, or rechargeable battery, is optionally provided.

FIGURE 17A illustrates a detail of sensor hub 123, which includes an aperture 190 through which sharp 224 and sensor 14 are configured to pass through. In some embodiments, aperture 190 is provided with an additional side channel 192 continuous with the aperture 190. Side channel 192 is positioned in the location in which the pointed tip 260 of the sharp 224 would first pass through the aperture. Ideally, the tip 260 passes through the aperture without contacting the sensor hub 123. However, if there is any misalignment, the tip 160 makes contact with the sensor hub 123 and may be damaged and/or it may become jammed or otherwise unable to pass through the aperture. The side channel 192 provides additional clearance for the tip 160 to pass through the aperture undamaged.

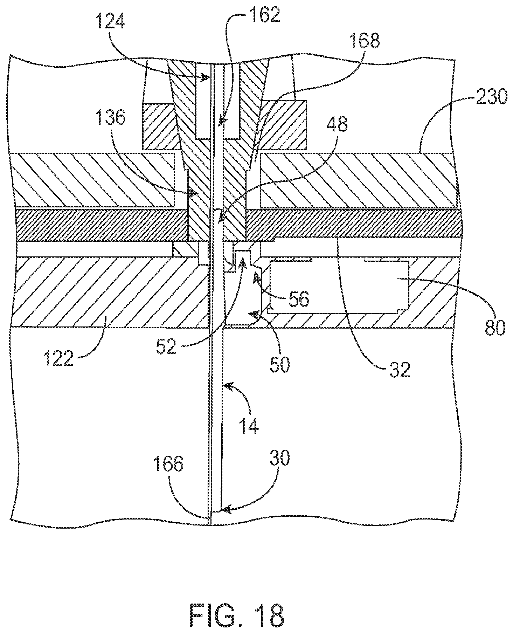

FIGURE 18 illustrates in cross-section the orientation of the on body housing 122 with respect to the sharp 224 of inserter 200. As discussed herein, sensor 14 is disposed in a substantially bent configuration in some embodiments, such that a portion of the sensor, *e.g*., the insertion portion 30 and the proximal retention portion 48, are substantially vertical (*e.g*., substantially aligned with the longitudinal axis of the inserter 200 and substantially perpendicular to the skin surface) and the contact portion 32 (shown in profile) is oriented in a substantially horizontal configuration, and in electrical contact with on body electronics 1100. The sensor tab 50 can be encapsulated in the plastic of the on body housing 122 and secured in place. The notch 56 provides further stability to the sensor 14, *e.g*., by allowing the sensor tab 50 to be encased by the material of the on body housing 122, and further provides a means for vertically orienting the sensor 14 during mounting, *e.g*., by allowing vertical positioning of the notch 56 with respect the on body housing 122.

The sensor 14, mounted with the on body housing 122, can be disposed within a recess of the carriage 230 such as a concave recess in the carriage 230. Alternatively, the sensor 14, mounted with the on body housing 122 can be disposed between the support structure and one or more projections extending from the wall of the sheath 242 (not shown). In yet another alternative, the sensor 14 mounted with the on body housing 122 can be held in position by a releasable friction fit coupling to the sharp 224. In this manner, the carriage need not have a recess within which the sensor mounted with the on body housing is disposed. In the initial configuration of the inserter 200 (see, *e.g*., FIGURE 7) the sharp 224 extends through a longitudinal aperture 268 formed in a carriage 230. In some embodiments, the aperture 268 is appropriately sized, such that neither the sharp 224 nor needle hub 236 is in contact with the carriage 230. Accordingly, the needle hub 236 (and sharp 224) on the one hand, and the carriage 230 and the on body housing 122, on the other hand, move simultaneously but independently from one another. In other embodiments, a friction fit may be provided between the aperture and the sharp.

The insertion portion 30 and proximal retention portion 48 of the sensor 14 are disposed within a longitudinal bore 162 within the sharp 224 (See, *e.g*., FIGURE 7). The proximal retention portion 48 is disposed within the longitudinal bore of the sharp 224 and provides additional stability to the mounting of the sensor 14 within the sharp 224. The longitudinal wall gap or opening 262 of sharp 224 is aligned with the sensor 14, such that the tab 50 and the contact portion 32 extend laterally outward from the sharp 224.

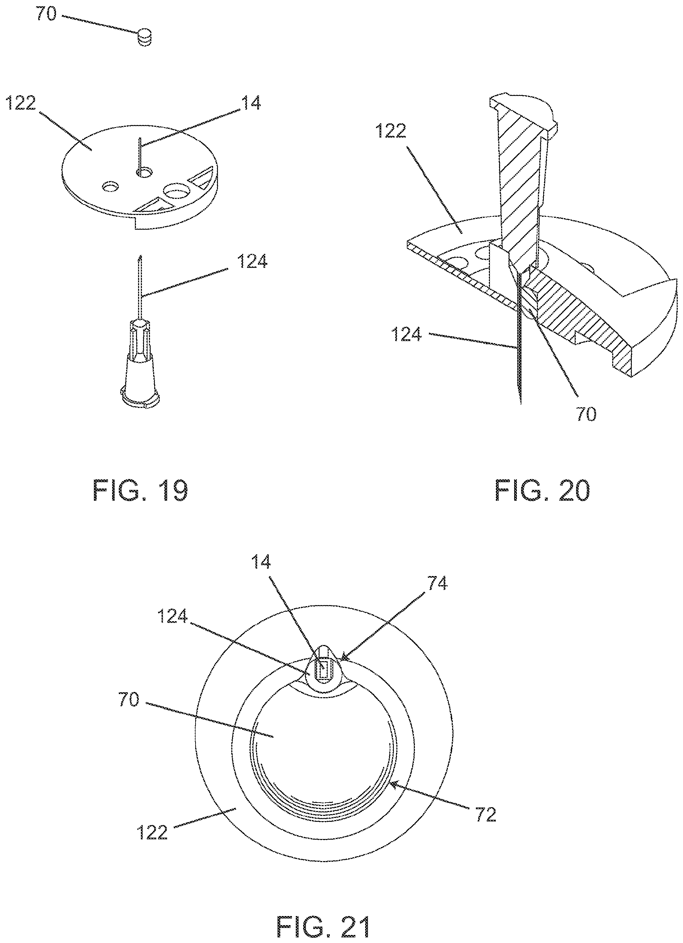

In some embodiments, a resilient member 70 may be included to provide frictional contact with the sharp 224 and/or the sensor 14. Such frictional contact provides additional stability between the on body housing 122 and sharp 224, as depicted in FIGURES 19-21. In some embodiments, resilient member 70 may be formed as a spherical, ovoid, cylindrical, cube-shaped member, etc. Resilient member 70 may be formed from any elastomeric material, e.g., molded plastic components, rubber, nitrile, viton, urethane, etc.

In some embodiments, resilient member 70 is press-fit into a recess, such as an eccentric bore 72 located in on, body housing 122 (FIGURE 21). When sharp 224 is inserted within an aperture in the on body housing 122, the resilient member 70 exerts a pressure on sharp 124 and sensor 14 to hold them firmly in groove 74. In some embodiments, groove 74 is a V-sliape. Alternatively, groove 74 may be U-shaped depending on the configuration of sensor 14 and sharp 224. In some embodiments, resilient member 70 is provided with a flattened or recessed surface which abuts sharp 224.

The sensor 14, mounted with the on body housing 122, is carried by the carriage, *e.g*., disposed within the concave recess 232a in the carriage 230, as described hereinabove (see, *e.g*., FIGURES 16-21). In the initial configuration of the inserter 200 (see, *e.g*., FIGURE 7), the sharp 224 extends through a longitudinal aperture formed in the carriage 230. In some embodiments, the aperture is appropriately sized, such that neither the sharp 224 nor needle hub 236 is in contact with the carriage 230. In other words, in some embodiments a clearance may be provided between the surfaces of the carriage and the sharp and needle hub. In some cases, sharp 224 is capable of substantial lateral movement or "play" with respect to aperture. Accordingly, the needle hub 236 (and sharp 224) on the one hand, and the carriage 230 and the on body housing 122, on the other hand, can move simultaneously but independently from one another.

Referring back to FIGURE 17, the insertion portion 30 and proximal retention portion 48 of the sensor 14 are disposed within a longitudinal bore of the sharp 224. The proximal retention portion 48 is disposed within the longitudinal bore 225 of the sharp 224 and provides additional stability to the disposition of the sensor 14 within longitudinal bore 225 of the sharp 224. The longitudinal wall gap of sharp 224 is aligned with the sensor 14, such that the tab 50 and the contact portion 32 extend laterally outward from the sharp 224.

With continued reference to FIGURE 7, an optional sheath 242 is positioned within housing 202, having an annular configuration and including a circumferential recess 244 in which a retraction spring 246 is positioned. The distal portion of spring 246 contacts a spring retention portion 248 in sheath 242. The proximal portion of spring 246 contacts one or more tabs 250 extending laterally outwardly from actuator 214. In the initial configuration, the spring 246 may be in a semi-compressed state, *i.e*., not fully compressed, nor fully extended. It is understood that sheath 242 may be omitted from inserter 200, and a recess, such as recess 244, provided within housing 202. Similarly, recess 244 may be omitted entirely, and spring 246 or other actuator may be disposed between stops in housing 202.

Depression of the actuator 214 causes distal longitudinal movement of the carriage 230 and sharp 224, from a proximal position (space apart from the skin of the subject) to a distal position (closer to the skin of the subject). During such downward, distal movement, spring 246 is further compressed between spring retention portion 248 and flanges 250.

As illustrated in FIGURE 8, depression of the contact surface 226 moves the actuator side walls 228 and the tabs 250 downwardly distally against the bias of spring 246. Contact of the side wall 228 of the actuator 214 with the upper surface of the carriage 230 during depression of the actuator 214 imposes a downward force and consequential distal movement of the carriage 230. As the sharp 224 is urged distally, it carries the sensor insertion portion 30 into the subcutaneous portion of the subject's skin S.

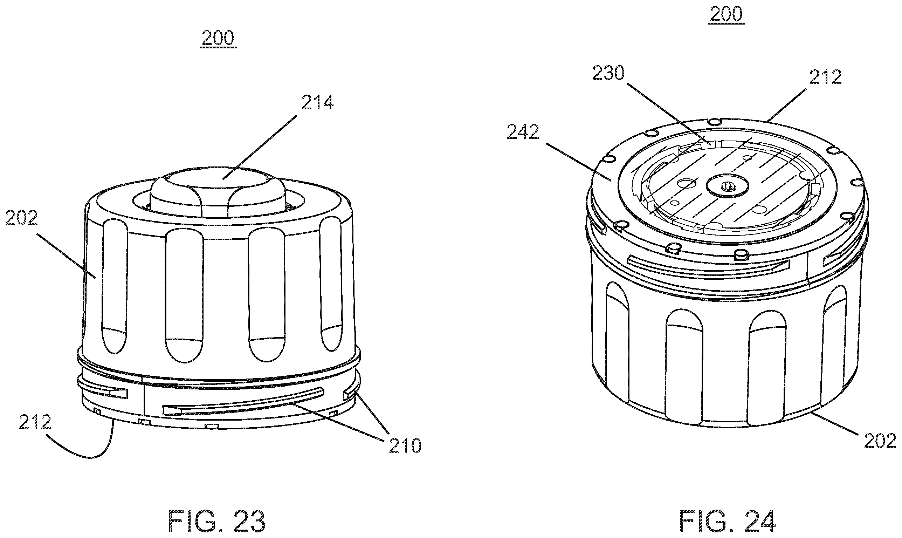

As illustrated in FIGURE 7, flanges 270 are disposed in the inner wall of sheath 242. When carriage 230 reaches a distal position, as shown in FIGURE 8, the flanges 270 engage the proximal (upper) surface of the carriage 230, and thereby inhibit proximal movement of the carriage 230 (see also FIGURE 24). The distal (lower) surface of the on body housing 122 engages the upper surface of adhesive pad 218, thereby becoming adhered to the skin surface S of the subject. As the flanges 270 engage the carriage 230, the flanges 270 also engage fingers 274 disposed on the proximal face of the carriage 230. Fingers 274 are pivoted inwards by flanges 270. Such pivoting of fingers 274 causes fingers 274 to become disengaged from retention tab 250 on actuator 214. Spring 246 is thereby permitted to decompress and expand, and thereby provide an upward force on actuator 214. If the user or some apparatus provides no downward force, or minimal downward force to overcome the bias of spring 246, the actuator 214, along with needle hub 236 and sharp 224 move proximally, withdrawing the sharp 224 from the skin S of the subject.

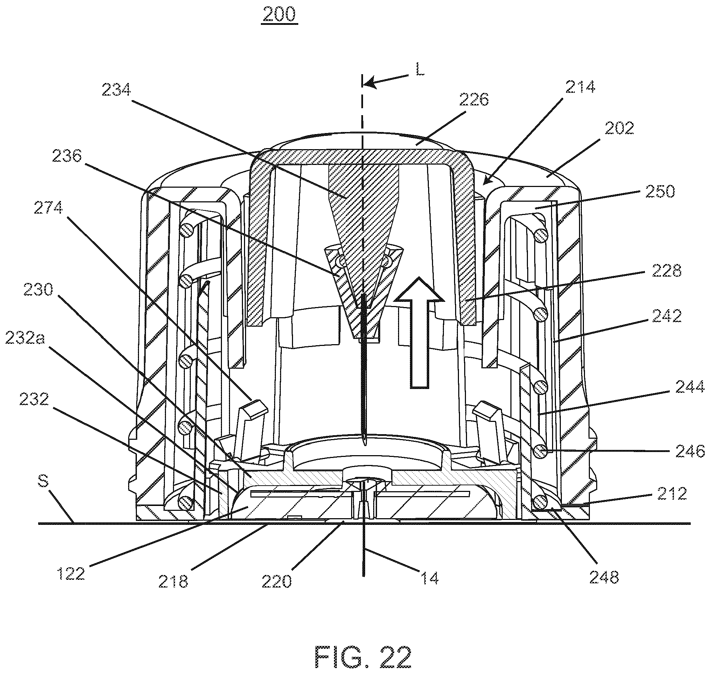

As shown in FIGURES 22 and 23, the actuator 214 and coupled sharp 224 advances to a more proximal position than at the initial configuration illustrated in FIGURES 5 and 7 due to the decoupling of actuator 214 from carrier 30. Thus the sharp 224 retracts from a distal position to a proximal position after installation of the on body housing 122 and insertion of at least a portion of the sensor. T

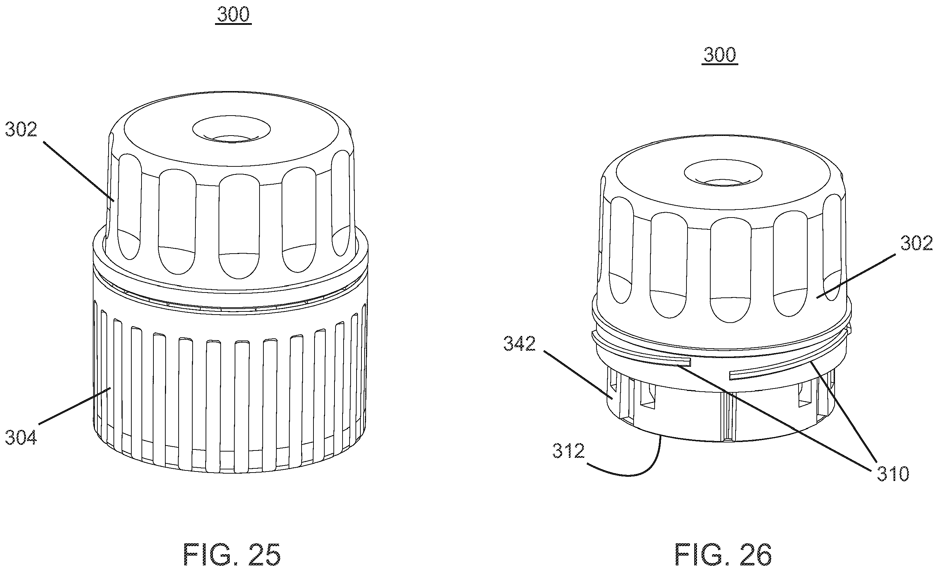

A further embodiment of an inserter is illustrated in FIGURES 25-39 and designated inserter 300. In some embodiments, inserter 300 has a maximum diameter of about 30mm to about 60 mm, e.g., about 40 mm, about 43 mm, about 43.5 mm, about 50.5 mm, about 54.5 mm, etc. In some embodiments, inserter 300 has a maximum height of about 40 mm to about 80 mm, e.g., about 44 mm, about 46 mm, about 50 mm, about 53 mm, about 67 mm, about 71 mm, etc. Such height is defined by the total length of the housing 302 and the sheath 342. In some embodiments, inserter 300 has a volume of about 35 cm³ to about 110 cm³, *e.g.*, about 40 cm³, about 41 cm³, about 50 cm³, about 60 cm³, about 61 cm³, about 62 cm³, about 69 cm³, about 70 cm³, about 79 cm³, about 90 cm³, about 106 cm³, etc. Such dimensions are defined by the total length of the housing 302 and the sheath 342.

As illustrated in FIGURES 25-26, inserter 300 in certain embodiments includes, *e.g*., a handle 302, a sheath 342, and a removable distal cap 304 for maintaining a sterile environment for the medical device and sharp housed therein. FIGURE 26 illustrates that distal cap 304 is removed from handle 302. Distal cap 304 is secured to handle 302 by one of a number of securement means, e.g., by use of threads 310. Sheath 342 defines a distal surface 312 for placement on the skin of a subject. Inserter 300 may be utilized to advance a medical device into the skin of the subject. In some embodiments, handle 302 is advanced relative to sheath 342 in order to advance the medical device into the skin of the patient.

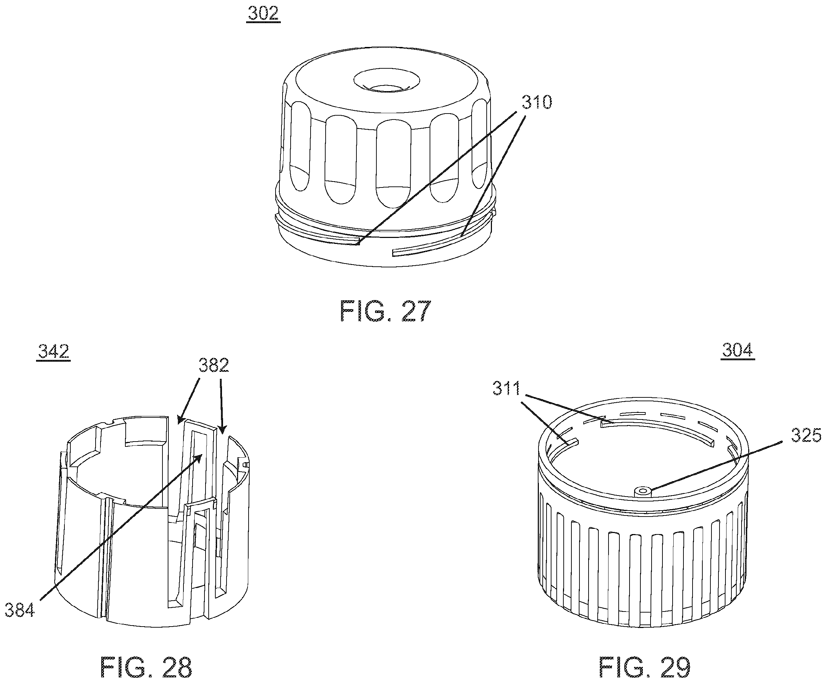

The components of inserter 300 in certain embodiments are illustrated in FIGURES 27-32. As illustrated in FIGURE 27, handle 302 may include threads 310 for attachment of cap 304 via threads 311 (as illustrated in FIGURE 29). It is understood that other securement techniques, such as a snap-fit or friction-fit may be used to secure cap 304. Cap 304 may include a receptacle 325 for positioning of the sharp 324. Sheath 342, as illustrated in FIGURE 28, includes longitudinal notches 382.

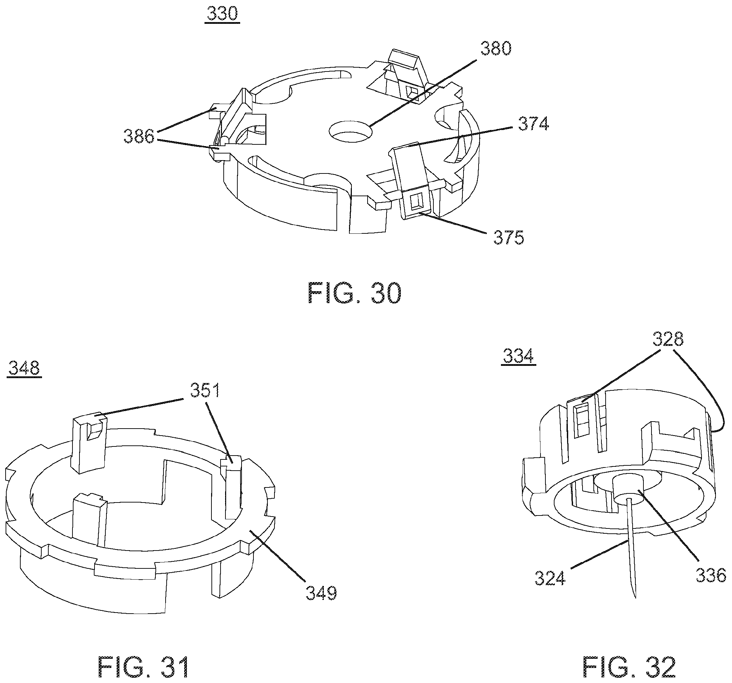

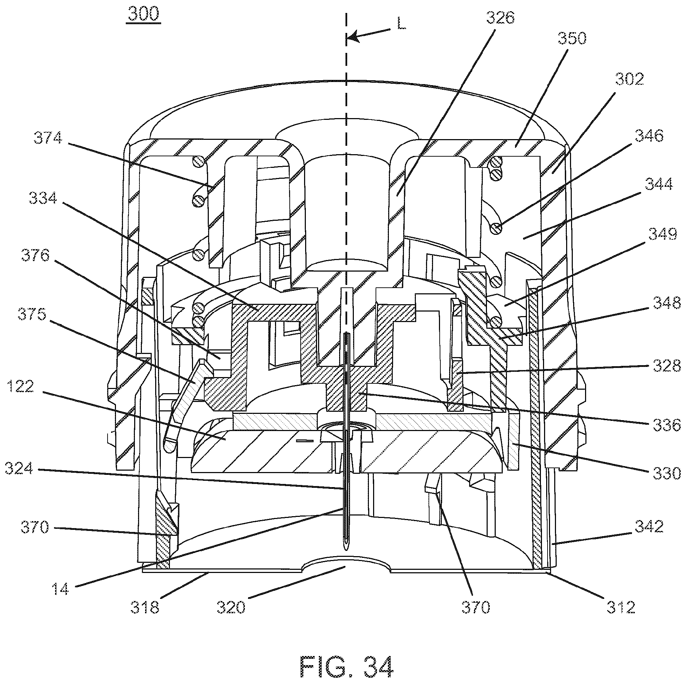

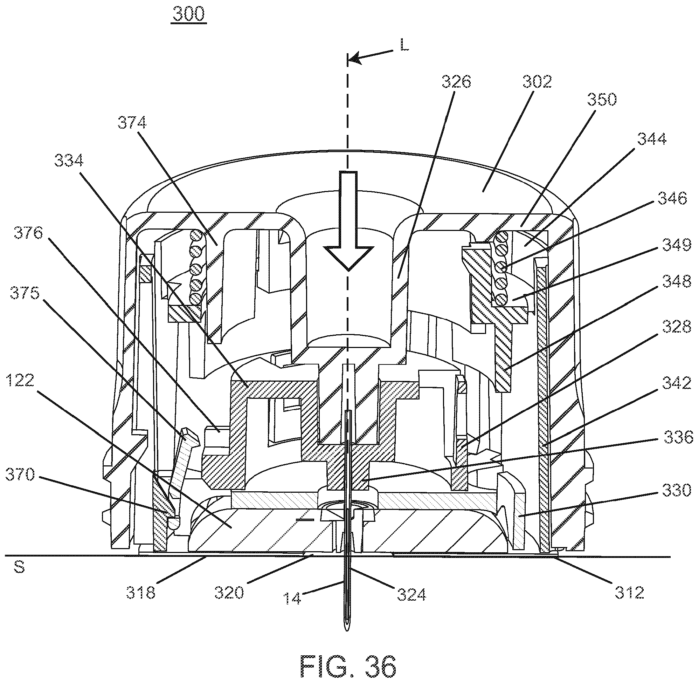

Projections 386 on carriage 330, as illustrated in FIGURE 30, are configured to engage sheath to secure carriage 330 within the inserter 300, thereby preventing release of the carriage 330 from the inserter 300. When the projections 386 of carrier reach the bottom of the notches 382, such bottom surface acts as the retention portion that prevents the carriage 330 from falling out of the inserter 300. Projections 375 engage with the triangular latch features 370 of the sheath 342 as illustrated in FIGURES 34 and 36.

Carriage 330 also is provided with fingers 375 which engage a shoulder wall 376 of sharp 334 (as illustrated in FIGURE 34), as will be described in greater detail herein.

In certain embodiments, inserter 300 also includes a spring retention component 348, as illustrated in FIGURE 31. Spring retention component 348 defines an upper surface 349, which engages spring 346 (as illustrated in FIGURE 34). Spring retention component 348 also includes locking towers 351 including projections, which engage apertures 328 of needle carrier 334 to prevent accidental deployment of the sharp 324 after use of the inserter 300 is completed.

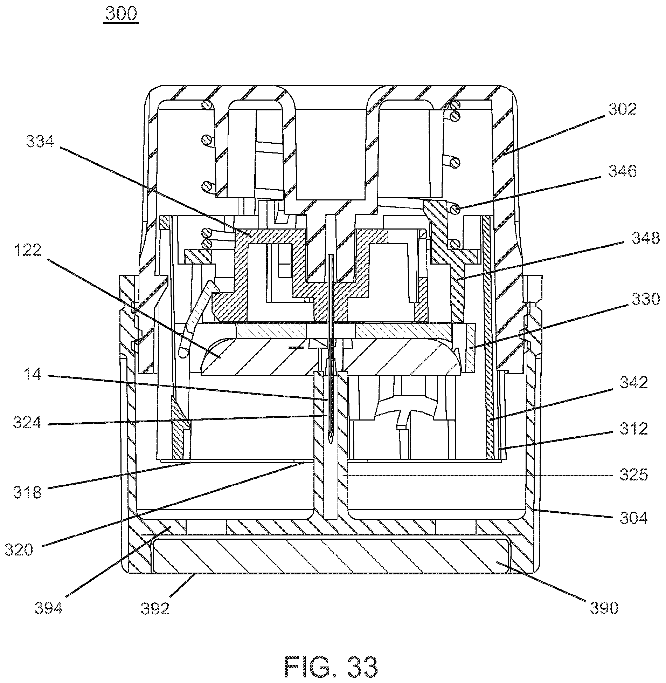

Inserter 300 is illustrated in cross-section in FIGURE 33 prior to use. Cap 304 is attached to the distal portion of inserter 300, via securement means, such as inter-engagement of threads 310 and 311. Cap 304 includes a desiccant tablet 390; a seal, such as a foil seal 392; and a Tyvek^{®} layer 394, which allows breathability between the desiccant tablet 390 and the interior of the inserter 300.

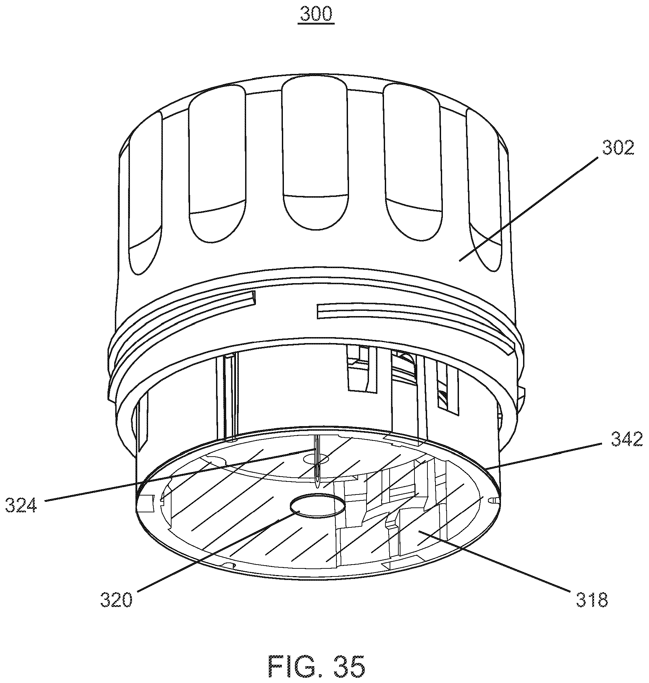

As illustrated in FIGURE 34, the inserter 300 includes an initial configuration in which the handle 302 is disposed in a proximal position with respect to the sheath 342. In such configuration, the sharp 324 is disposed in a configuration spaced apart from the aperture 320 of the adhesive layer 318. The distal portion of inserter 300 is illustrated in FIGURE 35.

With continued reference to FIGURE 34, the longitudinal axis L of the inserter 300 is illustrated. Extending distally from the upper surface of handle 302 is an inner wall portion 326 and intermediate wall portion 374. Support member 334 extends from wall portion 326 and supports needle hub 336, from which sharp 324 extends longitudinally within the inserter 300. In some embodiments, the sharp is supported at an oblique angle, e.g., between 0° and 90° with respect to the skin surface.

Sheath 342 is positioned within handle 302, having an annular configuration in which a retraction spring 346 is positioned. The distal portion of spring 346 contacts a surface 349 of spring retention component 348. The proximal portion of spring 346 contacts the inner surface 350 of handle 302. In the initial configuration, the spring 346 is in an extended or semi-extended configuration.

FIGURE 36 illustrates inserter 300 in cross-section, during insertion. Depression of handle 302 with respect to sheath 342 against the bias of spring 346 causes distal longitudinal movement of the carriage 330 and sharp 324, from a proximal position towards a distal position. During such downward, distal movement, spring 346 is compressed between surface 349 of spring retention component 348 and surface 350 of handle 302. As the sharp 324 is urged distally by housing 302, it carries the sensor insertion portion 30 of sensor 14 into the subject's skin S.

As carriage 330 reaches a distal position, the distal surface of the on body housing 122 engages the upper surface of adhesive pad 318, thereby becoming adhered to the skin surface S of the subject. Also, flange 370 engages fingers 375 disposed on the carriage 330. Fingers 375 are pivoted outwards by flanges 370 in direction T. Such pivoting of fingers 375 causes fingers 375 to become disengaged from slots 376 in intermediate housing walls 374. Carriage 330 is thereby disengaged from handle 302 and needle carrier 334.

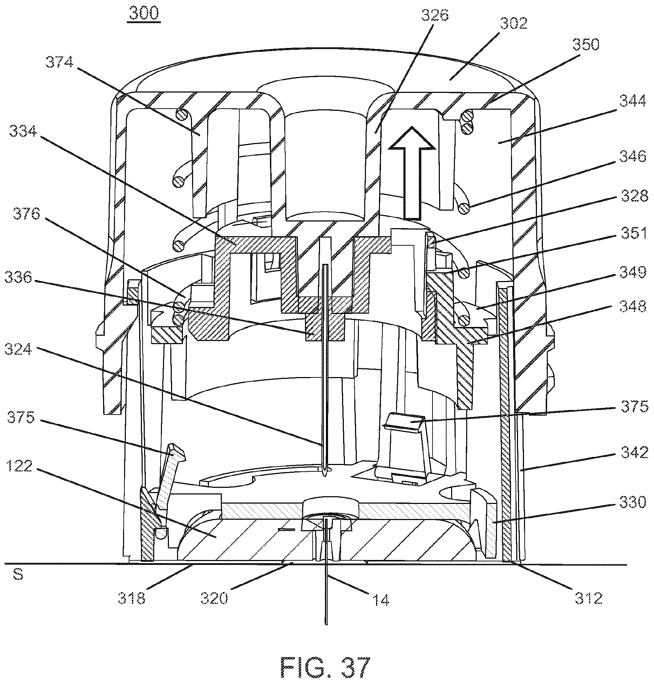

As illustrated in FIGURE 37, handle 302, along with needle hub 336 and sharp 324 are permitted to move proximally, while the sheath 342 and on body housing 122 remain adjacent to the skin of the subject. If the user or some apparatus provides no downward force, or minimal downward force to the handle 302 to overcome the bias of spring 346, spring 346 is permitted to expand, thereby withdrawing the sharp 324 from the skin S of the subject.

Upon reaching the proximal position, flanges 328 on needle carrier 334 engage locking towers 351 of needle floor component 348. The inter-engagement of flanges 328 and locking towers 351 prevents inadvertent deployment of sharp 324 after installation of the medical device.

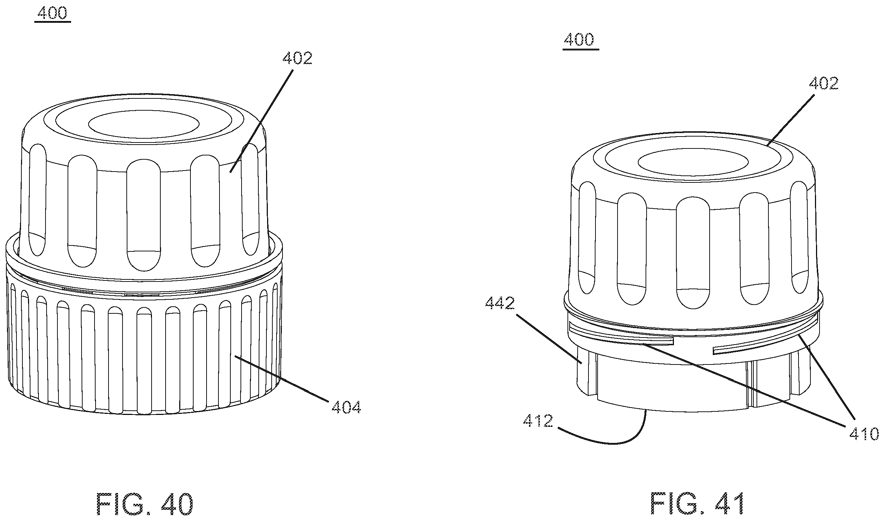

TA further embodiment of an inserter is illustrated in FIGURES 40-50, and designated inserter 400. In some embodiments, inserter 400 has a maximum diameter of about 30mm to about 60 mm, *e.g*., about 40 mm, about 43 mm, about 43.5 mm, about 50.5 mm, about 54.5 mm, etc. In some embodiments, inserter 400 has a maximum height of about 40 mm to about 80 mm, e.g., about 44 mm, about 46 mm, about 50 mm, about 53 mm, about 67 mm, about 71 mm, etc. In some embodiments, inserter 400 has a volume of about 35 cm³ to about 110 cm³, *e.g*., about 40 cm³, about 41 cm³, about 50 cm³, about 60 cm³, about 61 cm³, about 62 cm³, about 69 cm³, about 70 cm³, about 79 cm³, about 90 cm³, about 106 cm³, etc. The maximum height is measured from the top of the housing 402 to the distal surface 412. The volume is measured as the combined volume of the housing 402 and the sheath 442 in an expanded position.

Inserter 400 generally includes, *e.g*., a handle 402, sheath 442, and a removable distal cap 404 for maintaining a sterile environment for the medical device and sharp housed therein. As illustrated in FIGURE 41, distal cap 404 is shown removed from handle 402. Distal cap 404 is detachably secured to handle 402, *e.g*., by use of threads 410. Sheath 442 includes a distal surface 412 for placement on the skin of a subject. Inserter 400 may be utilized to advance a medical device into the skin of the subject. In some embodiments, handle 402 is advanced relative to sheath 442 in order to advance the medical device distally and into the skin of the patient.

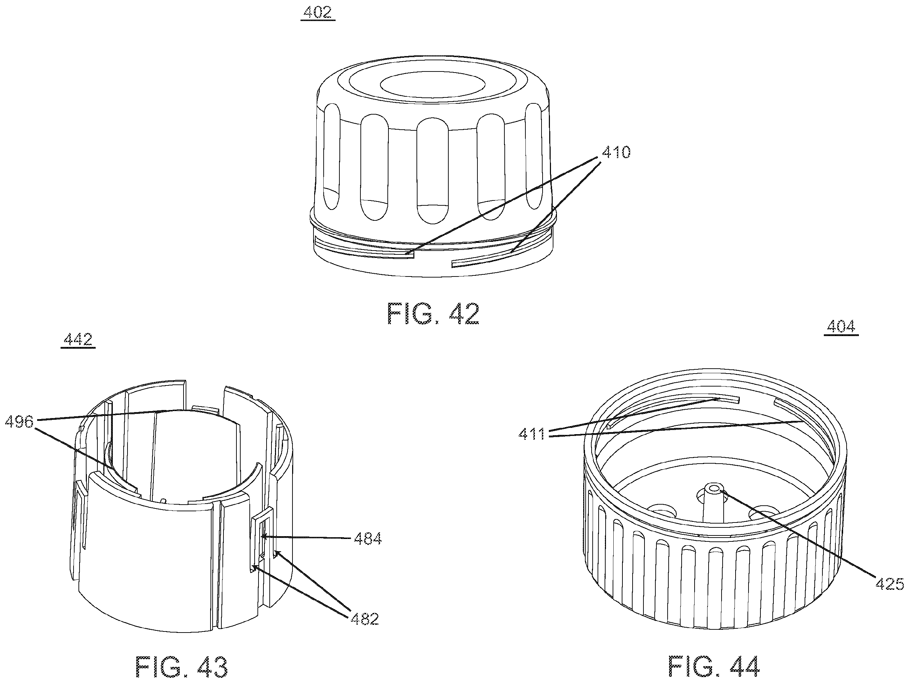

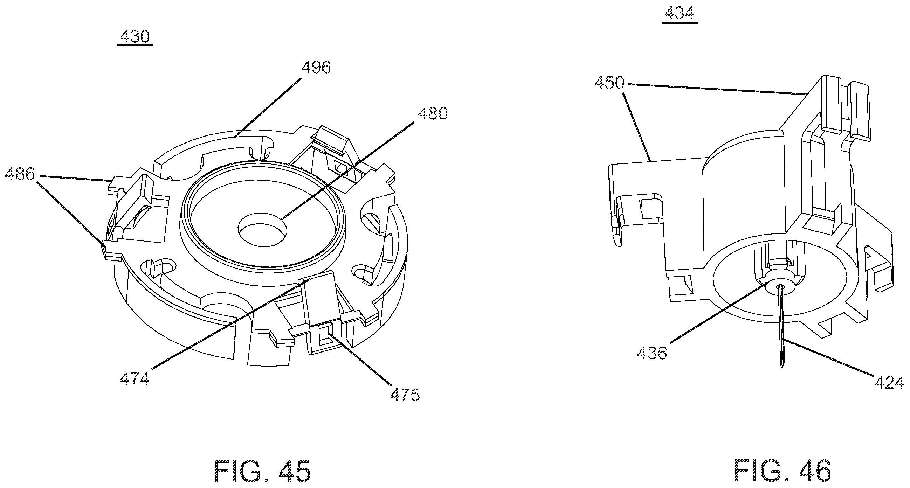

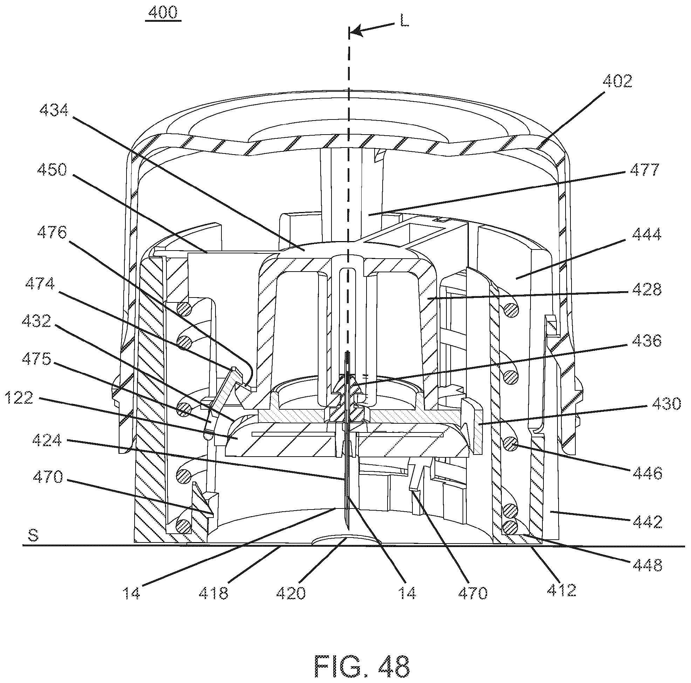

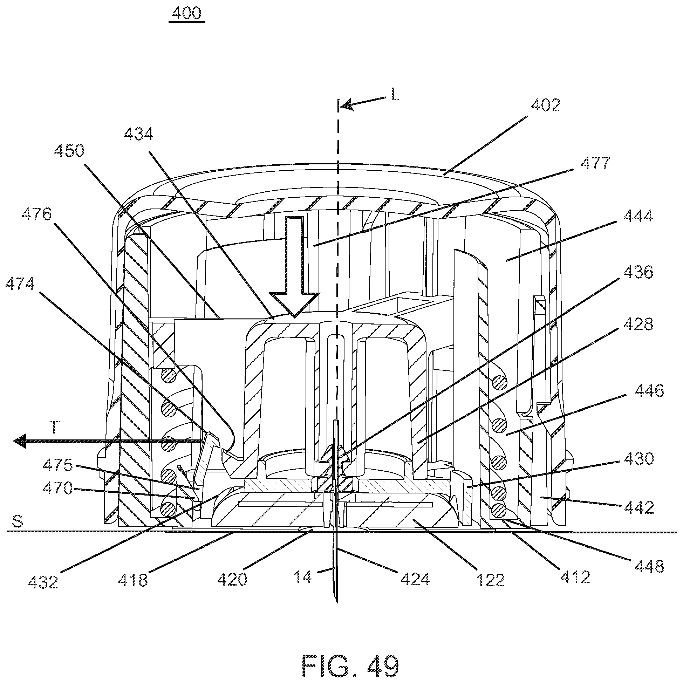

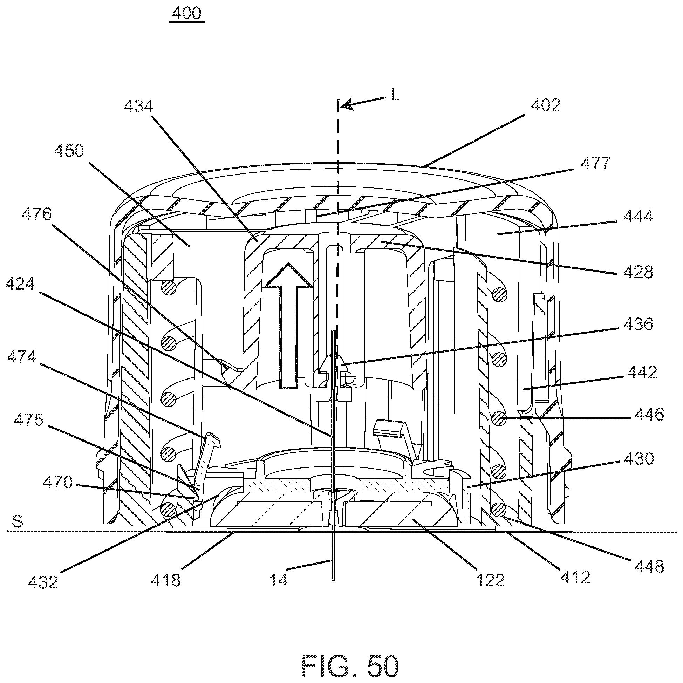

The components of inserter 400 are illustrated in FIGURES 42-46. As illustrated in FIGURE 42, handle 402 includes threads 410 for attachment of cap 404 via threads 411 (as illustrated in FIGURE 44). Cap 404 may include a receptacle 425 for positioning of the sharp 424. Sheath 442, as illustrated in FIGURE 43, includes longitudinal notches 482. Projections 486 on carriage 430, as illustrated in FIGURE 45, are configured to engage sheath 442 to secure carriage 430 within inserter 400, thereby preventing release of the carriage from the inserter. Sheath 442 also includes notches 484 which receive projection 475 of carriage 430. The bottom of the notches acts as the retention portion that prevents the carriage 430 from falling out of the inserter 400. Projections 475 engage with the latch features 470 of the sheath 442 as illustrated in FIGURES 49 and 50. Carriage 430 also is provided with fingers 474 which engages a shoulder wall 476 of needle carrier 436, as illustrated in FIGURES 48-49, and as will be described in greater detail herein.

Sheath 442 also includes a spring retention portion 448, provided at the distal end of circumferential notch 496, as illustrated in FIGURE 48. Needle carrier 434, as illustrated in FIGURE 46, includes wings 450, which provide an upper engagement surface for spring 446. Wings 450 also include a shoulder 476 for engagement with fingers 474 of carriage 430.

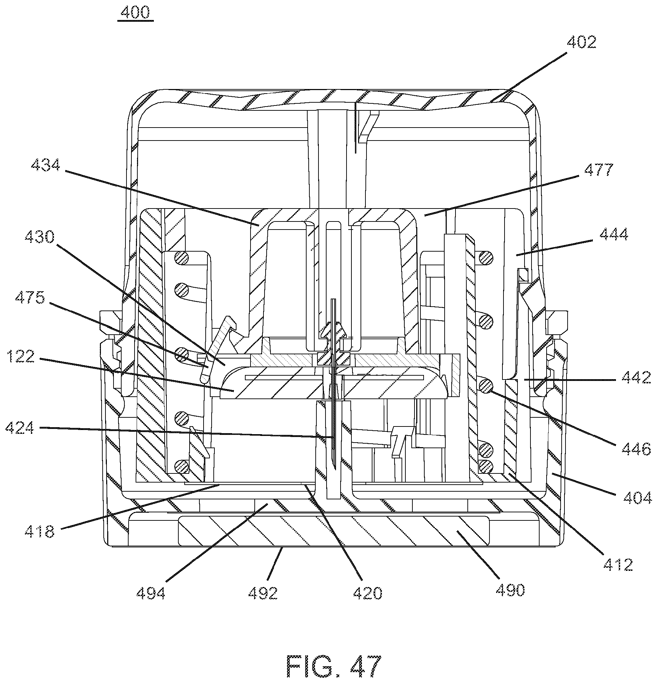

Inserter 400 is illustrated in cross-section in FIGURE 47 in a state prior to use and prior to removal of cap 404, which is shown attached to the distal portion of handle 402, via inter-engagement of threads 410 and 411. Cap 404 includes a desiccant tablet 490, a seal such as a foil seal 492, and a Tyvek^{®} layer 494, which allows breathability between the desiccant tablet 490 and the interior of the inserter 400.

As illustrated in FIGURE 48, the inserter 400 is shown in an initial configuration in which handle 402 is disposed in a proximal position with respect to the sheath 442. In such configuration, the sharp 424 is disposed in a configuration spaced apart from the aperture 420 of the adhesive layer 4 18. The longitudinal axis L of the inserter 400 is illustrated. Extending distally from the upper surface of handle 402 is inner wall 475. The distal end portions of wall 475 provide a downward force on the carriage 430 upon depression of the handle 402 by a user. Alternatively, instead of handle having a distally extending inner wall, the carriage can include one or more upwardly extending walls or projections (not shown). The one or more upwardly extending inner walls or projections can have a length sufficient to either contact the upper surface of 402 or, alternatively, contact corresponding downwardly extending inner walls of the handle 402. In this manner, depression of handle 402 by a user provides a downward force on the one or more upwardly extending walls or projections of the carriage to advance the carriage (and on-body unit) distally to an installation and insertion position. (See FIGURES 49-50.) In one embodiment a downwardly extending wall of the handle 402 and a corresponding upwardly extending wall of the carriage are aligned such that depression of the handle 402 by a user allows the upwardly extending wall and the downwardly extending wall to make direct contact, thereby permitting the carriage 430 and on-body unit to advance distally. In such embodiment, the downwardly extending inner wall of the handle has a distal end that is disposed proximally of the proximal most end of sheath 442.

Needle carrier 434 can be axially moveable within handle 402. Needle carrier 434 supports needle hub 436, from which sharp 424 extends longitudinally within inserter 400. In some embodiments, sharp 424 is supported at an oblique angle, *e.g.,* between and including about 0° and 90° with respect to the skin surface. Initially, needle carrier 434 is coupled to carriage 430 via inter-engagement of fingers 474 of carriage 430 with shoulder 476 of needle carrier 434. Spring 446 is disposed between spring retention portion 448 of sheath 442 and wings 450 (FIGURE 46) of needle carrier 434. Initially, spring 446 is in an expanded or semi-expanded state while handle 402 is disposed proximally from sheath 442. In another exemplary embodiment, needle carrier 434 can be secured to handle 402, for example, secured to downwardly extending inner wall 475. In this manner, needle carrier and carriage 430 are longitudinally moveable along the line defined by L shown in FIGURE 48 with respect to sheath 442. In this regard, needle carrier 434 includes one or more apertures to receive one or more downwardly extending inner walls 475 of handle 402. In some embodiments, neither the needle carrier 434 nor the carriage 430 are in slidable contact with sheath 442, e.g., spaced apart from sheath 442, during longitudinal movement of the needle carrier 434 and/or carriage 430.

FIGURE 49 illustrates inserter 400 in cross-section, during insertion. Depression of handle 402 with respect to sheath 442, against the bias of spring 446, causes distal longitudinal movement of the carriage 430 and needle carrier 434, from a proximal position towards a distal position. During such downward proximal movement, spring 446 is compressed between spring retention portion 448 and wings 450 (FIGURE 46) of needle carrier 434. As the sharp 424 is urged distally, it carries the sensor insertion portion 30 of sensor 14 (FIGURE 17) into the subcutaneous portion of the subject's skin S.

As carriage 430 reaches a distal position (close to the skin of the subject), the distal surface of the on body housing 122 engages the upper surface of adhesive pad 418, thereby becoming adhered to the skin surface S of the subject. Flange 470 engages fingers 474 disposed on the carriage 430. Fingers 474 are pivoted outwards by flanges 470 in direction T. Such pivoting of fingers 474 causes fingers 474 to become disengaged from shoulder 476 of needle carrier 434. Needle carrier 434 is thereby disengaged from carriage 430. Such pivoting of fingers 474 also engages opening in 474 with flange 470, thus locking carriage 430 in the distal position.

As illustrated in FIGURE 50, disengagement of the needle carrier 434 from the carriage 430 permits the spring 446 to expand, thereby advancing the needle carrier 434 to a proximal position (away from the skin of the subject) and withdrawing the sharp 424 from under the skin surface S of the subject while leaving the sensor 14 in the skin. Once the sharp 424 has been withdrawn from the subject, it is no longer accessible from the distal portion of the inserter 400 and unable to make contact by accident with the subject's skin because it is positioned at a proximal position within the carrier handle 402.

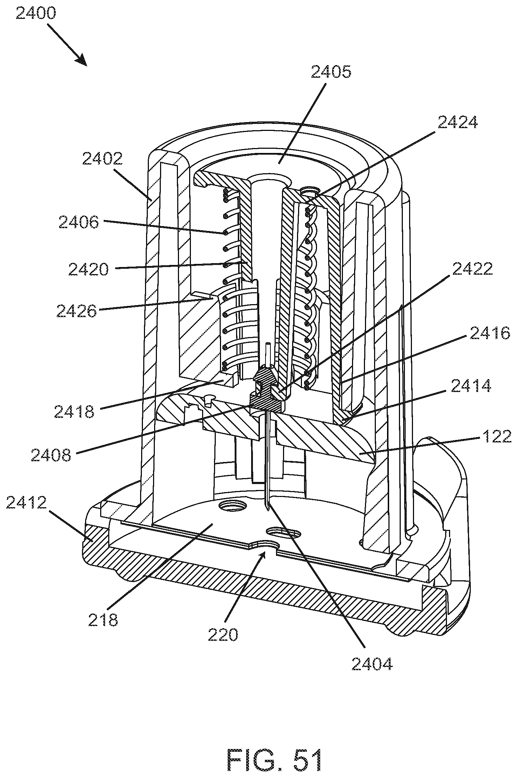

An inserter 2400 in accordance with another exemplary embodiment is illustrated in FIGURE 51. In some embodiments, inserter 2400 has a maximum diameter, of about 30mm to about 60 mm, e.g., about 40 mm, about 43 mm, about 43.5 mm, about 50.5 mm, about 54.5 mm, etc. In some embodiments, inserter 2400 has a maximum height of about 40 mm to about 80 mm, e.g., about 44 mm, about 46 mm, about 50 mm, about 53 mm, about 67 mm, about 71 mm, etc. In some embodiments, inserter 2400 has a volume of about 35 cm³ to about 110 cm³, *e.g.,* about 40 cm³, about 41 cm³, about 50 cm³, about 60 cm³, about 61 cm³, about 62 cm³, about 69 cm³, about 70 cm³, about 79 cm³, about 90 cm³, about 106 cm³, etc. The height is measured from the distal surface of the housing 2402 (adjacent to adhesive 218) to the top surface. The volume is measure by the volume of the housing 2402.

With reference to FIGURE 51, inserter 2400 includes a housing 2402 and a removable distal cap 2412 for protecting the medical device and sharp housed therein. Housing 2402 and distal cap 2412 may be fabricated from any suitable materials such as metal, plastic, etc. In some embodiments, cap 2412 may be fabricated from a polymer or plastic material.

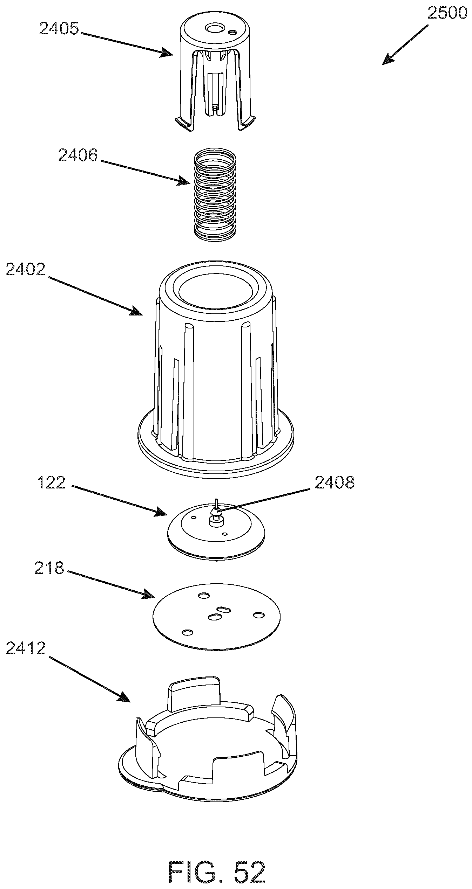

An exploded view of the components of inserter 2400 is illustrated in FIGURE 52. As shown, inserter 2400 generally comprises plunger 2405, spring 2406, housing 2402, sharp 2404 (not shown in FIGURE 52, on body housing 122, sharp holder 2408, adhesive patch 218, and cap 2412 when fully assembled.

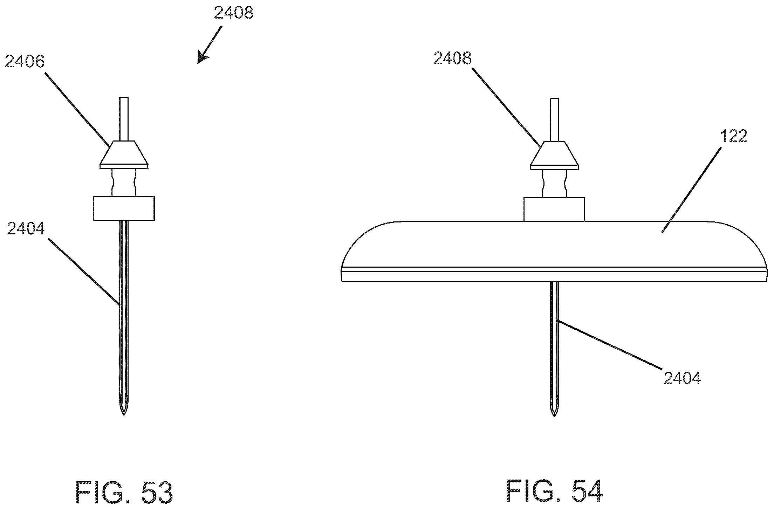

A more detailed view of sharp holder 2408 is shown in FIGURE 53. Needle holder 2408 retains sharp 2404 in a fixed position with respect to itself within inserter 2400, thereby allowing it to safely penetrate a subject's skin during later use.

To assemble inserter 2400, sharp 2404 and hub 2408 are inserted through an opening in on body housing 122 as shown in FIGURE 54. Needle holder 2408 prevents sharp 2404 from being fully inserted through on body housing 122. In some embodiments, on body housing 122 includes an analyte sensor 14 and on body electronics 1100.

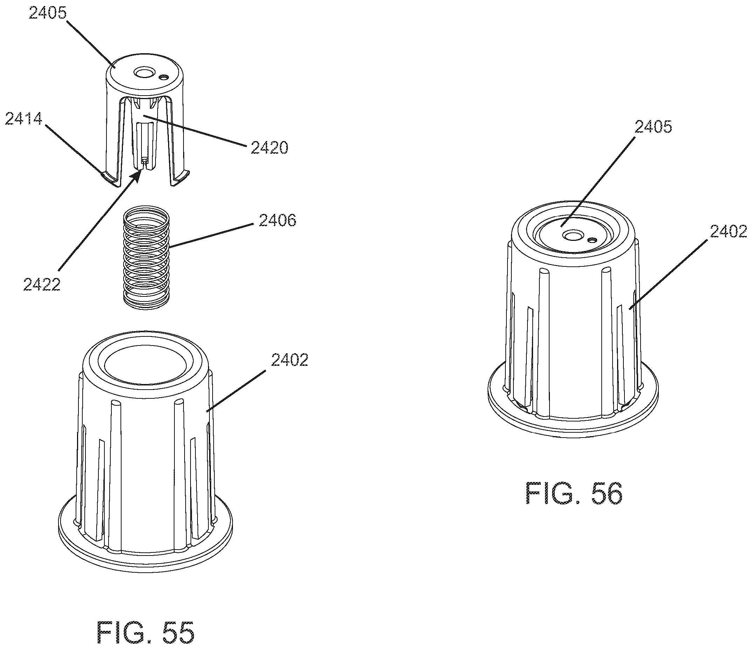

Next, plunger 2405, spring 2406, and housing 2402 are assembled as shown in FIGURES 55-57. Plunger 2405 contains a spring retention member which is inserted through the center of spring 2406. Lip 2414 of plunger 2405 engages inner wall 2416 of housing 2402 when assembled (FIGURE 51). This causes spring 2406 to be contained between lip 2418 of housing member 2402 and the bottom surface 2424 of plunger 2405. The resulting sub-assembly of inserter 2400 shown in allows plunger 2405 to move between a proximal position, with spring 2406 in a preloaded condition, and a distal position, wherein bottom surface 2424 engages wall 2426 of housing 2402.

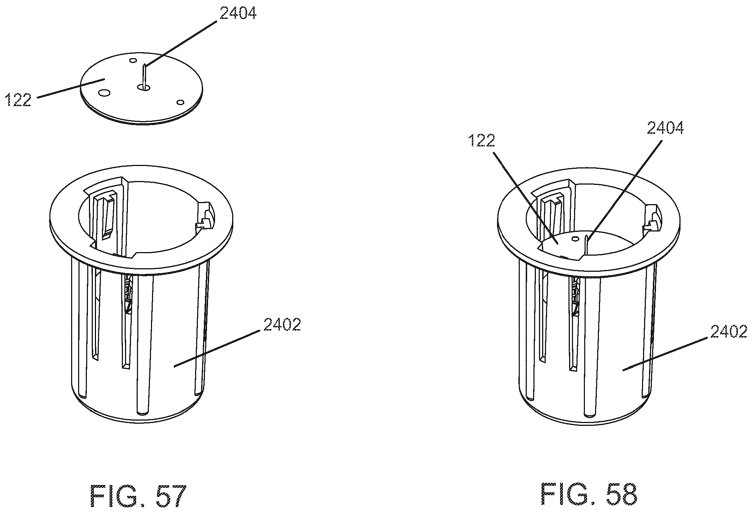

The on body housing assembly shown in FIGURE 54 is then inserted into the inserter sub-assembly shown in FIGURES 55-57. As shown in FIGURE 57, on body housing 122 is inserted into housing 2402 with the tip of sharp 2404 pointing away from plunger 2405. The resulting assembly is depicted in FIGURE 58. As shown in FIGURE 51, grooves on sharp holder 2408 engage tabs 2422 on plunger 2405. The on body housing 122 is axially retained in the housing 2402 by the housing arms detent features 2440.

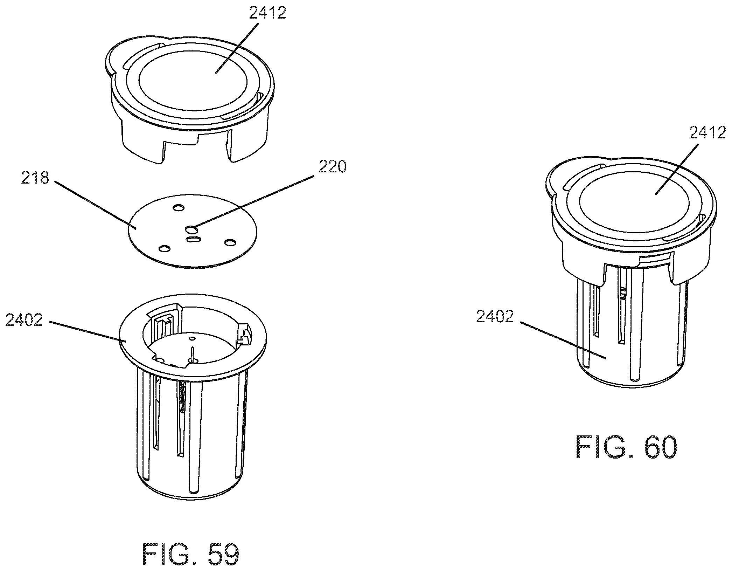

Finally, adhesive patch 218 is placed over the opening of housing 2402 and cap 2412 is friction fit over housing 2402 as shown in FIGURE 59. The fully assembled inserter 2400 is depicted in FIGURE 60. In some embodiments, adhesive pad 218 has an adhesive material on both faces. A central aperture 220 may be provided in adhesive pad 218 to allow sharp 2404 to be deployed into the skin of a subject. During insertion, sharp 2404 passes through aperture 220 and into the skin of the subject carrying at least the sensor with it.

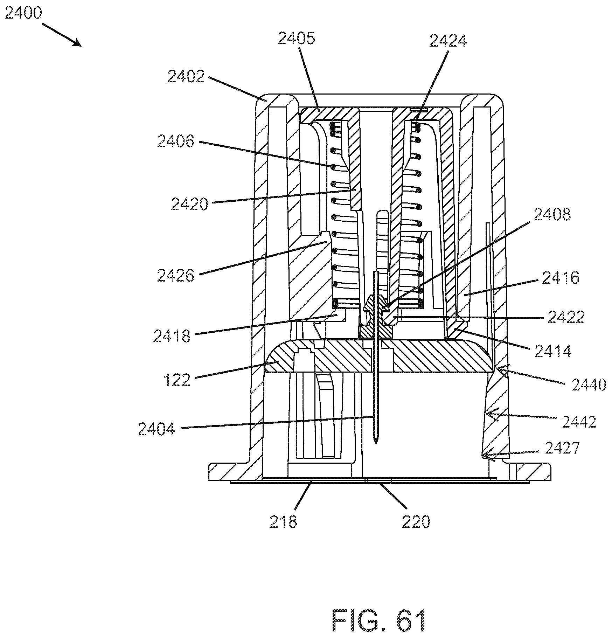

FIGURE 61 illustrates inserter 2400 in cross-section, in an initial configuration prior to use, after removal of the distal cap 2412. As shown, sharp 2404 extends longitudinally within the inserter 2400. In some embodiments, sharp 2404 is supported at an oblique angle, e.g., between and including about 0° and 90° with respect to the skin surface.

In some embodiments, sharp 2404 is provided with a substantially cylindrical configuration defining an interior bore, *e.g.,* a rigid cylindrical member or a hypodermic-style needle. Sharp 2404 may also be provided with an elongated longitudinal opening or gap in the wall of the sharp 2404. In some embodiments, sharp 2404 is a fabricated from a sheet of metal, and folded into a substantially "V" or "U" or "C" configuration in cross-section to define the longitudinal recess.

Depression of plunger 2405 causes distal longitudinal movement of on body housing 122 and sharp 2404, from a proximal position to a distal position. During such downward, distal movement, spring 246 is further compressed between lip 2418 and bottom surface 2424. Detent 2440 provides a minimum force threshold to overcome before on body housing 122 can continue on its downward distal movement. Beyond a minimum force threshold, detent 2440 is pushed outward by on body housing 122, and in body housing 122 then translates onto ramp 2442. The friction between on body housing 122 and ramp 24yy of the housing hold the on body housing 122 up against plunger 2405.

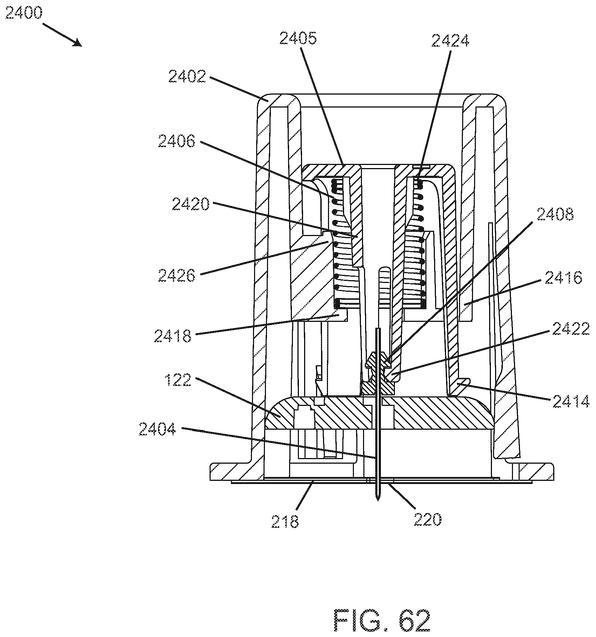

As illustrated in FIGURE 62, depression of plunger 2405 advances the inserter 2400 from an initial configuration to a deployed configuration. Contact of plunger 2405 and hub 2408 during depression of plunger 2405 imposes a downward force and consequential distal movement of sharp 2404.. As the sharp 2404 is urged distally, it carries the sensor insertion portion 30 into the subcutaneous portion of the subject's skin S. Contact of plunger 2405 and sensor housing 122 during depression of plunger 2405 imposes a downward force and consequential distal movement of sensor housing 122. Lip features 2414 of plunger 2405 maintain parallelism of sensor housing 122 to subject skin S during distal movement

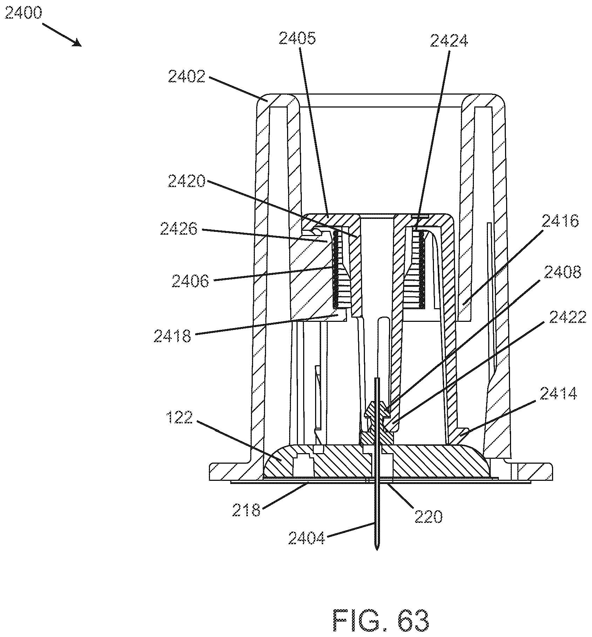

When plunger 2405 reaches a distal position, as shown in FIGURE 63, bottom surface 2424 engages wall 2426 and prevents further downward movement. The distal (lower) surface of on body housing 122 engages the upper surface of adhesive pad 218, thereby becoming adhered to the skin surface S of the subject.

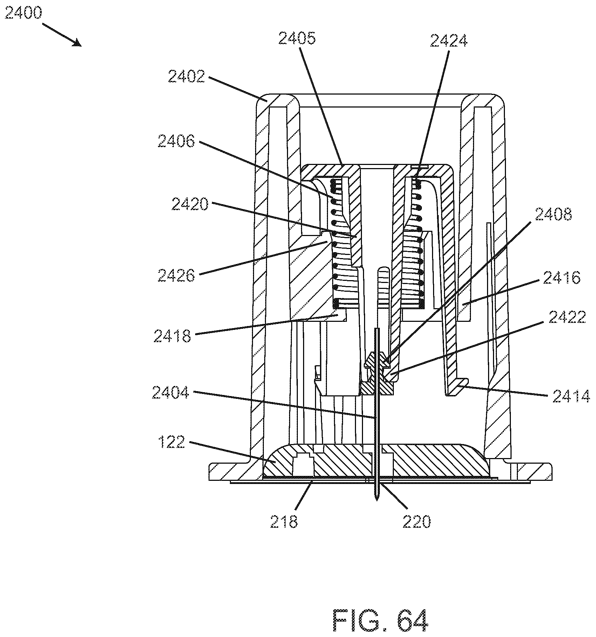

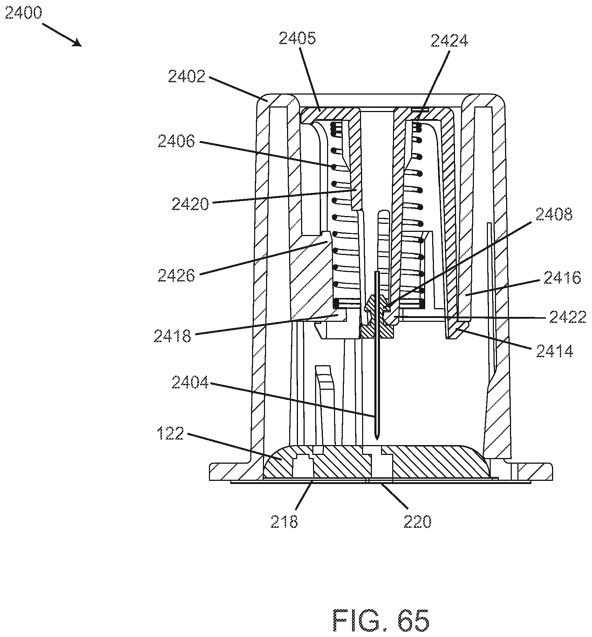

As the subject or some apparatus removes force from pusher 2405, spring 2406 urges plunger 2405 toward its proximal position (away from the skin surface) as shown in FIGURE 64, leaving on body housing 122 adhered to the skin surface S of the subject. Tabs 2427 provide additional downward force to the on body housing 122 to assᵢst holding it to adhesive patch 218 while the sharp 2404 is withdrawn through on body housing 122. Eventually, the upward force exerted by spring 2406 returns inserter 2400 to its initial configuration as illustrated in FIGURE 65.

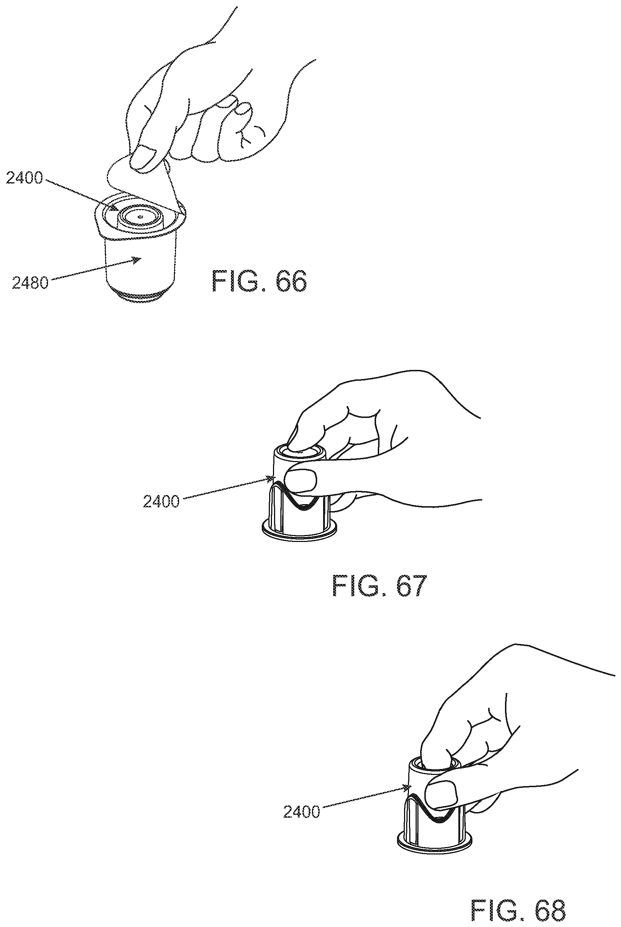

In some embodiments, inserter 2400 may be distributed in a sterilized package 2480 as depicted in FIGURE 66. To use inserter 2400 in this configuration, a user would first clean the insertion site on the skin with alcohol. The user would then remove inserter 2400 from sterilized package 2480 as shown in FIGURE 66. Next a user would place the inserter on the insertion site and push down on plunger 2405 until on body housing 122 is adhered to the subject's skin as shown in FIGURES 67-68. The user would then release the plunger 2405. Finally, the user would remove inserter 2400 from the insertion site and dispose of the inserter.

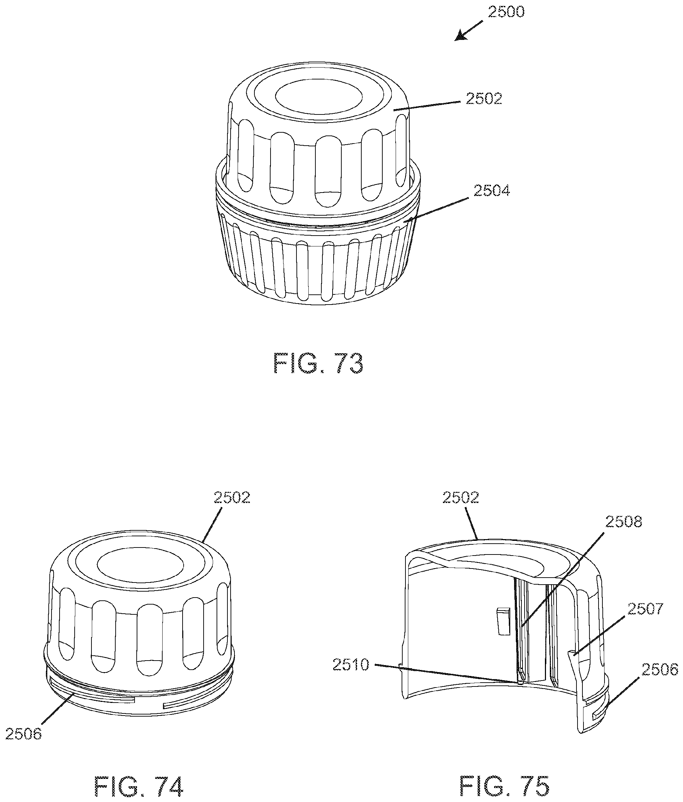

A further embodiment of an inserter is illustrated in FIGURES 73-87, and designated inserter 2500. In some embodiments, inserter 2500 has a maximum diameter of about 30mm to about 60 mm, *e.g.,* about 40 mm, about 43 mm, about 43.5 mm, about 50.25 mm, about 52 mm, etc. In some embodiments, inserter 2500 has a maximum height of about 40 mm to about 80 mm, e.g., about 44 mm, about 46 mm, about 50.25 mm, about 53 mm, about 67 mm, about 71 mm, etc. In some embodiments, inserter 2500 has a volume of about 35 cm" to about 110 cm³, e.g., about 40 cm³, about 41 cm³, about 50 cm³, about 60 cm³, about 61 cm³, about 62 cin³, about 69 cm³, about 70 cm³, about 79 cm³, about 90 cm³, about 106 cm³, etc. The height of the inserter is measured from the top of housing 2503 to the distal surface of the sheath 2512 that is intended to contact the skin of the subject. The volume of the inserter may be measured as the volume of the housing and the portion of the sheath 251 2 that may extend from the housing 2502.

Inserter 2500 generally includes, e.g., a handle 2502, sheath 2512, and a removable distal cap 2504 for maintaining a sterile environment for the medical device and sharp housed therein (FIGURE 73). As illustrated in FIGURES 74-75, handle 2502 is shown removed from distal cap 2504. Distal cap 2504 is detachably secured to handle 2502, *e.g.,* by use of threads 2506. It is understood that cap may be secured using snap-fit or press-fit configuration. Inserter 2500 may be utilized to advance a medical device into the skin of the subject. In some embodiments, handle 2502 is advanced relative to sheath 2512 in order to advance the medical device distally and into the skin of the patient.

Handle 2502 further includes needle carrier guides 2508 which allow the needle carrier 2514 to slidingly move relative to distal cap 2502 In an alternate embodiment, a detent prevents sheath 2512 from moving towards a "firing position" until a minimum force is applied. Location feature 2510 allows for the proper positioning of carriage 2516 when engaged.

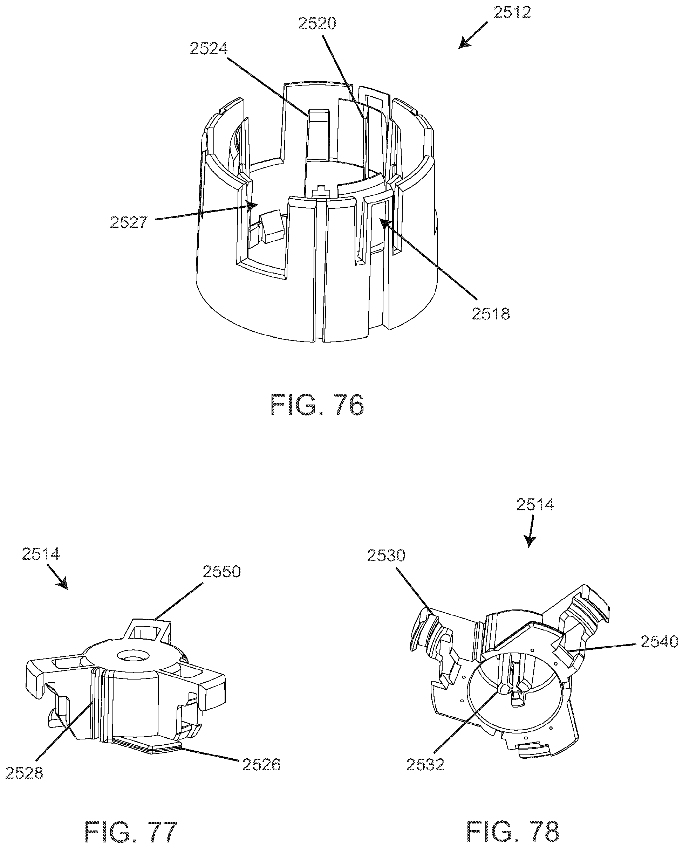

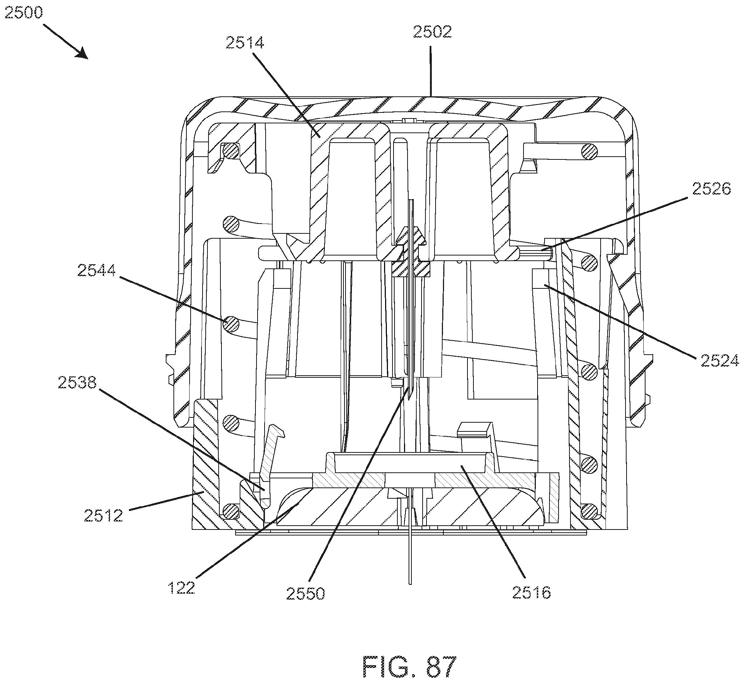

Further components of inserter 2500 are illustrated in FIGURES 76-80. Sheath 2512, as illustrated in FIGURES 76, may include longitudinal notches 2518 which snap into detents 2507. Retention members, such as ribs 2520, pinch spring arms 2522 located on carriage 2516 to prevent on body housing 122 from falling out of inserter 2500. Ribs 2520 do not extend to the bottom of sheath 2512, thus allowing carriage 2516 to release on body housing 122 when it has traveled to the bottom of sheath 2512 during insertion. Interfering structure, such as locking beam 2524, prevents inserter 2500 from being used again once needle carrier 2514 passes the locking beam (FIGURE 87). Specifically, locking feature 2526 of needle carrier 2514 engages with locking beam 2524 to prevent further use of inserter 2500.

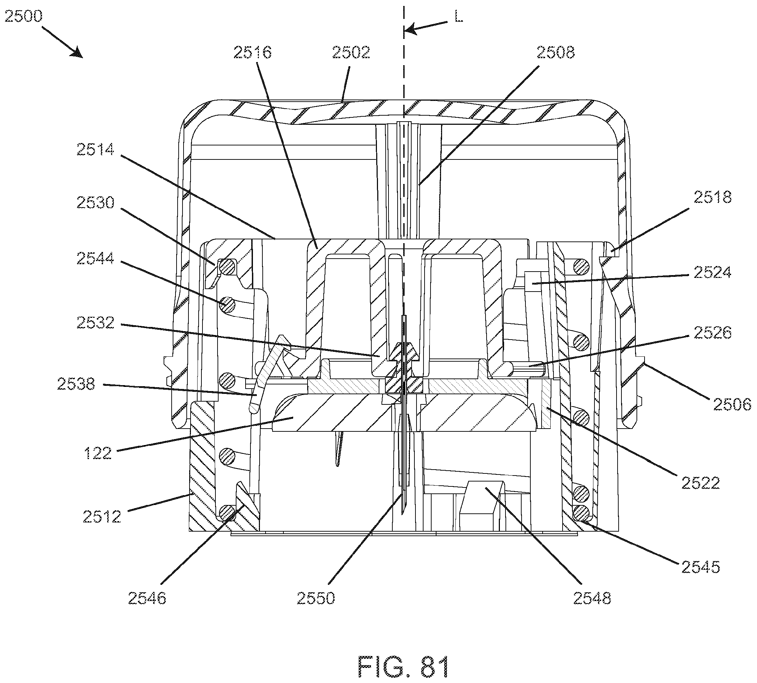

Needle carrier 2514 is illustrated in greater detail in FIGURES 77-78. In some embodiments, needle carrier 2514 includes guides, such as rail guides 2528, which interface with rail guides 2508, thereby allowing needle carrier 2514 to slidingly move relative to handle 2502. Notches 2527 are provided in sheath 2512 which has a larger dimension than the wings 2550 of needle carrier 2514, such that the needle carrier 2514 does not contact sheath 2512 during longitudinal movement of needle carrier 2514. Needle carrier 2514 also comprises detents/notches 2530 which interface with the upper edge of the spring when inserter 2500 is fully assembled (see FIGURES 81-87). In some embodiments, needle carrier 2514 comprises an attachment feature 2532 capable of accommodating a custom needle hub or attachment.

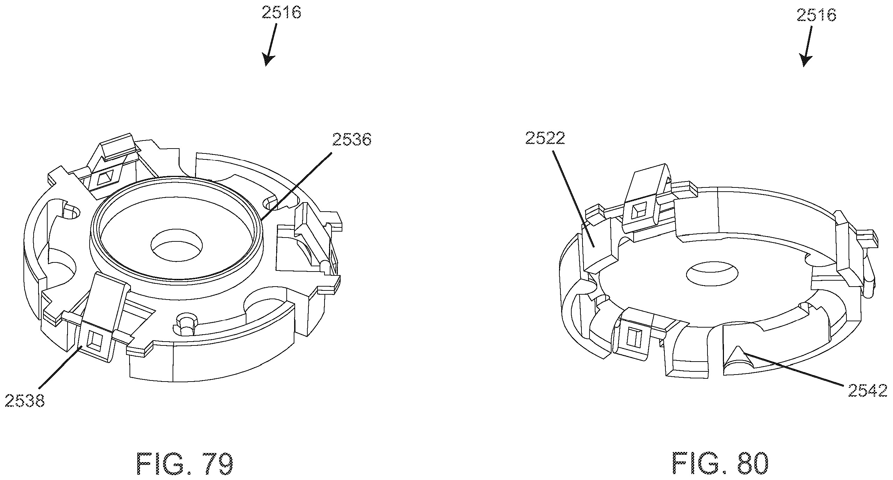

Carriage 2516 is illustrated in greater detail in FIGURES 79-80. As shown, carriage 2516 may comprise latches 2538 which connect it to needle carrier 2514 by locking with latches 2540. Spring hook 2542 allows for support for retaining on body housing 122 when the inserter has not been fired and allows for release of on body housing 122 when it has been attached to the skin of the user. (See, FIGURES 122, 125, 135-140.)

Inserter 2500 is illustrated in cross-section in FIGURE 81 in a state prior to use in which handle 2502 is disposed in a proximal position with respect to the sheath 2512. In such configuration, the sharp 2550 is disposed in a configuration spaced apart from the aperture 420 of the adhesive layer (not shown). The longitudinal axis L of the inserter 2500 is illustrated. The upper surface of spring 2544 is retained in inserter 2500 by detents/notches 2530 located on needle carrier 2514. The bottom surface of spring 2544 is retained by spring floor 2545 located on sheath 2512. Initially, spring 2544 is in an expanded or semi-expanded state while handle 2502 is disposed proximally from sheath 2512.

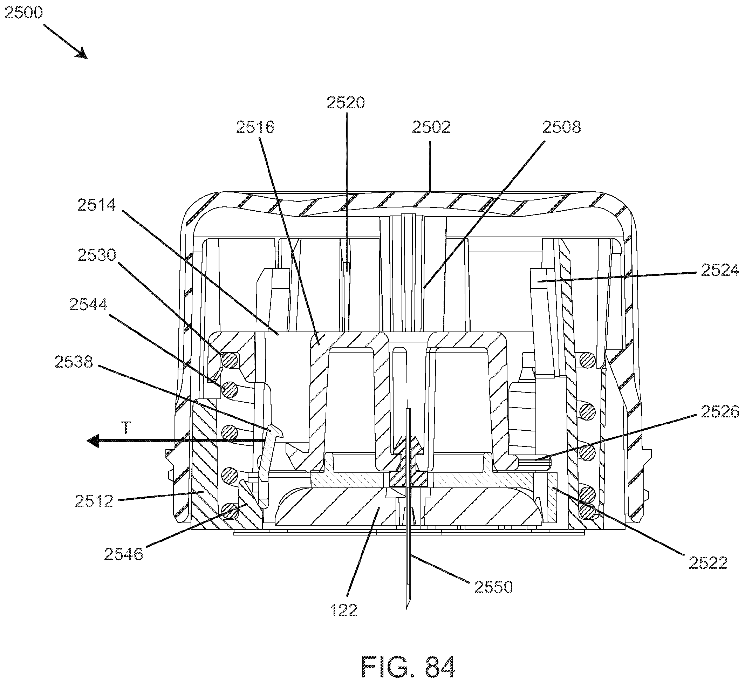

Extending distally from the upper surface of handle 2502 is inner wall 2508. In some embodiments, the distal end portions of wall 2508 provide a downward force on carriage 2516 upon depression of handle 2502 by a user. Alternatively, instead of handle 2502 having a distally extending inner wall 2508, carriage 2516 can include one or more upwardly extending walls or projections (not shown). The one or more upwardly extending inner walls or projections of the carriage 2516 can have a length sufficient to either contact the inside of the upper surface of handle 2502 or, alternatively, contact corresponding downwardly extending inner walls of handle 2502. In this manner, depression of handle 2502 by a user provides a downward force on the one or more upwardly extending walls or projections of carriage 2516 to advance carriage 25 16 (and on body housing 122) distally to an installation and insertion position. (FIGURE 84) In such embodiment, the downwardly extending inner wall of the handle has a distal end that is disposed proximally of the proximal most end of sheath 2512.

Sharp 2550 extends longitudinally from needle carrier 2514 within inserter 2500. In some embodiments, sharp 2550 is supported at an oblique angle, e.g., between about 0° and 90° with respect to the skin surface.

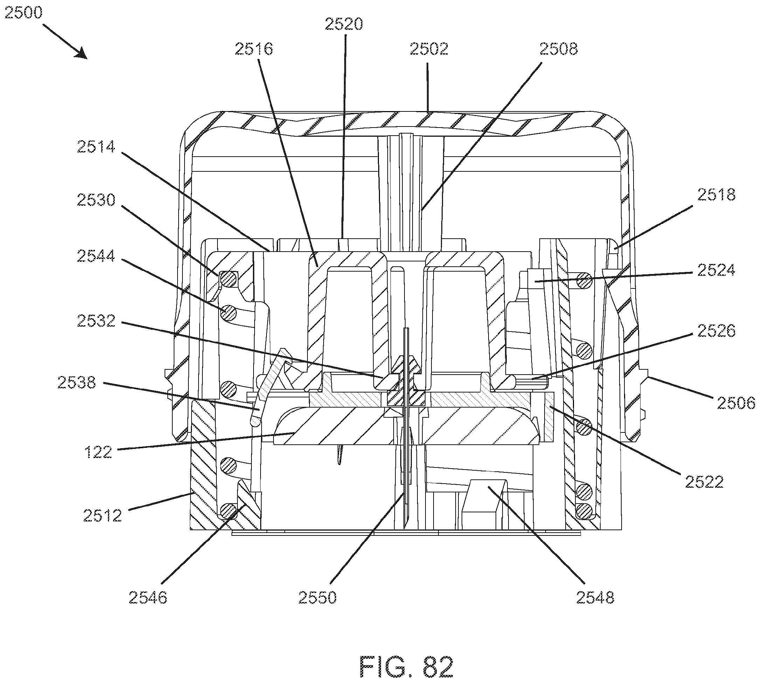

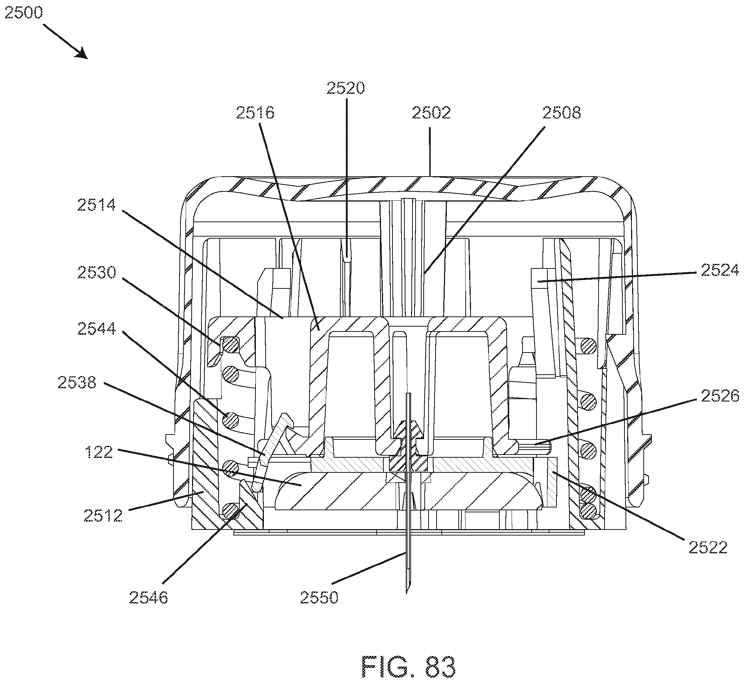

FIGURE 82 illustrates inserter 2500 in cross-section after a user applies an initial downward force to button 2502. Further depression of handle 2502 with respect to sheath 2512, against the bias of spring 2544, causes distal longitudinal movement of the carriage 2516 and needle carrier 2514, from a proximal position towards a distal position as shown in FIGURE 83. During such downward proximal movement, spring 2544 is compressed between detents/notches 2530 and retention tab 2546. As sharp 2550 is further urged distally, it carries the sensor insertion portion 30 of sensor 14 (FIGURE 17) into the subject's skin S.

As carriage 2516 reaches a distal position (near the subject's skin) as shown in FIGURE 83, the distal surface of the on body housing 122 engages the upper surface of adhesive pad (not shown), thereby becoming adhered to the skin surface S of the subject. Latch 2538 engages the upper surface of retention tab 2546 as shown in FIGURES 84. As a result, the top portion of latch 2538 is pivoted outward in direction T. Such pivoting of latch 2538 causes needle carrier to become disengaged from carriage 2516.

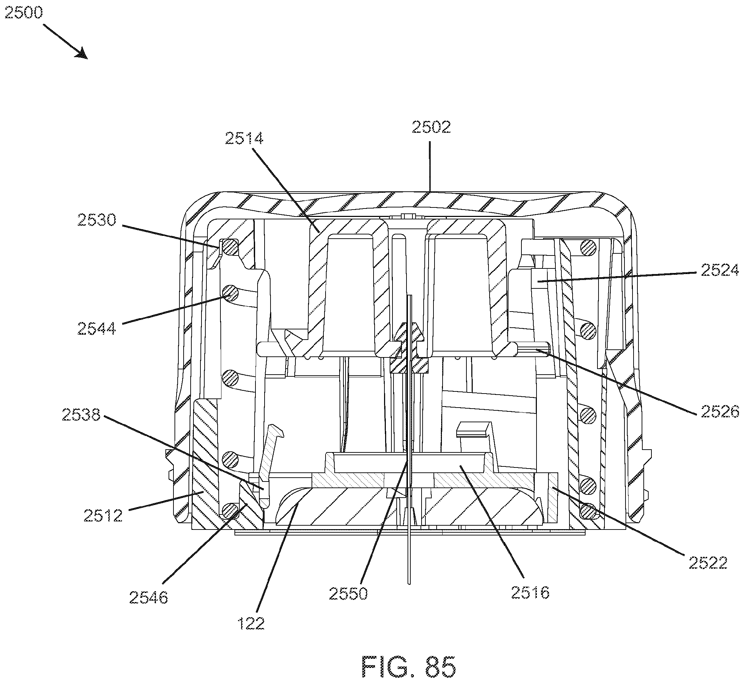

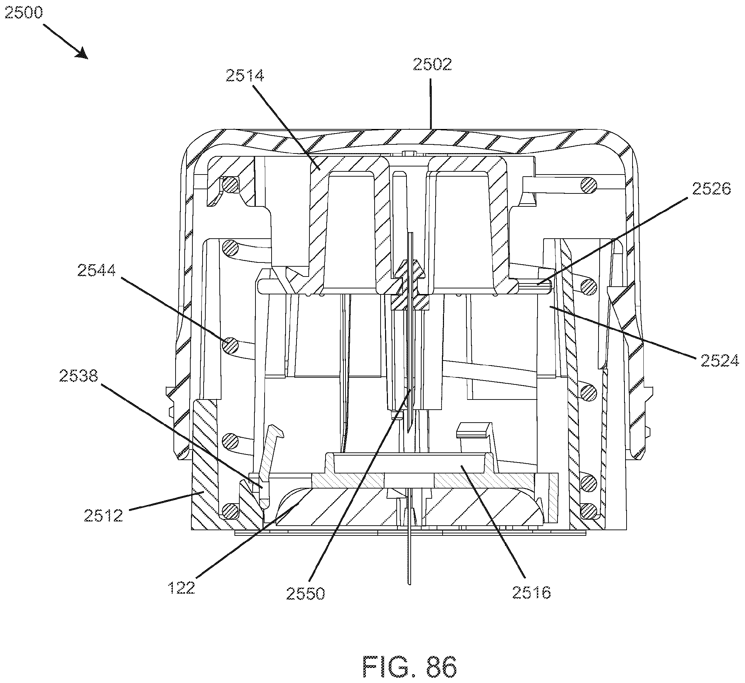

As illustrated in FIGURE 85, disengagement of the needle carrier 2514 from the carriage 2516 permits spring 2544 to expand, thereby advancing the needle carrier 2514 to a proximal position and withdrawing the sharp 2550 from the skin S of the subject while leaving the on body housing 122 attached to the skin. As the sharp is withdrawn (FIGURE 86), locking feature 2526 advances past locking beam 2524 because of the upward force exerted on needle carrier 2514 by spring 2544.

Referring now to FIGURE 87, once the sharp 2550 has been withdrawn from the subject, button 2500 cannot be pressed again because any downward movement will be blocked by the interaction of locking beam 2524 and locking feature 2526.

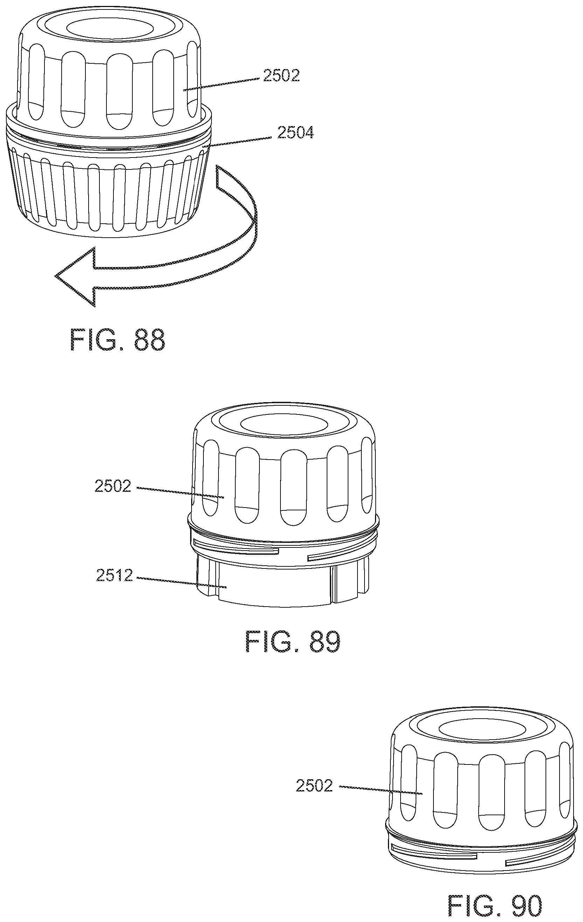

In some embodiments, inserter 2500 may come in a sterilized package which is capable of a one-time use as shown in FIGURES 88-90. To use inserter 2500 in this manner, a user would first sterilize the insertion site on the skin with alcohol. The subject would then twist off cap 2504 as shown in FIGURE 88. Next a subject would place the inserter on the sterilized insertion site and push down on inserter 2500 until on body housing 122 is adhered to the subject's skin as shown in FIGURES 89-90. Finally, the subject would remove inserter 2500 from the insertion site and dispose of the inserter. In this manner, the inserter 2500 itself serves as its own sterilized packaging. This procedure applies also to the other inserters described herein.

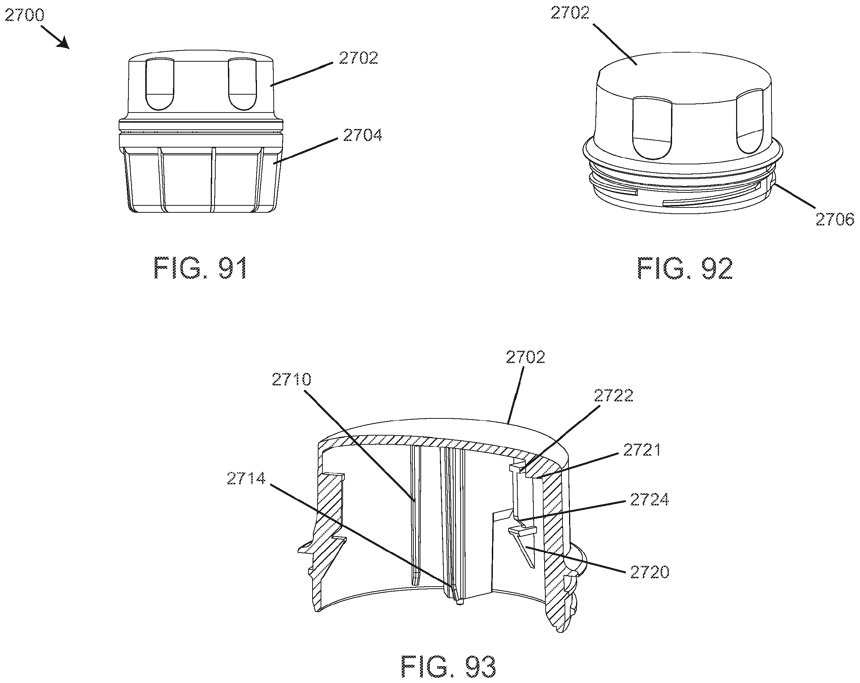

A further embodiment of an inserter is illustrated in FIGURES 91-108, and designated inserter 2700. In some embodiments, inserter 2700 has a maximum diameter of about 30mm to about 60 mm, e.g., about 40 mm, about 43 mm, about 43.5 mm, about 46 mm, about 50 mm, etc. In some embodiments, inserter 2700 has a maximum height of about 40 mm to about 80 mm, e.g., about 44 mm, about 46 mm, about 49.5 mm, about 55 mm, about 67 mm, about 71 mm, etc. In some embodiments, inserter 2700 has a volume of about 35 cm³ to about 110 cm³, e.g., about 40 cm³, about 41 cm³, about 50 cm³, about 60 cm³, about 61 cm³, about 62 cm³, about 69 cm³, about 70 cm³, about 79 cm³, about 90 cm³, about 106 cm³, etc. The maximum height refers to the height defined from the top of the housing 2702 to the portion of the sheath 2708 that contacts the subject's skin. The volume is measured as the volume of the housing 2702 and the portion of the sheath 2708 extending from the housing.

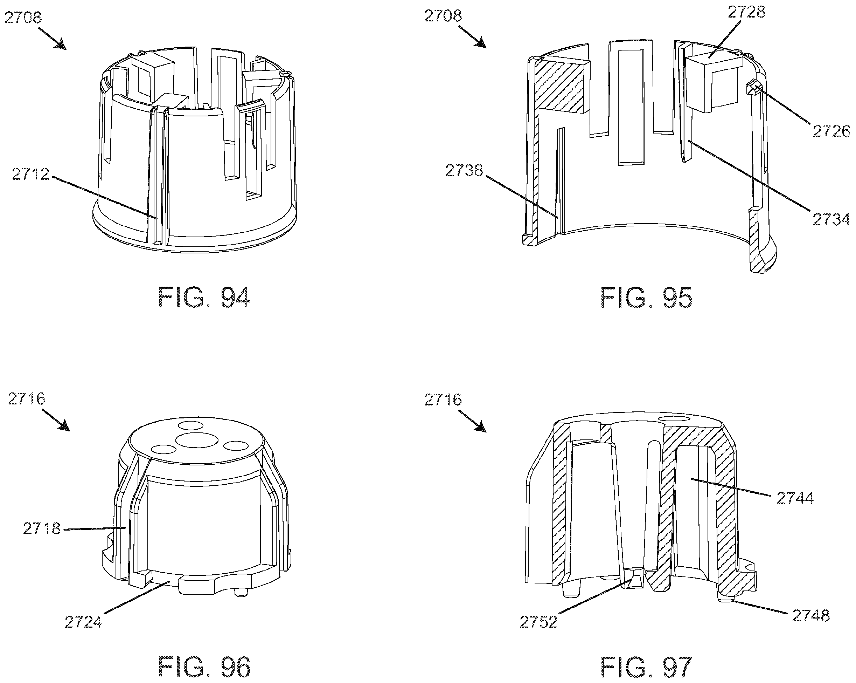

Inserter 2700 generally includes, e.g., a housing 2702 (FIGURES 92-93), sheath 2708 (FIGURES 94-95), and a removable distal cap 2704 for maintaining a sterile environment for the medical device and sharp housed therein (FIGURE 91). As illustrated in FIGURES 92-93, housing 2702 is shown removed from distal cap 2704. Distal cap 2704 is detachably secured to housing 2702, e.g., by use of threads 2706. It is understood that cap may be secured using snap-fit or press-fit configuration. Inserter 2700 may be utilized to advance a medical device into the skin of the subject.. Sheath 2708 generally defines a cavity or open space,within which sharp carrier 2716 and medical device carrier 2730 are moveable. In some embodiments, housing 2702 is advanced relative to sheath 2708 in order to advance the medical device distally and into the skin of the patient.

Housing 2702 includes sheath guide rail 2710 which interfaces with rail guides 2712 located on sheath 2708 (FIGURE 94), thereby allowing housing 2702 to slidingly move longitudinally relative to sheath 2708. Housing 2702 may further includes sharp carrier guide rail 2714 which interfaces with rail guides 2718 located on sharp carrier 2716 (FIGURE 97). Sheath 2708, sharp carrier 2716, and housing 2702 may alternatively move relative to one another without the use of guide rails.

Ledge 2720 and/or ledge 2722 are provided on an interior portion of housing 2702. Ledge 2720 engages sheath 2708 to hold sheath 2708 in a pre-use position prior to insertion of the medical device. Ledge 2722 engages sheath 2708 to secure sheath 2708 in a post-use position after insertion of the medical device. Housing 2702 further includes detent 2724 which prevents housing 2702 from moving relative to sheath 2708 until a minimum force has been applied, e.g., distally by user to housing 2702. The sheath 2708 is secured to the housing 2702 via snap 2726. Snap 2726 snaps into the housing detent 2724. (In some embodiments, it is pinched between ledge 2720 and detent 2724, thus controlling its longitudinal position relative to the housing 2702). The needle carrier 2716 is located and secured to the medical device carrier 2730 (located via interaction of locating features 2748 and 2750 and secured via interaction of carrier arms 2732 and angled top surface of 2716). The ledge 2720 is a controlled surface onto which the top of sheath surface 2728 will engage at the end of the insertion stroke to prevent further relative movement in some embodiments.

Further components of inserter 2700 are illustrated in FIGURES 94-99. Sheath 2708 is a generally cylindrical component. As illustrated in FIGURES 94-95, sheath 2708 may include attachment snaps 2726 which are biased into detent 2724 of housing 2702 to create a minimum force that must be overcome in order to advance sharp 224 into the subject's skin and install the on body housing 122. The interaction of the snap 2726 with the detent 2724 and ledge 2720 holds the assembly in a slop/rattle free position. In some embodiments, the force to be overcome can be about 0.23 kgf to about 2.27 kgf (about 0.5 lbf to about 5 lbf), e.g., about 0.45 kgf (about 1 lbf), about 0.91 kgf (about 2 lbf), about 1.36 kgf (about 3 lbf), about 1.81 kgf (4 lbf), etc. Support wall 2728 prevents carrier arms 2732 on carrier 2730 from bending outwardly, clear of sharp carrier 2716. Ribs 2734 pinch carrier arms 2732 on carrier 2730, thus preventing on body housing 122 from falling out of inserter 2700 when sheath 2708 is in the extended position. Ribs 2734 are not present at the bottom of sheath 2708, thus allowing room for spring arms 2736 on carrier 2730 to release on body housing 122 when carrier 2730 has traveled to the bottom of sheath 2708. Slot 2738, located on sheath 2708, interfaces with locating feature 2740 on carrier 2730, thus orienting carrier 2730 to sheath 2708 during assembly. Once force is overcome to allow carrier 2730 to move distally towards the subject's skin, no further force is required to retract the sharp 324 from the subject's skin.

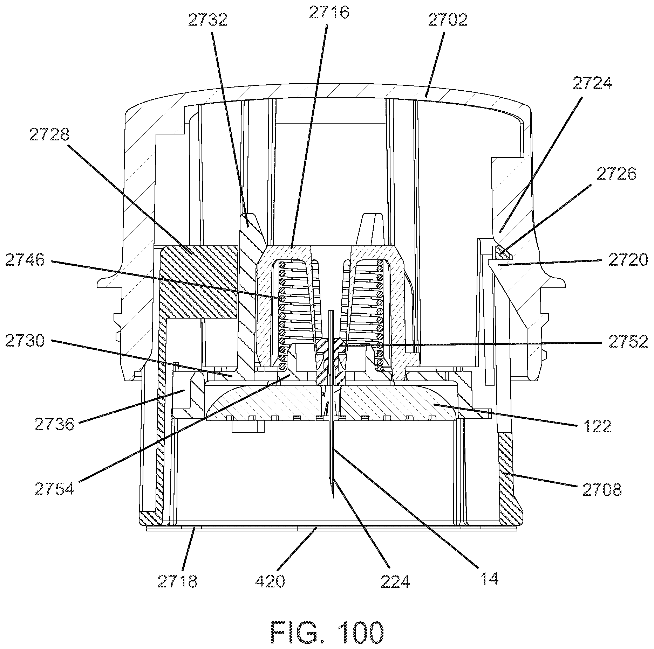

Referring next to FIGURES 96-97, depicted is sharp carrier 2716 in a perspective and cross-sectional view, respectively. Sharp carrier 2716 contains notches 2724 which allow clearance for the passage of carrier arms 2732 located on medical device carrier 2730. Guidance walls 2744 securely hold spring 2746 in place (FIGURE 100). Locating features 2748, e.g., bosses or tabs, align with locating features 2750, e.g., recesses or apertures, on carrier 2730. Snap features 2752 secure sharp 224 securely within inserter 2700. It is contemplated that sharp 224 may be secured to sharp carrier 2716 by other techniques, e.g., friction fit, adhesive, welding, etc.

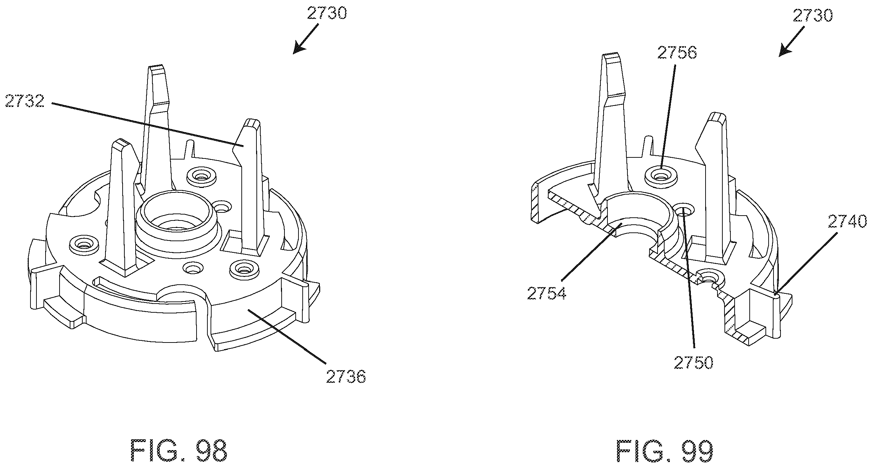

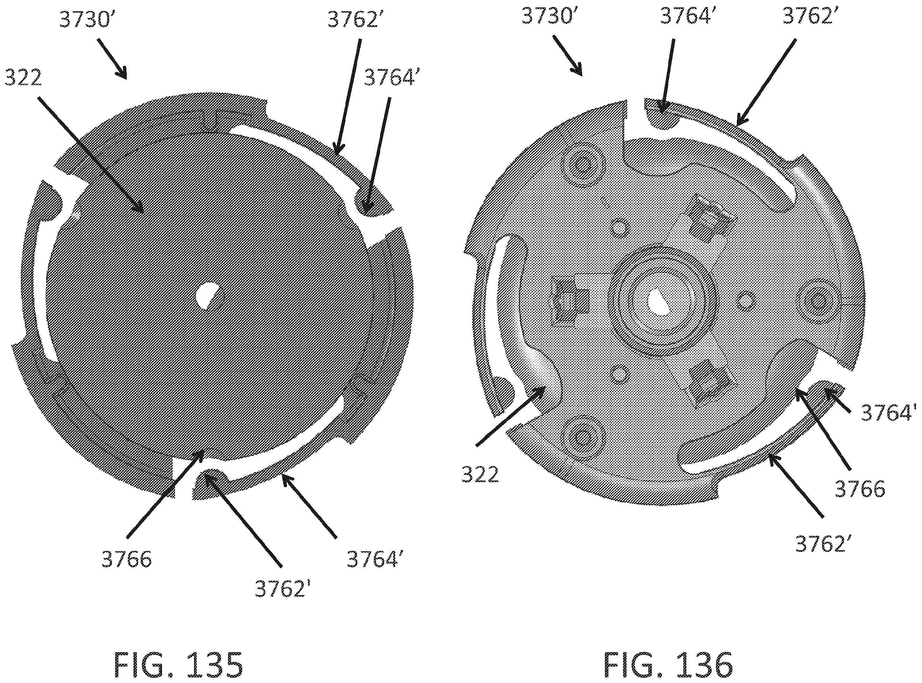

Medical device carrier 2730 is depicted in more detail in FIGURES 98-99. As shown, carrier 2730 contains spring locating ring 2754 which receives one end of spring 2746. In some embodiments, spring 2746 surrounds spring locating ring 2754. In some embodiments, the inner area remains clear to leave room for the deflection of sharp carrier feature snaps 2752 that move outwardly when the sharp is inserted. Carrier 2730 further comprises locating features 2756 which interface with locating features on housing 2702. (See FIGURES 135-136).

Inserter 2700 is illustrated in cross-section in FIGURE 100 in a state prior to use in which housing 2702 is disposed in a proximal position with respect to the sheath 2708. In such orientation, sharp 224 is disposed in a configuration spaced apart from the aperture 420 of the adhesive layer 118. The upper surface of spring 2746 is retained in inserter 2700 by sharp carrier 2716. The bottom surface of spring 2746 is retained by spring location ring 2754. Initially, spring 2746 is in a compressed or semi-compressed state while housing 2702 is disposed proximally from sheath 2708.

Sharp 224 extends longitudinally from sharp carrier 2716 within inserter 2700. In some embodiments, sharp 224 is supported at an oblique angle, e.g., between and including about 0° and 90° with respect to the skin surface.

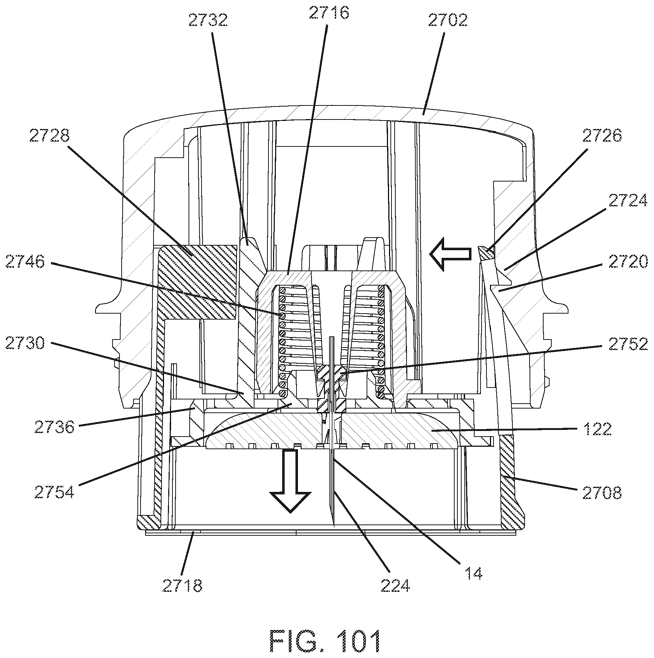

FIGURE 101 illustrates inserter 2700 in cross-section after a user applies an initial downward force to housing 2702. In some embodiments, a predetermined minimum force must be used so that attachment snaps 2726 advance past detent 2724.

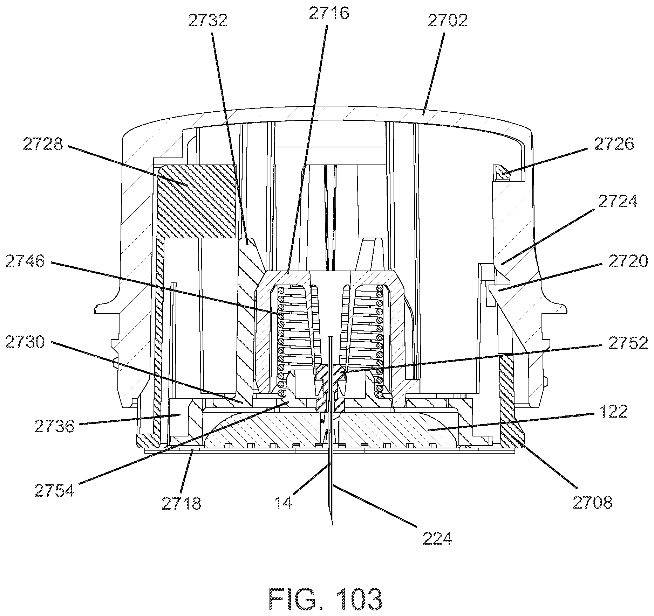

After detent 2724 has been overcome, e.g., snap 2726 is radially displaced, further depression of housing 2702 with respect to sheath 2708 causes distal longitudinal movement of the carrier 2730 and sharp carrier 2716, from a proximal position towards a distal position as shown in FIGURE 103. As sharp 224 is further urged distally, it carries the sensor insertion portion 30 of sensor 14 (FIGURE 17) into the subcutaneous portion of the subject's skin S.

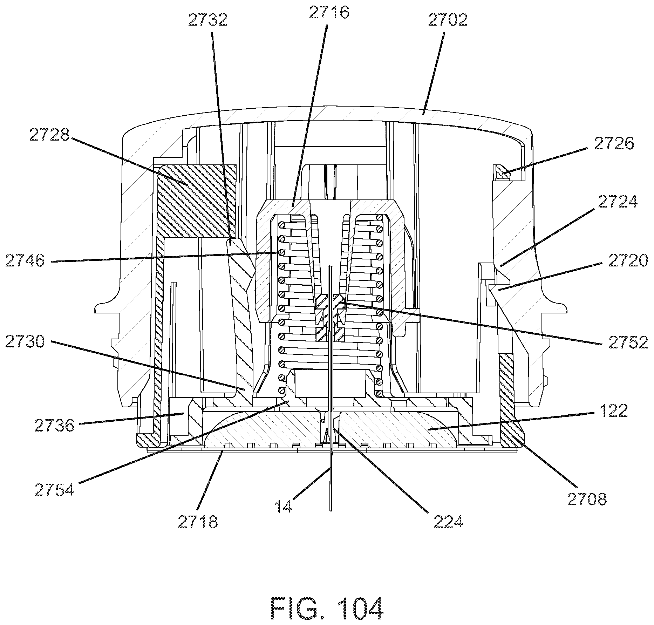

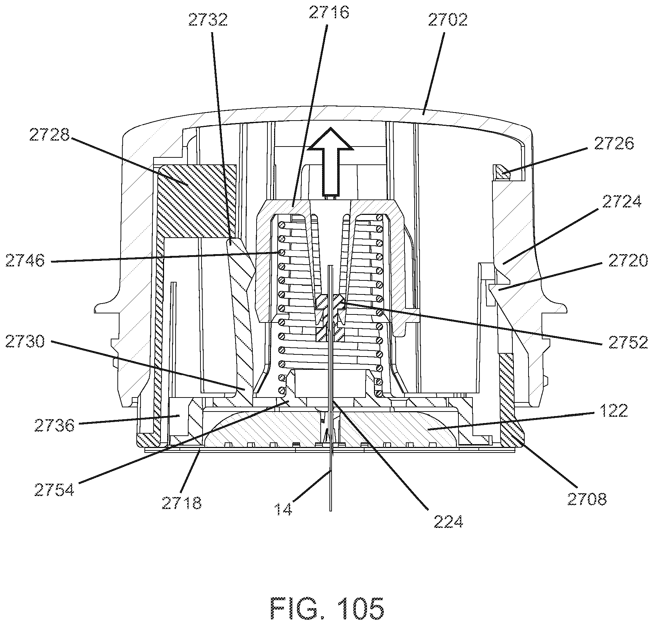

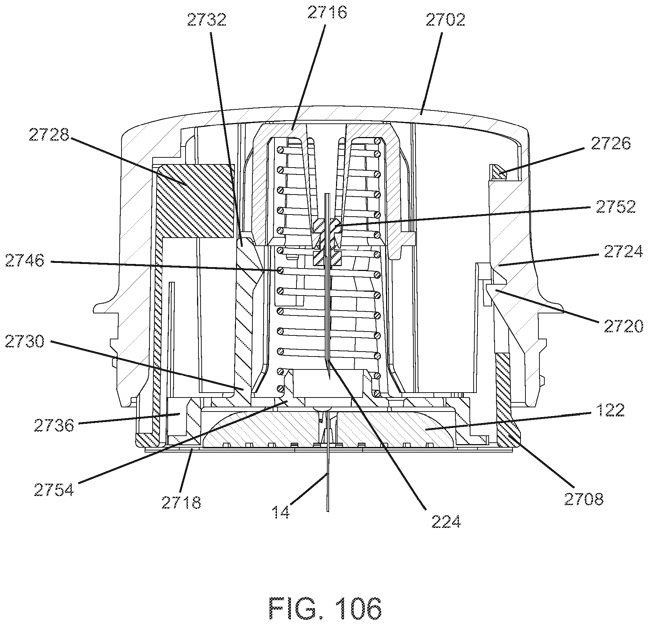

As carrier 2716 reaches a distal position (FIGURE 103), the distal surface of the on body housing 122 engages the upper surface of adhesive pad 2718, thereby becoming adhered to the skin surface S of the subject. Concurrently, carrier arms 2732 are advanced distally and clear the support wall 2728. This allows carrier arms 2732 to deflect radially outwardly. (See, FIGURE 104). When carrier arms 2732 deflect radially outwardly, shoulder portions of carrier arms 2732 are no longer in an interference relationship with the sharp carrier 2716. Thus spring 2746 is permitted to expand as shown in FIGURE 105, thereby advancing the sharp carrier 2716 to a proximal position and withdrawing the sharp 224 from the skin S of the subject while leaving the on body housing 122 attached to the skin. Handle 2702 is maintained in the distal position. Sheath stiap 2726 of the sheath 2708 have now moved up to lock over feature 2722 of the housing 2702. Now the housing 2702 and the sheath 2708 can no longer move longitudinally with respect to each other, and provides an indication to a user that the inserter has been used. In FIGURE 106, the medical device carrier 2730 acts as a needle guard to prevent a user for touching the needle.

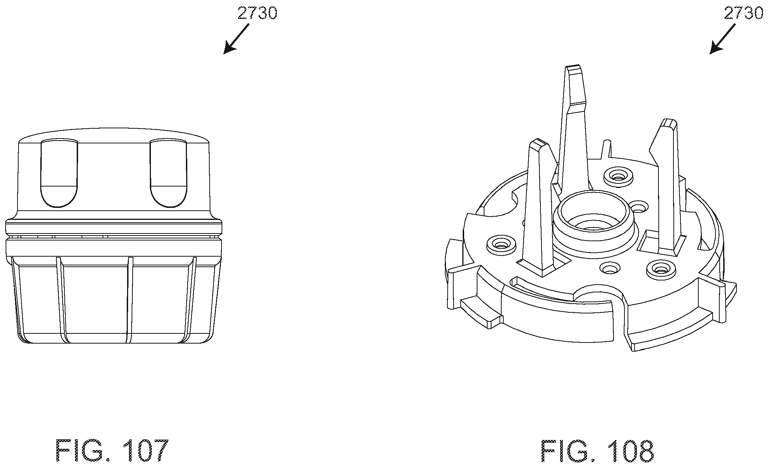

In some embodiments, the changing interaction of sheath snap 2726 with the housing detent/ledges 2720, 2724, and 2722 determine whether the sheath 2708 is locked. When snap 2726 is in the pre-fire position, ledge 2720 prevents sheath 2708 from being pulled out of the housing 2702. In this position, detent 2724 may also impede the movement of pushing the sheath 2708 into the housing 2702. When the detent is overcome by a at least at minimum force, the sheath 2704 moves longitudinally with respect to the housing 2702 until the snap 2726 snaps over housing ledge 2722. At this point, ledge 2722 prevents the sheath 2708 from being pulled out of the housing again, but from a new position (this position may be referred to as the used/post-fire position). Sharp carrier snap 2752 function is to hold onto the sharp 224. in some embodiments, the sharp carrier 2716 is held in the post-fire position relative to the housing 2702 by, e.g., an interference between the rails of the housing 2714 and the guide rails of the sharp carrier 2718 (this interference is only present once the sharp carrier is fully retracted) and/or by medical device carrier projections 2732 interfering with the bottom/floor of the sharp carrier (See, e.g., FIG. 106). In another embodiment of inserter 2700, adhesive pad 118 may be attached directly to on body housing 122. This necessitates a different shape of inserter 2700 as depicted in FIGURE 107. Additionally, carrier 2630 is slightly wider to accommodate adhesive pad 118 attached to on body housing 122 (FIGURE 108).

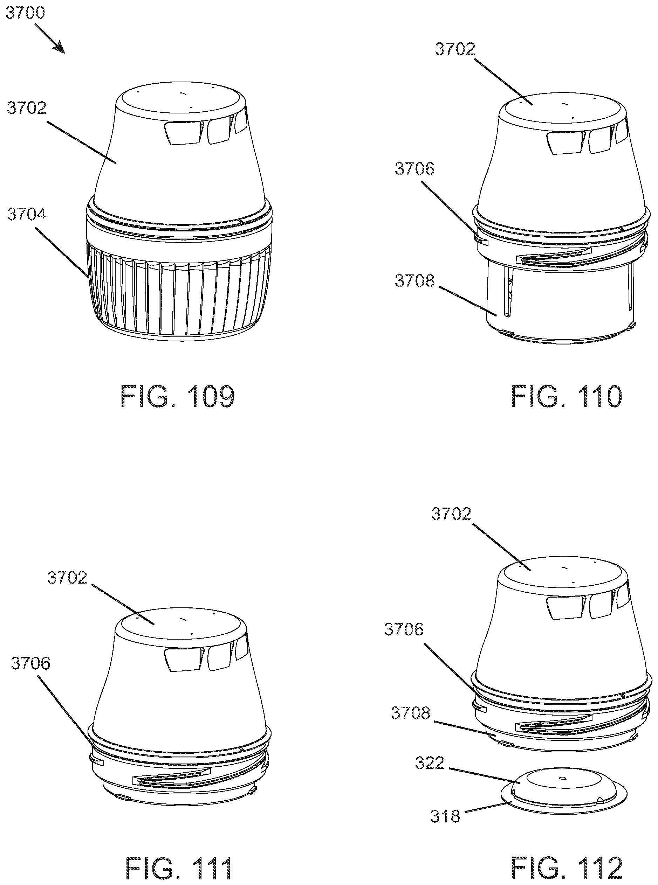

A further embodiment of an inserter is illustrated in FIGURES 109-134. In some embodiments, inserter 3700 has a maximum diameter of about 30mm to about 60 mm, *e.g.,* about 40 mm, about 43 mm, about 43.5 mm, about 46 mm, about 50 mm, etc. In some embodiments, inserter 3700 has a maximum height of about 40 mm to about 80 mm, *e.g.,* about 44 mm, about 46 mm, about 49.5 mm, about 55 mm, about 67 mm, about 71 mm, etc. In some embodiments, inserter 3700 has a volume of about 35 cm³ to about 110 cm³, e.g., about 40 cm³, about 41 cm³, about 50 cm³, about 60 cm³, about 61 cm³, about 62 cm³, about 69 cm³, about 70 cm³, about 79 cm³, about 90 cm³, about 106 cm³, etc. The maximum height refers to the height defined from the top of the housing 3702 to the portion of the sheath 3708 that contacts the subject's skin. The volume is measured as the volume of the housing 3702 and the portion of the sheath 3708 extending from the housing.

Figures 109-112 depict the various stages of insertion from an initial stage in which the cap is attached (FIGURE 109), to removal of the cap (FIGURE 110), deployment of the sharp and on body housing unit (FIGURE 111) and removal of the inserter from the subject's skin (FIGURE 112).

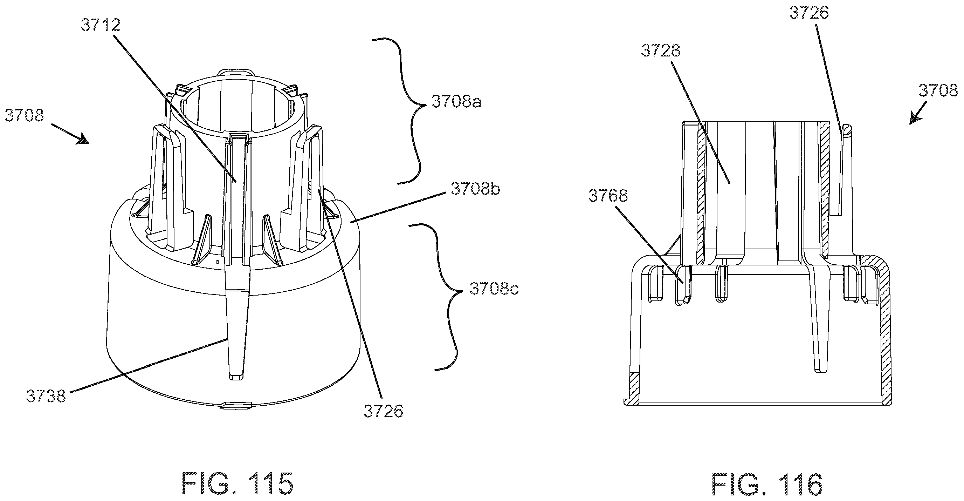

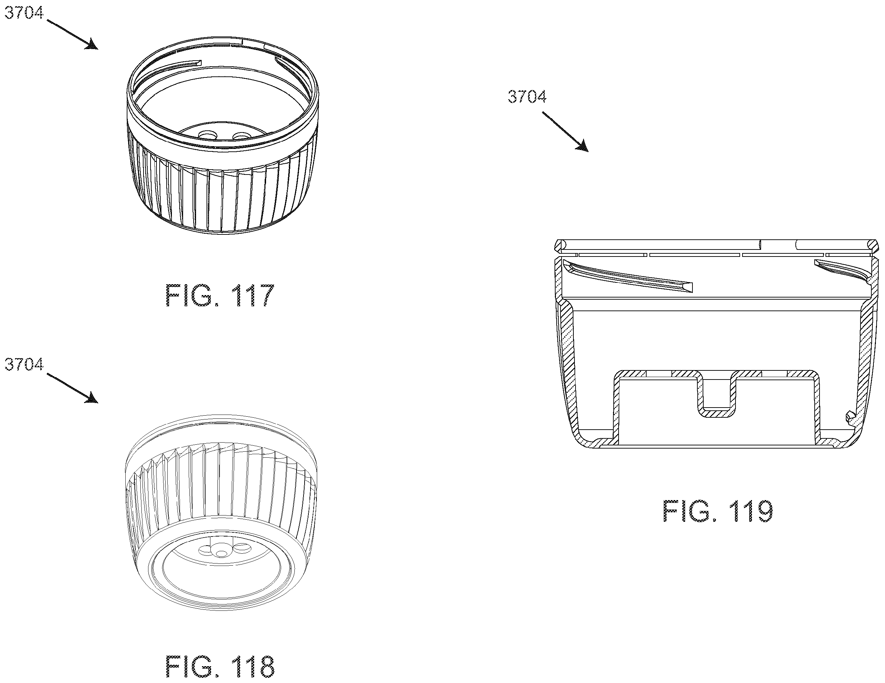

Inserter 3700 generally includes, *e.g.,* a housing 3702 (FIGURES 109, 113-114), sheath 3708 (FIGURES 115-116), and a removable distal cap 3704 (FIGURES 117-119) for maintaining a sterile environment for the medical device and sharp housed therein. As illustrated in FIGURES 109 and 117-119, housing 3702 is shown removed from distal cap 3704. Distal cap 3704 is detachably secured to housing 3702, *e.g.,* by use of threads 3706. It is understood that in some embodiments, the cap may be secured using snap-fit or press-fit configuration.

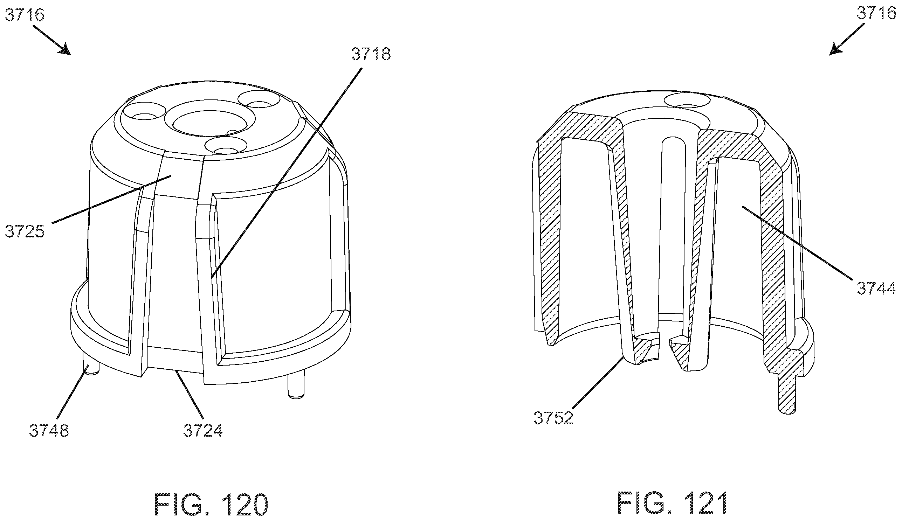

Inserter 3700 may be utilized to advance a medical device into the skin of the subject. Sheath 3708 generally encloses or defines a cavity, within which sharp carrier 3716 (FIGURES 120-121) and medical device carrier 3730 (FIGURE 122) are moveable. In some embodiments, housing 3702 is advanced relative to sheath 3708 in order to advance the medical device distally and into the skin of the patient.

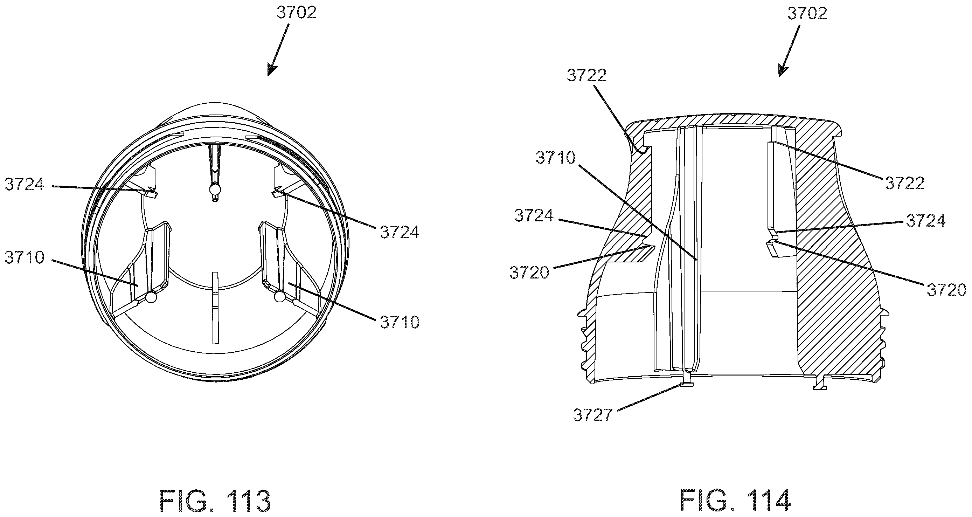

Housing 3702 includes sheath guide rail 3710 which interfaces with rail guides 3712 located on sheath 3708, thereby allowing housing 3702 to slidingly move relative to sheath 3708. Sheath 3708, sharp carrier 3716, and housing 3702 may alternatively move relative to one another without the use of guide rails. The housing can include a distally extending sidewall having a non-linear or arcuate shape. In the embodiment illustrated in FIGURES 109-134 the housing is configured with an undulating sidewall which transitions from a concave portion (upper portion of housing 3702) to a convex portion (lower portion of housing 3702). (FIGURE 114). This contour enhances the users tactile recognition and provides a more ergonomic gripping surface which reduces accidental slippage by the user's hand. Further, the housing is configured with a cavity sized to receive the sheath 3708, as described in further detail below.

Housing ledge 3720 and/or ledge 3722 are provided on an interior portion of housing 3702. Ledge 3720 engages sheath 3708 to hold sheath 3708 in a pre-use position prior to insertion of the medical device. Ledge 3722 engages sheath 3708 to secure sheath 3708 in a post-use position after insertion of the medical device. Housing 3702 further includes detent 3724 which prevents housing 3702 from moving relative to sheath 3708 until a minimum force has been applied, e.g., distally by user to housing 3702. The sheath 3708 is secured to the housing 3702 via retention features 3726, which can be configured, e.g., as a snap. Retention feature 3726 snaps into the housing detent 3724 (In some embodiments, it is pinched between ledge 3720 and detent 3724, thus controlling its height relative to the housing 3702). In some embodiments, the surfaces of the housing ledges 3720, 3722, 3724 and retention features 3726 are configured to engage in a single point of contact or a plurality of discrete points of contact, e.g., line. Such discrete points of contact are advantageous over conformal surface-to-surface contact in that a more thorough sterilization process can be performed. A variety of sterilization mediums can be employed, e.g., Ethylene Oxide (EtO), wherein the gaseous medium is delivered over the various inserter components. Accordingly, the discrete points of contact allow for a greater surface area of each inserter component to be exposed to the gaseous medium, thereby providing for a more thorough and rapid sterilization process. The housing includes distally extending protrusions 3727 which are received in apertures 3756 of the medical device carrier 3730 to couple the housing and medical device carrier, by such techniques as, e.g., heat staking, ultrasonic bonding, adhesive bonding, snap fit, etc. Coupling the housing and the medical device is performed, in some embodiments, by e.g., heat staking, ultrasonic bonding, adhesive bonding, snap fit, etc. Consequently, there is no relative movement between the housing 3702 and the medical device carrier 3730.

Sheath 3708 is a generally formed as a unitary tubular member having proximal 3708a and distal 3708c cylindrical portions. In some embodiments, the portions 3708a and 3708c have an elliptical, square, hexagonal, or other cross-section. As illustrated in FIGURES 1 15-116, the distal cylindrical portion 3708c (i.e., the lower portion) can be formed with a greater diameter than the proximal portion 3708a, with the proximal and distal portions integrally connected via a shelf 3708b. Accordingly, the sheath 3708 can be formed as a single -piece and generally cylindrical member with the proximal portion having sufficient rigidity to prevent displacement of the carrier arms 3732, as described in further detail below. Sheath 3708 can include retention members 3726, e.g., detent snaps, which are biased into detent 3724 of housing 3702 to create a minimum force that must be overcome in order to advance sharp 324 into the subject's skin and install the on body housing 322. The retention members 3726 can extend proximally from the shelf 3708b of the sheath and be formed as a separate member such that the retention members are spaced or offset from the cylindrical wall of the sheath. The actuation force of the inserter is determined by the stiffness of retention members 3726 (which is a function of length, thickness, and cross section) as well as the steepness of the angle of detent 3724 of the housing. In some embodiments, the force to be overcome can be about 0.23 kgf to about 2.27 kgf to about 2.27 kgf (about 0.5 lbf to about 5 lbf), e.g., about 0.45 kgf (about 1 lbf), about 0.91 kgf (about 2 lbf), about 1.36 kgf (about 3 lbf), about 1.81 kgf (about 4 lbf, etc.

As described above, the proximal portion 3708a of the sheath is sized such that an interior support wall surface 3728 prevents carrier arms 3732 on medical device carrier 3730 from displacement or bending outwardly, clear of sharp carrier 3716. Maintaining the carrier arms 3732 in a fixed or constrained position within the sheath allows a user to accurately know the relative positioning of the needle within the inserter. Conversely, the distal portion 3708c of the sheath is sized such that the diameter of the interior wall surface is greater than the carrier arms 3732, thus allowing room for spring arms 3732 on carrier 3730 to expand or displace radially outward thereby releasing the sharp carrier 3716 to retract to the proximal position. Guide rails 3712 are included on the exterior surface of the proximal portion of the sheath 3708a. The guide rails 3712 remain engaged with the housing guide rail 3710 of the housing throughout the insertion operation, i.e., from advancement of the housing from the proximal position to the distal position. Thus even prior to insertion, rotational position of the housing and sheath is controlled and "rocking" is minimized. In general, rocking is minimized by increasing the length of engagement with respect to the diameter of engagement. In the embodiment disclosed herein, the length of engagement between the sheath and housing, i.e. along the longitudinal axis, is relatively large while the diameter at which the engagement occurs is relatively small, i.e. at proximal portion of sheath 3708a. Additionally, sheath 3708 includes a slot 3738 extending distally from the shelf 3708b and configured to receive the guide rail 3710 of the housing upon delivery of the medical device and insertion of the sharp into the subject.

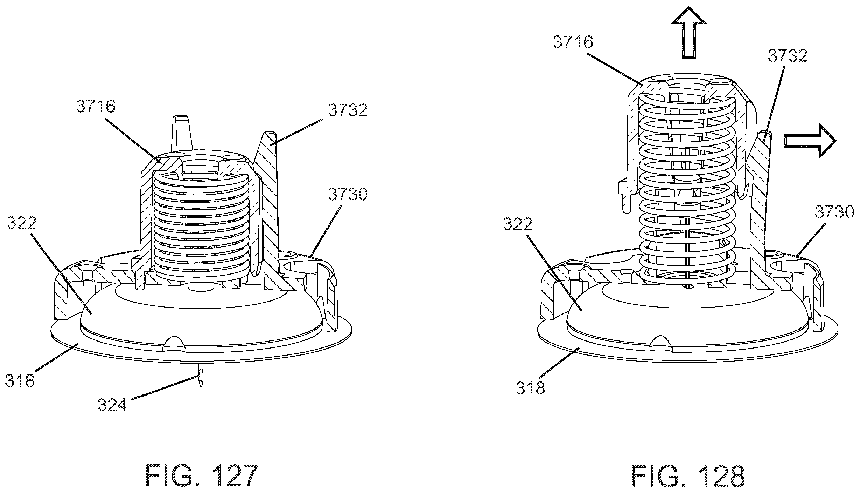

Referring next to FIGURES 120-121, depicted is sharp carrier 3716 in a perspective and cross-sectional view, respectively. Sharp carrier 3716 contains notches 3724 which allow clearance for the passage of carrier arms 3732 located on medical device carrier 3730. Guidance walls 3744 securely hold spring 3746 in place (FIGURES 127-128). The top or proximal edge of the sharp carrier includes a chamfered or sloped edge 3725. Locating features 3748, e.g., standoffs or distally extending protrusions, align with locating features 3750, e.g., recesses or apertures, on carrier 3730. Accordingly, the sharp carrier 3716 is located and secured to the medical device carrier 3730 (located via interaction of locating features 3748 and 3750 and secured via interaction of carrier arms 3732 and angled edge surface of 3725. These locating features can extend through the medical device carrier 3730 and directly engage the on body housing 322. Accordingly, when a user actuates the inserter, the sharp carrier drives the on body housing 322 and sharp 324 towards the subject via the protrusions 3748. The direct coupling of the sharp carrier enhances the control of the positioning of on body housing 322, and prevents skewing of the on body housing 322 or sharp 324. Additionally, snap features 3752 secure sharp 324 securely within inserter 3700. It is contemplated that sharp 324 may be secured to sharp carrier 3716 by other techniques, e.g., friction fit, adhesive, welding, etc.

Medical device carrier 3730 is depicted in more detail in FIGURE 122. As shown, carrier 3730 contains spring locating ring 3754 that receives one end of spring 3746. In some embodiments, spring 3746 surrounds spring locating ring 3754. In some embodiments, the inner area remains clear to leave room for the deflection of sharp carrier feature snaps 3752 that deflect out when the sharp is inserted. As described above, carrier 3730 further comprises locating features 3756 which interface with locating features on housing 3702. Furthermore, detents can be formed at the end of carrier arms 3732 of the medical device carrier to abut or otherwise the sloped edge 3725 of the sharp carrier. As described above, the detents on carrier arms 3732 are configured to engage the edge 3725 of the sharp carrier in a discrete point of contact fashion in order to realize the aforementioned sterilization advantages. Additionally, these surfaces can be configured with rounded surfaces that ensure that there is no surface to "snag" during the release of the sharp carrier. The medical device carrier 3700 further includes one or more housing gripping arms 3762 (e.g., three are depicted in FIGURE 96) which hold the on body housing 322 in place. In some embodiments, gripping arms 3762 are provided with engagement boss 3764 which are configured to engage with corresponding recesses 3766 provided on the side walls of the on body housing 322. Such engagement of the recesses 3766 with the gripping arms 3762 maintains the proper height location of the on body housing 322. Ribs 3768 or other projections on the interior surface of the distal portion 3708c of the sheath 3708 hold these gripping arms 3762 securely in place against the on body housing 322 while the sheath is fully extended. When the medical device carrier 3730 advances along the sheath 3708 to reach the proximal position during use, the gripping arms 3762 are no longer supported by the sheath 3708 and the force of the adhesive skin patch 318 overcomes the retention force of the gripping arms 3762.

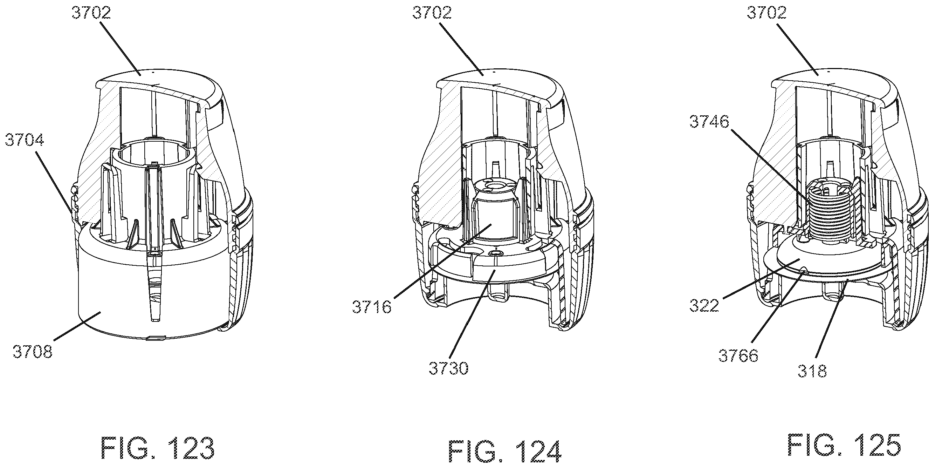

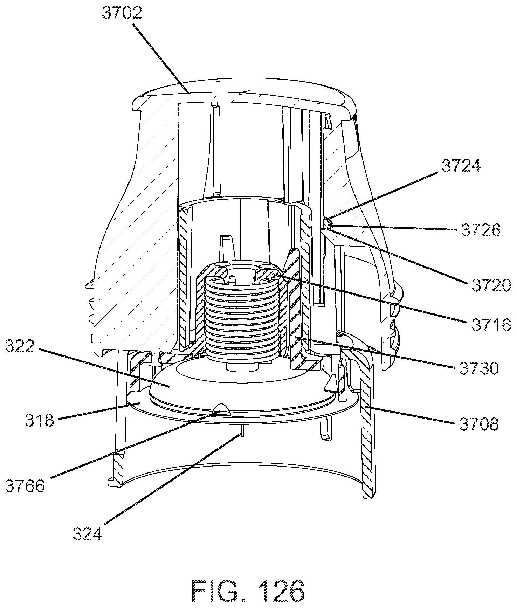

Inserter 3700 is illustrated in cross-section in FIGURES 123-125 in a state prior to use in which housing 3702 is disposed in a proximal position with respect to the sheath 3708 and the cap 3704 secured to the housing. FIGURE 126 illustrates a cross-sectional view of the inserter in a state prior to use after the cap 3704 has been removed. The upper surface of spring 3746 is retained in inserter 3700 by sharp carrier 3716. The bottom surface of spring 3746 is retained by spring location ring 3754 of the medical device carrier 3730. Initially, spring 3746 is in a compressed or semi-compressed state while housing 3702 is disposed proximally from sheath 3708.

Sharp 324 extends longitudinally from sharp carrier 3716 within inserter 3700. In some embodiments, sharp 324 is supported at an oblique angle, e.g., between and including about 0° and 90° with respect to the skin surface.

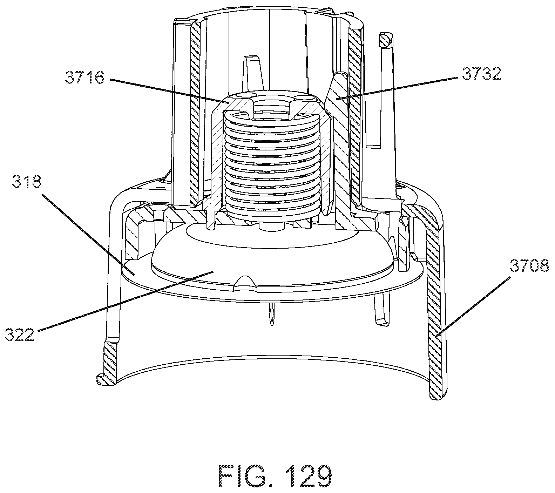

FIGURES 127-128 depict the relationship between the medical device carrier 3730 and the sharp carrier 3716 (with the housing 3702 and sheath 3708 omitted for sake of clarity). FIGURE 127 depicts the initial position of the medical device carrier 3730 and the sharp carrier 3716 with the carrier arms 3732 engaged with the sloped edge 3725 of the sharp carrier. In this position there is no relative movement between the medical device carrier 3730 and the sharp carrier 3716. However the carrier arms 3732 are not of sufficient rigidity or bias to counteract the bias of the spring 3746 in order to maintain the sharp carrier in the position shown in FIGURE 127 without support from the sheath, as shown in FIGURE 129. Accordingly, the spring 3746 urges the sharp carrier 3716 in the proximal direction thereby displacing the carrier arms 3732 radially outward as shown in FIGURE 128.

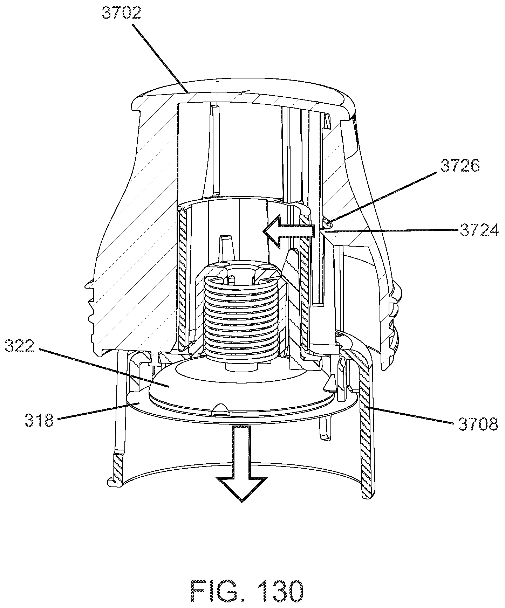

FIGURE 130 illustrates inserter 3700 in cross-section after a user applies an initial downward force to housing 3702. In some embodiments, a predetermined minimum force must be used so that attachment snaps 3726 advance past detent 3724.

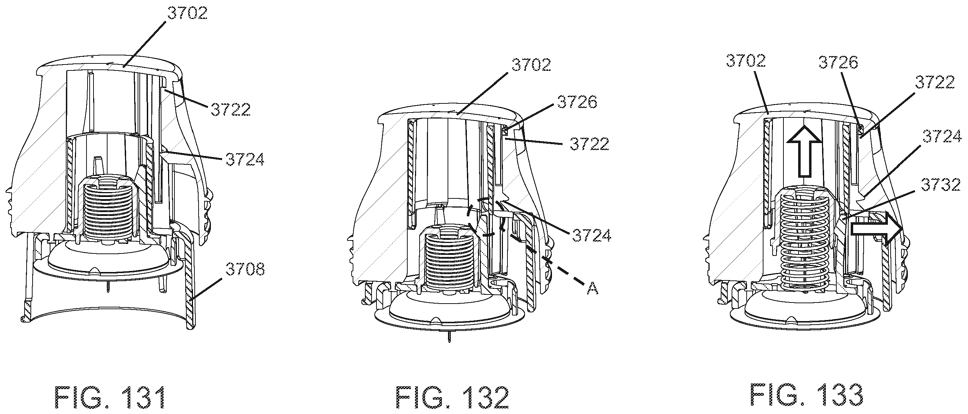

After detent 3724 has been overcome, e.g., snap 3726 of the sheath is displaced radially inward, further depression of housing 3702 with respect to sheath 3708 causes distal longitudinal movement of the medical device carrier 3730 and sharp carrier 3716, from a proximal position towards a distal position as shown in FIGURES 131-132. During this phase of insertion the interior surface of proximal portion 3708a of the sheath remains engaged with the carrier arms 3732 to prevent radial displacement of the arms 3732, and thus maintains the coupling of the medical device carrier 3730, on body housing 322, sharp 3724 and sharp carrier 3716. As sharp 324 is further urged distally, it carries the sensor insertion portion 30 of sensor 14 (FIGURE 17) into the subcutaneous portion of the subject's skin S.

As carrier 3716 reaches a distal position, the on body housing 322 along with the adhesive pad 318 engage the skin surface S of the subject, thereby becoming adhered. Concurrently, carrier arms 3732 are advanced distally beyond shelf 3708b of the sheath and clear the support wall 3708a (as highlighted by focus point "A" in FIGURE 132). This allows carrier arms 3732 to deflect radially outwardly into the larger diameter distal portion 3708c of the sheath as shown in FIGURE 133. When carrier arms 3732 deflect outwardly, shoulder portions of carrier arms 3732 are no longer in an interference relationship with the sharp carrier 3716. Thus spring 3746 is permitted to expand as shown in FIGURE 133, thereby retracting the sharp carrier 3716 to a proximal position and withdrawing the sharp 324 from the skin S of the subject while leaving the on body housing 322 attached to the skin. Housing (or handle) 3702 is maintained in the distal position and extends over the sheath in a telescoping manner. Sheath detent or snap 3726 of the sheath 3708 can then lock over feature 3722 of the housing 3702. Accordingly, the housing 3702 and the sheath 3708 can no longer move longitudinally with respect to each other.

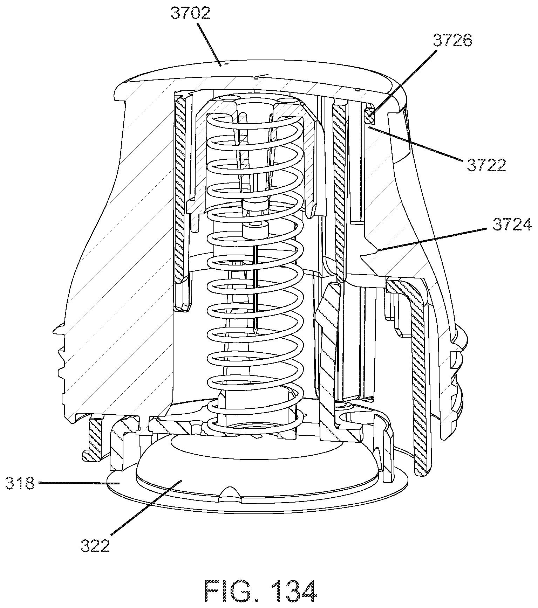

In some embodiments, the changing interaction of sheath detent or snap 3726 with the housing detent/ledges 3720, 3724, and 3722 determine whether the sheath 3708 is locked. When snap 3726 is in the pre-fire position, ledge 3720 prevents sheath 3708 from being pulled out of the housing 3702. In this position, detent 3724 may also impede the movement of pushing the sheath 3708 into the housing 3702. When the detent is overcome by a at least at minimum force, the sheath 3704 moves longitudinally with respect to the housing 3702 until the snap 3726 snaps over housing ledge 3722. At this point, ledge 3722 prevents the sheath 3708 from being pulled out of the housing again, but from a new position (this position may be referred to as the used/post-fire position). Sharp carrier snap 3752 function is to hold onto the sharp/needle, in some embodiments, the sharp/needle carrier 3716 is held in the post-fire position relative to the housing 3702 by, e.g., an interference between the rails of the housing 3714 and the guide rails of the sharp carrier 3718 (this interference is only present once the sharp carrier is fully retracted) and/or by medical device carrier projections 3732 interfering with the bottom/floor of the sharp carrier (See, e.g., FIG. 134). In another embodiment of inserter 3700, adhesive pad 318 may be attached to sheath 3708 prior to use. Upon reaching the distal position, the distal surface of on body housing 322 engages the upper surface of adhesive pad 318, thereby becoming adhered to the skin surface S of the subject.

Another embodiment of the inserter 3700' is substantially identical to the inserter 3700 discussed hereinabove with the differences noted herein. As illustrated in FIGURES 135-136, the medical device carrier 3730' is substantially identical to carrier 3730. However, carrier 3730' includes one or more gripping arms 3762' including an engagement boss 3764' which is configured to engage with corresponding recesses 3766' provided on the side walls of the on body housing 322. In some embodiments, the gripping arms 3742' are configured to be spaced radially apart from the on body housing 322 in the relaxed, unstressed configuration. When an inwardly directed force is applied to the gripping arms 3762', they may be directed into contact with the on body housing 322.

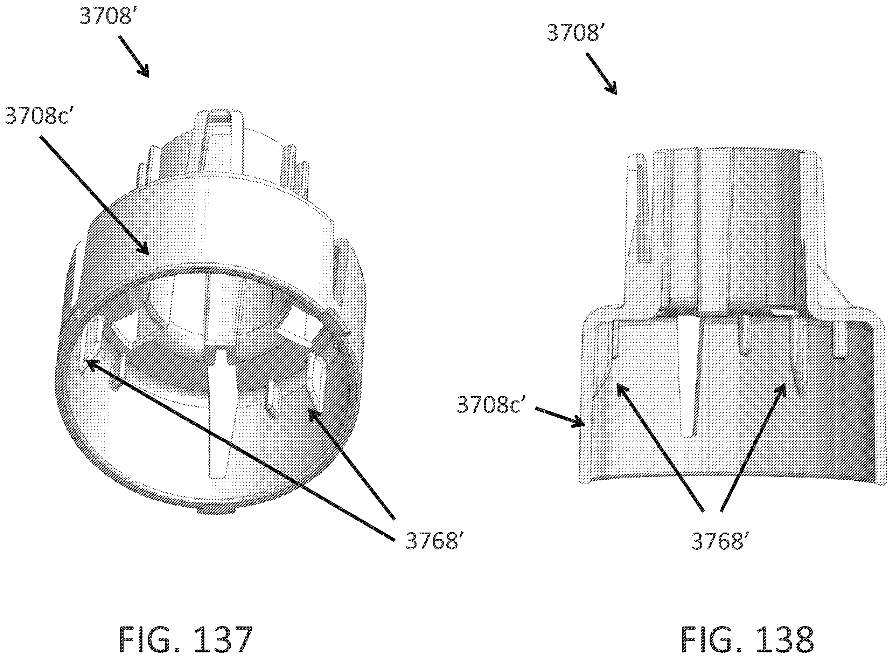

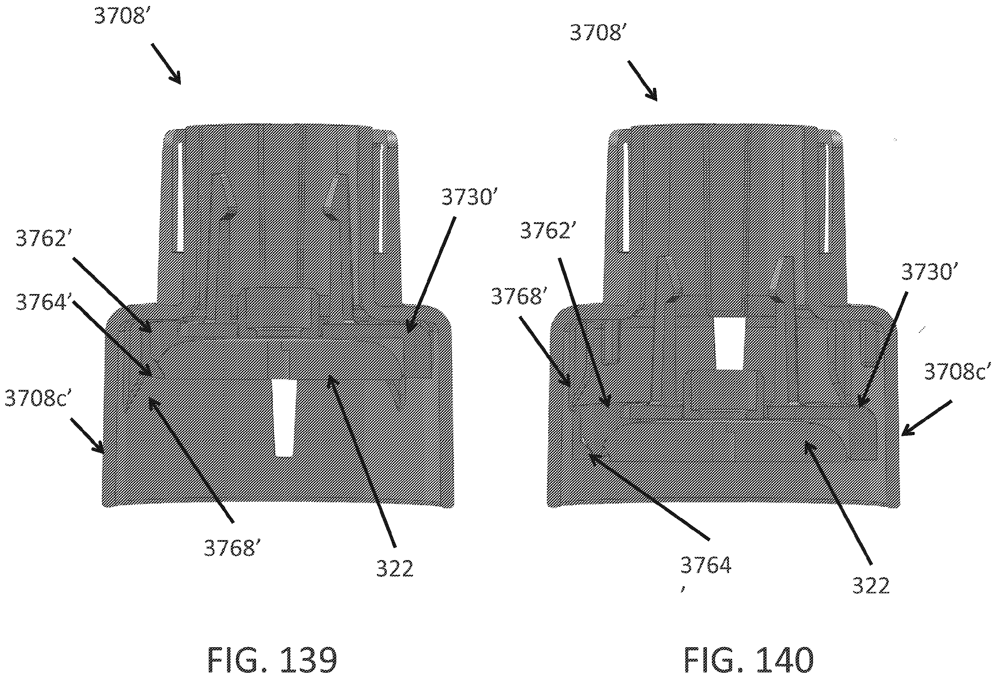

Perspective and sectional views of sheath 3708' are illustrated, respectively, in FIGURES 137-138. The inside of distal portion 3708c' includes one or more ramp members 3748', which are positioned to engage the gripping arms 3762' and provide a radially inwardly directed force. As illustrated in FIGURE 139, in the initial configuration, the medical device carrier 3730' is positioned in a proximal position with respect to the sheath 3708'. In this configuration, the gripping arms 3762' are deflected radially inwardly by the ramp member 3768' such that the engagement boss 3764' is in contact with the recesses 3766' of the on body housing 322. This configuration provides support for the on body housing 322. As illustrated in FIGURE 140, as the carrier 3730' is advanced distally, the gripping arms 3762' clear the ramp member 3768', the gripping arms 3762' begin to deflect radially outwardly according to their normal bias, thereby releasing the engagement boss 3766' from the recesses 3768' of on body housing 322. Release of the gripping arms 3762' facilitates the separation of the on body housing 322 form the inserter 3700'.

In some embodiments, the on body housing is assembled on the body of the user. For example, the on body housing may be comprised of a mounting unit 3780 and an electronics housing 3782. The mounting unit 3780 may include a mount and a sensor. In some embodiments, the sensor is at least partially positioned within the mount and the distal insertion portion extends out of the mount. An inserter, such as inserter 3700 described herein, is used to advance the distal portion of the sensor into the skin of the subject and to adhere the mount to the skin of the user. Subsequently, the electronics housing 3782 is mounted onto the mounting unit 3780. Electrical contact is made between the electronics housing 3782 and the sensor in order to transfer the analyte readings from the sensor to the electronics housing 3782.

As illustrated in FIGURE 141, the inserter 3700 is initially arranged with the cap 3704 attached to the housing 3702. The mounting unit 3780 is positioned in the medical device carrier 3730, with the sharp 324 extending distally in a surrounding position about the sensor. FIGURES 142-144 illustrate the sequence of inserting the sensor into the skin of the user and the attachment of the mounting unit 3780 to the skin of the user. In FIGURE 142, the sheath 3708 is placed on the skin. In FIGURE 143, the housing 3702 is advanced distally towards the skin of the user, thereby advancing the medical device carrier, the mounting unit 3780 and the sharp 324 towards the skin of the patient. In FIGURE 144, upon reaching the distal position of the housing 3702, the sharp carrier 3716 is released, thereby moving to the proximal position.

As illustrated in FIGURES 145a and 145b, the mounting unit 3780 is positioned on the skin with the distal portion of the sensor inserted into the skin. As illustrated in FIGURES 146a and 146b, the electronics housing 3782 is inserted into the mounting unit 3780, and shown in the final configuration in FIGURES 147a and 147b.

In an exemplary embodiment of on body housing 3800 illustrated in FIGURE 148, electronics housing 3882 is mounted on mounting unit 3880. Mounting unit 3880 includes a detent 3884 for coupling with a recess 3886 on the electronics housing 3882 in, e.g., a toe-in snap configuration. It is understood that the detent and recess configuration may be reversed such that the recess is on the mounting unit and the detent is on the electronics housing. The electronics housing 3882 electrically couples with the mounting unit 3880 by the electrical contacts 3888 on the mounting unit 3880 which are coupled to electrical contacts (not shown) on the electronics housing 3882. The sensor hub 3890 stores at least a portion of the sensor which is electrically coupled to the contacts 3888.

In another exemplary embodiment illustrated in FIGURE 149, the on body housing 3900 is attach by first attaching the mounting unit 3980 to the skin of the user. Subsequently, sensor 14 is positioned at least partially beneath the skin of the user. Electronics housing 3982 is coupled to the mounting unit 3980 by inserting the flanges 3986 under a corresponding flange of the mounting unit 3980. Contacts 3988 of the electronics housing 3982 are then coupled to contacts on the sensor 14 in order to provide the sensor readings from the sensor to the electronics housing 3982.

In some embodiments, the on body housing is assembled on a surface (such as a tabletop) prior to insertion into the user. For example, as illustrated in FIGURES 150-156, the on body housing may be comprised of a housing unit 4020 and an sensor hub 4022. The housing unit 4020 may include a mount and on body electronics 14. In some embodiments, the sensor is at least partially positioned within the sensor hub 4022 and the distal insertion portion extends out of the sensor hub 4022. The sensor hub 4022 is contained in the inserter, and the housing unit 4020 is positioned in the inserter 3700. Electrical contact is made between the housing unit 4020 and the sensor in order to transfer the analyte readings from the sensor to the housing unit 4020. The inserter, similar to inserter 3700 described herein, is used to advance the distal portion of the sensor into the skin of the subject and to adhere the housing unit 4020 to the skin of the user.

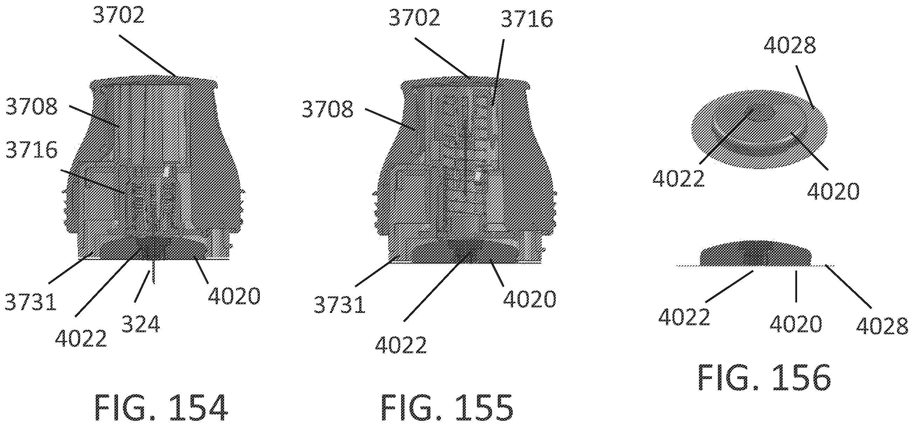

As illustrated in FIGURE 150, the inserter 3700 is initially arranged with the cap 3704 attached to the housing 3702. The sensor hub 4022 is supported by the sharp carrier 3716, with the sharp 324 extending distally in a surrounding position about the sensor. FIGURES 151-155 illustrate the sequence of inserting the sensor into the skin of the user and the attachment of the housing unit 4020 to the skin of the user. In FIGURE 151, the cap 3704 is removed. In FIGURES 152-153, the housing unit 4020 is positioned in the housing support 3731, for example, by use of adhesive patch 4028. In FIGURE 154, the sharp carrier 3716 is advanced distally, thereby advancing the sensor hub 4022 distally and into engagement with the housing unit 4020. In FIGURE 155, the sharp carrier 3716 is released, thereby allowing the sharp carrier 3716 to move proximally. The inserter 3700 is removed, leaving the sensor hub 4022 coupled to the housing unit 4020, as illustrated in FIGURE 156.

In some embodiments, the housing unit 4020 and the adhesive patch 4028 are stored in a sealed compartment 4100 as shown in FIGURE 157. The compartment 4100 includes a lower cap portion 4104 and a cover portion 4102, manufactured from a flexible material such as metal foil or plastic. As shown in FIGURE 158, the lower cap portion 4104 stores the sterilized housing unit 4020 and adhesive patch 4028 therein until ready for use. In some embodiments, the adhesive patch 4028 includes adhesive on both sides.

In some embodiments, on body housing 4200 includes housing unit 4220 and sensor hub 4222 as illustrated in FIGURE 159. The sensor may be insert molded with mechanical contacts. The PCB in the housing unit 4220 may include leaf spring contacts 4230. The sensor hub 4222 may be mechanically attached to the housing unit 4220, e.g., the electrical contacts may function as mechanical snaps. Sealing may be provided by an elastomeric gasket. The housing unit 4220 may be macromelt or injection molded.

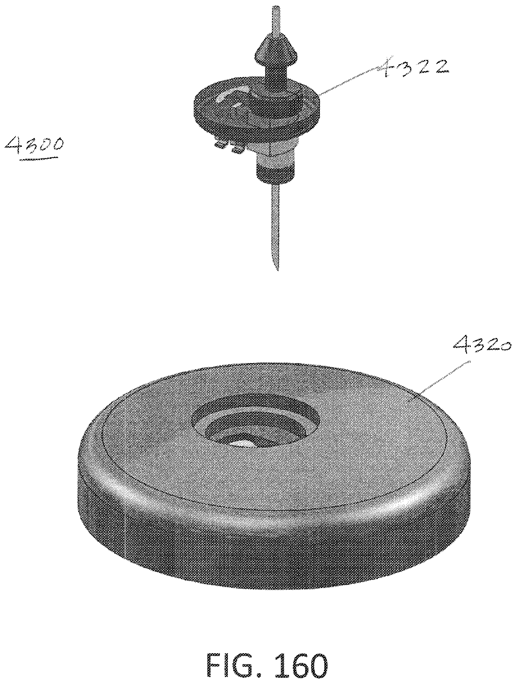

In some embodiments, on body housing 4300 includes housing unit 4320 and sensor hub 4322 as illustrated in FIGURE 160. The sensor may have insert molded contacts. The PCB in housing unit 4320 may include exposed pads. Mechanical attachment of the housing unit and sensor hub may be accomplished by molded snaps. The needle guide may be injection molded or overmolded macromelt of TPE (thermoplastic elastomer). The housing unit 4320 may be macromelt or injection molded.

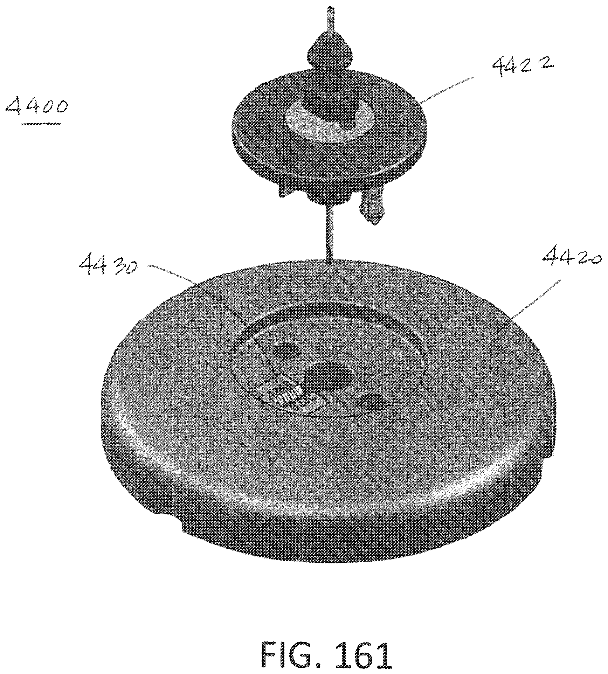

In some embodiments, on body housing 4400 includes housing unit 4420 and sensor hub 4422 as illustrated in FIGURE 161. The sensor may have exposed pads on the flag or contact portion of the sensor. The PCB in housing unit 4420 may include a SMT ZIF connector, or similar 4430. Mechanical attachment of the housing unit and sensor hub may be accomplished by molded snaps. The needle guide may be injection molded plastic with elastomer overmold. The housing unit 4320 may be macromelt overmold.

In some embodiments, a clamshell type an-angement 4500 is provided which includes a needle guide 4550 having a living hinge arrangement 4552. The sensor may include bent metal contacts that are inserted after molding. The PCB may include PCB pads. The mechanical attachment is performed by adhesive of mechanical snap to PCB. The transponder housing, not shown, may be injection molded , UV or ultrasonic bonded.

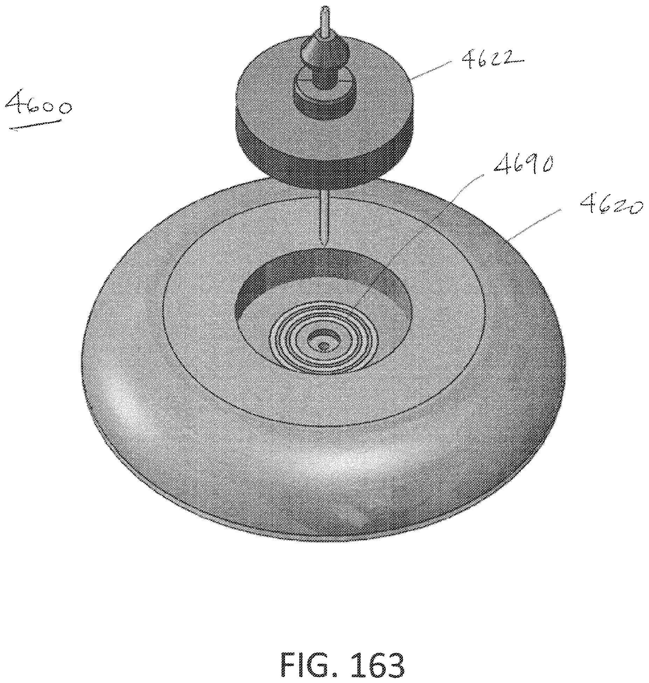

In some embodiments, on body housing 4600 includes housing unit 4620 and sensor hub 4622 as illustrated in FIGURE 163. The sensor 14 may have exposed pads on the flag or contact portion of the sensor. The PCB in housing unit 4620 may include concentric exposed pads 4690. Mechanical attachment of the housing unit and sensor hub may be accomplished by molded snaps or PSA. The needle guide may be injection molded plastic with elastomer overmold. The housing unit 4620 may be macromelt overmold.

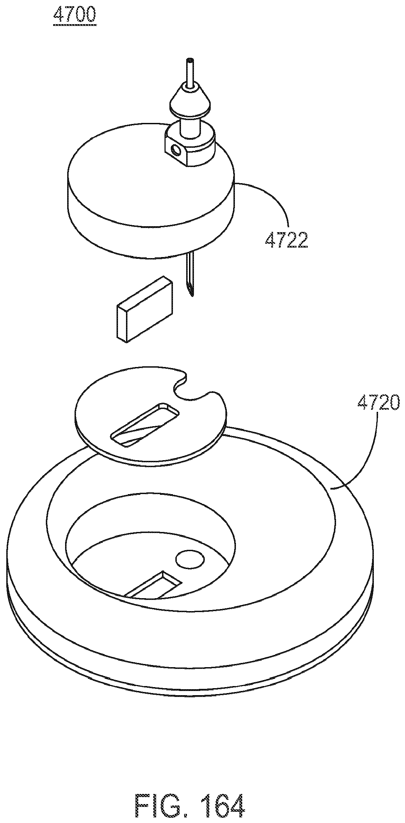

In some embodiments, on body housing 4700 includes housing unit 4720 and sensor hub 4722 as illustrated in FIGURE 164. The sensor may have exposed pads on the flag and may also include a compressed anisotropic zebra, conductive elastomeric or similar. The PCB in housing unit 4720 may include exposed pads. Mechanical attachment of the housing unit and sensor hub may be accomplished by snaps. The needle guide may overmold macromelt or TPE. The housing unit 4720 may be macromelt overmold.

It is understood that the subject matter described herein is not limited to particular embodiments described, as such may, of course, vary. It is also understood that the terminology used herein is for the purpose of describing particular embodiments only, and is not intended to be limiting, since the scope of the present subject matter is limited only by the appended claims.

## Claims

1. An insertion system (200, 300, 400, 2500, 2700, 3700, 3700') and an integrated in vivo glucose sensor and on body electronics assembly (122, 322), wherein the insertion system (200, 300, 400, 2500, 2700, 3700, 3700') comprises:
a sheath (242, 342, 442, 2512, 2708, 3708, 3708') defining a distal surface for placement on a skin surface of the subject, the sheath (242, 342, 442, 2512, 2708, 3708, 3708') having at least one biased retention feature (2726, 3726);
a device support (230, 330, 430, 2516, 2730, 3730, 3730') movable between a proximal and distal position, and adapted to support the integrated in vivo glucose sensor and on body electronics assembly (122, 322);
a sharp support movable between a proximal and a distal position and adapted to support a sharp (224, 324, 424, 2550) extending through a portion of the device support, wherein the device support includes at least one feature adapted to operatively engage the sharp support;
a handle (202, 302, 402, 2502, 2702, 3702) movable between a proximal position and a distal position relative to the sheath (242, 342, 442, 2512, 2708, 3708, 3708') and adapted to urge the device support and the sharp support from a proximal to a distal position to insert the sharp into the skin of the subject, wherein advancing the handle (202, 302, 402, 2502, 2702, 3702) distally comprises applying a minimum force to overcome the at least one biased retention feature (2726, 3726) to allow distal movement of the handle (202, 302, 402, 2502, 2702, 3702) relative to the sheath (242, 342, 442, 2512, 2708, 3708, 3708'); and
a driver for advancing the sharp support towards the proximal position when the sharp support reaches the distal position; and
wherein the integrated in vivo glucose sensor and on body electronics assembly (122, 322) comprises on body electronics (1100) configured to process one or more signals indicative of in vivo glucose levels obtained from the in vivo glucose sensor (14).

2. A method, comprising:
providing an insertion system (200, 300, 400, 2500, 2700, 3700, 3700') and an integrated in vivo glucose sensor and on body electronics assembly (122, 322), wherein the insertion system (200, 300, 400, 2500, 2700, 3700, 3700') comprises a sheath (242, 342, 442, 2512, 2708, 3708, 3708') defining a distal surface, the sheath (242, 342, 442, 2512, 2708, 3708, 3708') having at least one biased retention feature (2726, 3726), a device support (230, 330, 430, 2516, 2730, 3730, 3730') adapted to support the integrated in vivo glucose sensor and on body electronics assembly (122, 322), a sharp support adapted to support a sharp (224, 324, 424, 2550) extending through a portion of the device support, wherein the device support includes at least one feature adapted to operatively engage the sharp support, a handle (202, 302, 402, 2502, 2702, 3702) movable relative to the sheath (242, 342, 442, 2512, 2708, 3708, 3708'), and a driver for displacing the sharp support and the sharp (224, 324, 424, 2550), and wherein the integrated in vivo glucose sensor and on body electronics assembly (122, 322) comprises on body electronics (1100) configured to process one or more signals indicative of in vivo glucose levels obtained from the in vivo glucose sensor (14);
disposing the distal surface of the sheath (242, 342, 442, 2512, 2708, 3708, 3708') on a skin surface of the subject;
displacing the handle (202, 302, 402, 2502, 2702, 3702) in a first longitudinal direction to urge the device support, the integrated in vivo glucose sensor and on body electronics assembly (122, 322), the sharp support, and the sharp (224, 324, 424, 2550) in the first longitudinal direction to insert the sharp (224, 324, 424, 2550) and the portion of the in vivo glucose sensor (14) under the skin surface, wherein the first longitudinal direction is from a proximal to a distal position, and wherein displacing the handle (202, 302, 402, 2502, 2702, 3702) comprises applying a minimum force to overcome the at least one biased retention feature (2726, 3726) to allow distal movement of the handle (202, 302, 402, 2502, 2702, 3702) relative to the sheath (242, 342, 442, 2512, 2708, 3708, 3708');
releasing the driver; and
displacing the sharp support in a second longitudinal direction by the driver, wherein the second longitudinal direction is from a distal to a proximal position.

3. The insertion system and integrated in vivo glucose sensor and on body electronics assembly of claim 1, or the method of claim 2, further comprising an insertion driver for displacing the device support (230, 330, 430, 2516, 2730, 3730, 3730'), the integrated in vivo glucose sensor and on body electronics assembly (122, 322), the sharp support, and the sharp (224, 324, 424, 2550) in the first longitudinal direction to insert the sharp (224, 324, 424, 2550) and the portion of the in vivo glucose sensor (14) under the skin surface.

4. The insertion system and integrated in vivo glucose sensor and on body electronics assembly of claim 1 or 3, or the method of claim 2 or 3, wherein the integrated in vivo glucose sensor and on body electronics assembly (122, 322) comprises a housing, and optionally wherein the on body electronics (1100) and a portion of the in vivo glucose sensor (14) are received in the housing of the integrated in vivo glucose sensor and on body electronics assembly (122, 322).

5. The insertion system and integrated in vivo glucose sensor and on body electronics assembly of claim 4, or the method of claim 4, wherein the housing of the integrated in vivo glucose sensor and on body electronics assembly (122, 322) comprises a macromelt overmold.

6. The insertion system and integrated in vivo glucose sensor and on body electronics assembly of any of claims 1 or 3 to 5, or the method of any of claims 2 to 5, wherein the sharp (224, 324, 424, 2550) extends through an aperture (168) in the integrated in vivo glucose sensor and on body electronics assembly (122, 322).

7. The insertion system and integrated in vivo glucose sensor and on body electronics assembly of any of claims 1 or 3 to 6, or the method of any of claims 2 to 6, wherein the integrated in vivo glucose sensor and on body electronics assembly (122, 322) comprises an adhesive layer (218, 318, 418, 2718, 118) for attachment to the skin of the subject.

8. The insertion system and integrated in vivo glucose sensor and on body electronics assembly of any of claims 1 or 3 to 7, or the method of any of claims 2 to 7, wherein the integrated in vivo glucose sensor and on body electronics assembly (122, 322) comprises a power supply (82).

9. The insertion system and integrated in vivo glucose sensor and on body electronics assembly of any of claims 1 or 3 to 8, or the method of any of claims 2 to 8, wherein the on body electronics (1100) comprises one or more printed circuit boards, optionally wherein the one or more printed circuit boards comprises control logic implemented in ASIC, a microprocessor, and memory.

10. The insertion system and integrated in vivo glucose sensor and on body electronics assembly of any of claims 1 or 3 to 9, or the method of any of claims 2 to 9, wherein the integrated in vivo glucose sensor and on body electronics assembly (122, 322) is configured to communicate glucose information to a monitor unit (1200), optionally wherein the integrated in vivo glucose sensor and on body electronics assembly (122, 322) is configured to provide temperature information to the monitor unit (1200).

11. The insertion system and integrated in vivo glucose sensor and on body electronics assembly of claim 10, or the method of claim 10, wherein the integrated in vivo glucose sensor and on body electronics assembly (122, 322) is configured to communicate the glucose information to the monitor unit (1200) when the integrated in vivo glucose sensor and on body electronics assembly (122, 322) receives a request from the monitor unit (1200).

12. The insertion system and integrated in vivo glucose sensor and on body electronics assembly of any of claims 1 or 3 to 11, or the method of any of claims 2 to 11, further comprising activating the on body electronics (1100) by the insertion system (200, 300, 400, 2500, 2700, 3700, 3700').

13. The insertion system and integrated in vivo glucose sensor and on body electronics assembly of any of claims 1 or 3 to 12, or the method of any of claims 2 to 12, wherein advancing the sharp (224, 324, 424, 2550) towards the proximal position comprises retracting the sharp (224, 324, 424, 2550) while the integrated in vivo glucose sensor and on body electronics assembly (122, 322) remains on the subject.

14. The insertion system and integrated in vivo glucose sensor and on body electronics assembly of any of claims 1 or 3 to 13, or the method of any of claims 2 to 13, wherein advancing the sharp (224, 324, 424, 2550) towards the proximal position comprises automatically retracting the sharp (224, 324, 424, 2550).

15. The insertion system and integrated in vivo glucose sensor and on body electronics assembly of any of claims 1 or 3 to 14, or the method of any of claims 2 to 14, wherein advancing the sharp (224, 324, 424, 2550) towards the proximal position comprises automatically retracting the sharp (224, 324, 424, 2550) upon advancement of the sharp (224, 324, 424, 2550) and the device support (230, 330, 430, 2516, 2730, 3730, 3730') by a predetermined amount.

16. The insertion system and integrated in vivo glucose sensor and on body electronics assembly of any of claims 1 or 3 to 15, or the method of any of claims 2 to 15, wherein the driver comprises a spring (246, 346, 446, 2544, 2746, 3746).

17. The insertion system and integrated in vivo glucose sensor and on body electronics assembly of any of claims 1 or 3 to 16, or the method of any of claims 2 to 16, wherein the device support (230, 330, 430, 2516, 2730, 3730, 3730') comprises one or more movable arms (2542, 3762, 3762') for engaging the integrated in vivo glucose sensor and on body electronics assembly (122, 322), optionally wherein the insertion system (200, 300, 400, 2500, 2700, 3700, 3700') is configured to displace the one or more movable arms (2542, 3762, 3762') radially outwardly to release the integrated in vivo glucose sensor and on body electronics assembly (122, 322) from the device support (230, 330, 430, 2516, 2730, 3730, 3730').

18. The insertion system and integrated in vivo glucose sensor and on body electronics assembly of claim 17, or the method of claim 17, wherein the one or more movable arms (2542, 3762, 3762') comprises three arms.

19. The insertion system and integrated in vivo glucose sensor and on body electronics assembly of claim 17 or 18 when dependent on claim 4, or the method of claim 17 or 18 when dependent on claim 4, wherein each of the gripping arms comprises an engagement boss configured to engage with a corresponding recess (3766, 3766'), the recesses (3766, 3766') provided on side walls of the housing of the integrated in vivo glucose sensor and on body electronics assembly (122, 322).

20. The insertion system and integrated in vivo glucose sensor and on body electronics assembly of any of claims 1 or 3 to 19, or the method of any of claims 2 to 19, further comprising a removable distal cap (204, 304, 404, 2504, 2704, 3704) detachably secured to the handle (202, 302, 402, 2502, 2702, 3702), optionally wherein the removable distal cap (204, 304, 404, 2504, 2704, 3704) is secured to the handle (202, 302, 402, 2502, 2702, 3702) by threads (210, 310, 311, 410, 411, 2506, 2706, 3706).

21. The insertion system and integrated in vivo glucose sensor and on body electronics assembly of any of claims 1 or 3 to 20, or the method of any of claims 2 to 20, wherein the sharp (224, 324, 424, 2550) extends longitudinally within the insertion system (200, 300, 400, 2500, 2700, 3700, 3700') and the portion of the in vivo glucose sensor (14) that is inserted under the skin surface is substantially aligned with a longitudinal axis of the insertion system (200, 300, 400, 2500, 2700, 3700, 3700') and substantially perpendicular to the skin surface prior to insertion.

22. The insertion system and integrated in vivo glucose sensor and on body electronics assembly of any of claims 1 or 3 to 21, or the method of any of claims 2 to 21, wherein the in vivo glucose sensor (14) is configured to bend such that it is positioned in two substantially perpendicular, intersecting planes, and wherein the portion of the in vivo glucose sensor (14) that is inserted under the skin surface is substantially vertically aligned with a longitudinal axis of the insertion system (200, 300, 400, 2500, 2700, 3700, 3700') and substantially perpendicular to the skin surface prior to insertion, and wherein a contact portion is orientated in a substantially horizontal configuration and in electrical contact with the on body electronics (1100).

23. The insertion system and integrated in vivo glucose sensor and on body electronics assembly of any of claims 1 or 3 to 22, or the method of any of claims 2 to 22, wherein the biased retention feature (2726, 3726) is biased against the handle (202, 302, 402, 2502, 2702, 3702).

## Patentansprüche

1. Einsetzsystem (200, 300, 400, 2500, 2700, 3700, 3700') und eine integrierte In-vivo-Glucosesensor- und Am-Körper-Elektronikanordnung (122, 322), wobei das Einsetzsystem (200, 300, 400, 2500, 2700, 3700, 3700') Folgendes umfasst:
eine Hülle (242, 342, 442, 2512, 2708, 3708, 3708'), die eine distale Oberfläche zur Platzierung auf einer Hautoberfläche des Probanden definiert, wobei die Hülle (242, 342, 442, 2512, 2708, 3708, 3708') mindestens ein vorgespanntes Halteelement (2726, 3726) aufweist;
einen Vorrichtungsträger (230, 330, 430, 2516, 2730, 3730, 3730'), der zwischen einer proximalen und einer distalen Position bewegbar ist und dazu angepasst ist, die integrierte In-vivo-Glucosesensor- und Am-Körper-Elektronikanordnung (122, 322) zu tragen;
einen Träger des scharfen Elementes, der zwischen einer proximalen und einer distalen Position bewegbar ist und dazu angepasst ist, ein scharfes Element (224, 324, 424, 2550) zu tragen, das sich durch einen Abschnitt des Vorrichtungsträgers erstreckt, wobei der Vorrichtungsträger mindestens ein Element enthält, das dazu angepasst ist, mit dem Träger des scharfen Elementes betriebsmäßig in Eingriff zu kommen;
einen Griff (202, 302, 402, 2502, 2702, 3702), der relativ zur Hülle (242, 342, 442, 2512, 2708, 3708, 3708') zwischen einer proximalen Position und einer distalen Position bewegbar ist und dazu angepasst ist, den Vorrichtungsträger und den Träger des scharfen Elementes von einer proximalen in eine distale Position zu drücken, um das scharfe Element in die Haut des Probanden einzusetzen, wobei das Vorrücken des Griffs (202, 302, 402, 2502, 2702, 3702) distal das Aufbringen einer minimalen Kraft umfasst, um das mindestens eine vorgespannte Halteelement (2726, 3726) zu überwinden, um eine distale Bewegung des Griffs (202, 302, 402, 2502, 2702, 3702) relativ zur Hülle (242, 342, 442, 2512, 2708, 3708, 3708') zu ermöglichen; und
einen Treiber zum Vorrücken des Trägers des scharfen Elementes zur proximalen Position, wenn der Träger des scharfen Elementes die distale Position erreicht; und
wobei die integrierte In-vivo-Glucosesensor- und Am-Körper-Elektronikanordnung (122, 322) eine Am-Körper-Elektronik (1100) umfasst, die dazu konfiguriert ist, ein oder mehrere Signale zu verarbeiten, die auf In-vivo-Glucosespiegel hinweisen, die vom In-vivo-Glucosesensor (14) erhalten wurden.

2. Verfahren, umfassend:
Bereitstellen eines Einsetzsystems (200, 300, 400, 2500, 2700, 3700, 3700') und einer integrierten In-vivo-Glucosesensor- und Am-Körper-Elektronikanordnung (122, 322), wobei das Einsetzsystem (200, 300, 400, 2500, 2700, 3700, 3700') eine Hülle (242, 342, 442, 2512, 2708, 3708, 3708'), die eine distale Oberfläche definiert, wobei die Hülle (242, 342, 442, 2512, 2708, 3708, 3708') mindestens ein vorgespanntes Halteelement (2726, 3726) aufweist, einen Vorrichtungsträger (230, 330, 430, 2516, 2730, 3730, 3730'), der dazu angepasst ist, die integrierte In-vivo-Glucosesensor- und Am-Körper-Elektronikanordnung (122, 322) zu tragen, einen Träger des scharfen Elementes, der dazu angepasst ist, ein scharfes Element (224, 324, 424, 2550) zu tragen, das sich durch einen Abschnitt des Vorrichtungsträgers erstreckt, wobei der Vorrichtungsträger mindestens ein Element enthält, das dazu angepasst ist, mit dem Träger des scharfen Elementes betriebsmäßig in Eingriff zu kommen, einen Griff (202, 302, 402, 2502, 2702, 3702), der relativ zur Hülle (242, 342, 442, 2512, 2708, 3708, 3708') bewegbar ist, und einen Treiber zum Vorrücken des Trägers des scharfen Elementes und des scharfen Elementes (224, 324, 424, 2550), und wobei die integrierte In-vivo-Glucosesensor- und Am-Körper-Elektronikanordnung (122, 322) eine Am-Körper-Elektronik (1100) umfasst, die dazu konfiguriert ist, ein oder mehrere Signale zu verarbeiten, die auf In-vivo-Glucosespiegel hinweisen, die vom In-vivo-Glucosesensor (14) erhalten wurden, umfasst;
Anlegen der distalen Oberfläche der Hülle (242, 342, 442, 2512, 2708, 3708, 3708') auf eine Hautoberfläche des Probanden;
Verschieben des Griffs (202, 302, 402, 2502, 2702, 3702) in einer ersten Längsrichtung, um den Vorrichtungsträger, die integrierte In-vivo-Glucosesensor- und Am-Körper-Elektronikanordnung (122, 322), den Träger des scharfen Elementes und das scharfe Element (224, 324, 424, 2550) in die erste Längsrichtung zu drücken, um das scharfe Element (224, 324, 424, 2550) und den Abschnitt des In-vivo-Glucosesensors (14) unter die Hautoberfläche einzusetzen, wobei die erste Längsrichtung von einer proximalen zu einer distalen Position verläuft und wobei das Verschieben des Griffs (202, 302, 402, 2502, 2702, 3702) das Aufbringen einer minimalen Kraft umfasst, um das mindestens eine vorgespannte Halteelement (2726, 3726) zu überwinden, um eine distale Bewegung des Griffs (202, 302, 402, 2502, 2702, 3702) relativ zur Hülle (242, 342, 442, 2512, 2708, 3708, 3708') zu ermöglichen;
Lösen des Treibers; und
Verschieben des Trägers des scharfen Elementes in einer zweiten Längsrichtung durch den Treiber, wobei die zweite Längsrichtung von einer distalen zu einer proximalen Position verläuft.

3. Einsetzsystem und integrierte In-vivo-Glucosesensor- und Am-Körper-Elektronikanordnung nach Anspruch 1 oder Verfahren nach Anspruch 2, ferner umfassend einen Einsetztreiber zum Verschieben des Vorrichtungsträgers (230, 330, 430, 2516, 2730, 3730, 3730'), der integrierten In-vivo-Glucosesensor- und Am-Körper-Elektronikanordnung (122, 322), des Trägers des scharfen Elementes und des scharfen Elementes (224, 324, 424, 2550) in der ersten Längsrichtung, um das scharfe Element (224, 324, 424, 2550) und den Abschnitt des In-vivo-Glucosesensors (14) unter die Hautoberfläche einzusetzen.

4. Einsetzsystem und integrierte In-vivo-Glucosesensor- und Am-Körper-Elektronikanordnung nach Anspruch 1 oder 3 oder Verfahren nach Anspruch 2 oder 3, wobei die integrierte In-vivo-Glucosesensor- und Am-Körper-Elektronikanordnung (122, 322) ein Gehäuse umfasst, und wobei optional die Am-Körper-Elektronik (1100) und ein Abschnitt des In-vivo-Glucosesensors (14) im Gehäuse der integrierten In-vivo-Glucosesensor- und Am-Körper-Elektronikanordnung (122, 322) aufgenommen werden.

5. Einsetzsystem und integrierte In-vivo-Glucosesensor- und Am-Körper-Elektronikanordnung nach Anspruch 4 oder Verfahren nach Anspruch 4, wobei das Gehäuse der integrierten In-vivo-Glucosesensor- und Am-Körper-Elektronikanordnung (122, 322) eine Macromelt-Umspritzung umfasst.

6. Einsetzsystem und integrierte In-vivo-Glucosesensor- und Am-Körper-Elektronikanordnung nach einem der Ansprüche 1 oder 3 bis 5 oder Verfahren nach einem der Ansprüche 2 bis 5, wobei sich das scharfe Element (224, 324, 424, 2550) durch eine Öffnung (168) in der integrierten In-vivo-Glucosesensor- und Am-Körper-Elektronikanordnung (122, 322) erstreckt.

7. Einsetzsystem und integrierte In-vivo-Glucosesensor- und Am-Körper-Elektronikanordnung nach einem der Ansprüche 1 oder 3 bis 6 oder Verfahren nach einem der Ansprüche 2 bis 6, wobei die integrierte In-vivo-Glucosesensor- und Am-Körper-Elektronikanordnung (122, 322) eine Klebeschicht (218, 318, 418, 2718, 118) zur Befestigung an der Haut des Probanden umfasst.

8. Einsetzsystem und integrierte In-vivo-Glucosesensor- und Am-Körper-Elektronikanordnung nach einem der Ansprüche 1 oder 3 bis 7 oder Verfahren nach einem der Ansprüche 2 bis 7, wobei die integrierte In-vivo-Glucosesensor- und Am-Körper-Elektronikanordnung (122, 322) eine Stromversorgung (82) umfasst.

9. Einsetzsystem und integrierte In-vivo-Glucosesensor- und Am-Körper-Elektronikanordnung nach einem der Ansprüche 1 oder 3 bis 8 oder Verfahren nach einem der Ansprüche 2 bis 8, wobei die Am-Körper-Elektronik (1100) eine oder mehrere Leiterplatten umfasst, optional wobei die eine oder mehrere Leiterplatten eine in einem ASIC implementierte Steuerlogik, einen Mikroprozessor und einen Speicher umfassen.

10. Einsetzsystem und integrierte In-vivo-Glucosesensor- und Am-Körper-Elektronikanordnung nach einem der Ansprüche 1 oder 3 bis 9 oder Verfahren nach einem der Ansprüche 2 bis 9, wobei die integrierte In-vivo-Glucosesensor- und Am-Körper-Elektronikanordnung (122, 322) dazu konfiguriert ist, Glucoseinformationen an eine Überwachungseinheit (1200) zu kommunizieren, wobei die integrierte In-vivo-Glucosesensor- und Am-Körper-Elektronikanordnung (122, 322) optional dazu konfiguriert ist, Temperaturinformationen an die Überwachungseinheit (1200) bereitzustellen.

11. Einsetzsystem und integrierte In-vivo-Glucosesensor- und Am-Körper-Elektronikanordnung nach Anspruch 10 oder Verfahren nach Anspruch 10, wobei die integrierte In-vivo-Glucosesensor- und Am-Körper-Elektronikanordnung (122, 322) dazu konfiguriert ist, die Glucoseinformationen an die Überwachungseinheit (1200) zu kommunizieren, wenn die integrierte In-vivo-Glucosesensor- und Am-Körper-Elektronikanordnung (122, 322) eine Anforderung von der Überwachungseinheit (1200) empfängt.

12. Einsetzsystem und integrierte In-vivo-Glucosesensor- und Am-Körper-Elektronikanordnung nach einem der Ansprüche 1 oder 3 bis 11 oder Verfahren nach einem der Ansprüche 2 bis 11, ferner umfassend das Aktivieren der Am-Körper-Elektronik (1100) durch das Einsetzsystem (200, 300, 400, 2500, 2700, 3700, 3700').

13. Einsetzsystem und integrierte In-vivo-Glucosesensor- und Am-Körper-Elektronikanordnung nach einem der Ansprüche 1 oder 3 bis 12 oder Verfahren nach einem der Ansprüche 2 bis 12, wobei das Vorrücken des scharfen Elementes (224, 324, 424, 2550) zur proximalen Position das Zurückziehen des scharfen Elementes (224, 324, 424, 2550) umfasst, während die integrierte In-vivo-Glucosesensor- und Am-Körper-Elektronikanordnung (122, 322) am Probanden verbleibt.

14. Einsetzsystem und integrierte In-vivo-Glucosesensor- und Am-Körper-Elektronikanordnung nach einem der Ansprüche 1 oder 3 bis 13 oder Verfahren nach einem der Ansprüche 2 bis 13, wobei das Vorrücken des scharfen Elementes (224, 324, 424, 2550) zur proximalen Position das automatische Zurückziehen des scharfen Elementes (224, 324, 424, 2550) umfasst.

15. Einsetzsystem und integrierte In-vivo-Glucosesensor- und Am-Körper-Elektronikanordnung nach einem der Ansprüche 1 oder 3 bis 14 oder Verfahren nach einem der Ansprüche 2 bis 14, wobei das Vorrücken des scharfen Elementes (224, 324, 424, 2550) zur proximalen Position das automatische Zurückziehen des scharfen Elementes (224, 324, 424, 2550) beim Vorrücken des scharfen Elementes (224, 324, 424, 2550) und des Vorrichtungsträgers (230, 330, 430, 2516, 2730, 3730, 3730') um einen vorbestimmten Betrag umfasst.

16. Einsetzsystem und integrierte In-vivo-Glucosesensor- und Am-Körper-Elektronikanordnung nach einem der Ansprüche 1 oder 3 bis 15 oder Verfahren nach einem der Ansprüche 2 bis 15, wobei der Treiber eine Feder (246, 346, 446, 2544, 2746, 3746) umfasst.

17. Einsetzsystem und integrierte In-vivo-Glucosesensor- und Am-Körper-Elektronikanordnung nach einem der Ansprüche 1 oder 3 bis 16 oder Verfahren nach einem der Ansprüche 2 bis 16, wobei der Vorrichtungsträger (230, 330, 430, 2516, 2730, 3730, 3730') einen oder mehrere bewegliche Arme (2542, 3762, 3762') zum Eingriff mit der integrierten In-vivo-Glucosesensor- und Am-Körper-Elektronikanordnung (122, 322) umfasst, optional wobei das Einsetzsystem (200, 300, 400, 2500, 2700, 3700, 3700') dazu konfiguriert ist, den einen oder die mehreren beweglichen Arme (2542, 3762, 3762') radial nach außen zu verschieben, um die integrierte In-vivo-Glucosesensor- und Am-Körper-Elektronikanordnung (122, 322) von dem Vorrichtungsträger (230, 330, 430, 2516, 2730, 3730, 3730') zu lösen.

18. Einsetzsystem und integrierte In-vivo-Glucosesensor- und Am-Körper-Elektronikanordnung nach Anspruch 17 oder Verfahren nach Anspruch 17, wobei der eine oder die mehreren beweglichen Arme (2542, 3762, 3762') drei Arme umfassen.

19. Einsetzsystem und integrierte In-vivo-Glucosesensor- und Am-Körper-Elektronikanordnung nach Anspruch 17 oder 18, sofern von Anspruch 4 abhängig, oder Verfahren nach Anspruch 17 oder 18, sofern von Anspruch 4 abhängig, wobei jeder der Greifarme einen Eingriffsvorsprung umfasst, der dazu konfiguriert ist, in eine entsprechende Aussparung (3766, 3766') einzugreifen, wobei die Aussparungen (3766, 3766') an den Seitenwänden des Gehäuses der integrierten In-vivo-Glucosesensor- und Am-Körper-Elektronikanordnung (122, 322) bereitgestellt werden.

20. Einsetzsystem und integrierte In-vivo-Glucosesensor- und Am-Körper-Elektronikanordnung nach einem der Ansprüche 1 oder 3 bis 19 oder Verfahren nach einem der Ansprüche 2 bis 19, ferner umfassend eine entfernbare distale Kappe (204, 304, 404, 2504, 2704, 3704), die abnehmbar am Griff (202, 302, 402, 2502, 2702, 3702) gesichert ist, optional wobei die entfernbare distale Kappe (204, 304, 404, 2504, 2704, 3704) am Griff (202, 302, 402, 2502, 2702, 3702) durch Gewinde (210, 310, 311, 410, 411, 2506, 2706, 3706) gesichert ist.

21. Einsetzsystem und integrierte In-vivo-Glucosesensor- und Am-Körper-Elektronikanordnung nach einem der Ansprüche 1 oder 3 bis 20 oder Verfahren nach einem der Ansprüche 2 bis 20, wobei sich das scharfe Element (224, 324, 424, 2550) längs innerhalb des Einsetzsystems (200, 300, 400, 2500, 2700, 3700, 3700') erstreckt und der Abschnitt des In-vivo-Glucosesensors (14), der unter die Hautoberfläche eingesetzt wird, im Wesentlichen auf eine Längsachse des Einsetzsystems (200, 300, 400, 2500, 2700, 3700, 3700') ausgerichtet ist und vor dem Einsetzen im Wesentlichen senkrecht zur Hautoberfläche steht.

22. Einsetzsystem und integrierte In-vivo-Glucosesensor- und Am-Körper-Elektronikanordnung nach einem der Ansprüche 1 oder 3 bis 21 oder Verfahren nach einem der Ansprüche 2 bis 21, wobei der In-vivo-Glucosesensor (14) dazu konfiguriert ist, sich derart zu biegen, dass er in zwei im Wesentlichen senkrechten, sich schneidenden Ebenen positioniert ist, und wobei der Abschnitt des In-vivo-Glucosesensors (14), der unter die Hautoberfläche eingesetzt wird, im Wesentlichen vertikal auf eine Längsachse des Einsetzsystems (200, 300, 400, 2500, 2700, 3700, 3700') ausgerichtet ist und vor dem Einsetzen im Wesentlichen senkrecht zur Hautoberfläche steht, und wobei ein Kontaktabschnitt in einer im Wesentlichen horizontalen Konfiguration orientiert ist und in elektrischem Kontakt mit der Am-Körper-Elektronik (1100) steht.

23. Einsetzsystem und integrierte In-vivo-Glucosesensor- und Am-Körper-Elektronikanordnung nach einem der Ansprüche 1 oder 3 bis 22 oder Verfahren nach einem der Ansprüche 2 bis 22, wobei das vorgespannte Halteelement (2726, 3726) gegen den Griff (202, 302, 402, 2502, 2702, 3702) vorgespannt ist.

## Revendications

1. Système d'insertion (200, 300, 400, 2500, 2700, 3700, 3700') et un ensemble intégré de capteur de glucose in vivo et d'électronique corporelle (122, 322), dans lesquels le système d'insertion (200, 300, 400, 2500, 2700, 3700, 3700') comprend :
une gaine (242, 342, 442, 2512, 2708, 3708, 3708') définissant une surface distale pour un placement sur une surface cutanée du sujet, la gaine (242, 342, 442, 2512, 2708, 3708, 3708') ayant au moins une caractéristique de retenue sollicitée (2726, 3726) ;
un support de dispositif (230, 330, 430, 2516, 2730, 3730, 3730') capable de mouvement entre une position proximale et distale, et conçu pour supporter l'ensemble intégré de capteur de glucose in vivo et d'électronique corporelle (122, 322) ;
un support d'élément tranchant capable de mouvement entre une position proximale et distale et conçu pour supporter un élément tranchant (224, 324, 424, 2550) s'étendant à travers une partie du support de dispositif, dans lesquels le support de dispositif inclut au moins une caractéristique conçue pour entrer en prise de manière fonctionnelle avec le support d'élément tranchant ;
une poignée (202, 302, 402, 2502, 2702, 3702) capable de mouvement entre une position proximale et une position distale par rapport à la gaine (242, 342, 442, 2512, 2708, 3708, 3708') et conçue pour pousser le support de dispositif et le support d'élément tranchant d'une position proximale à distale pour insérer l'élément tranchant dans la peau du sujet, dans lesquels l'avancée de la poignée (202, 302, 402, 2502, 2702, 3702) de manière distale comprend l'application d'une force minimale pour outrepasser l'au moins une caractéristique de retenue sollicitée (2726, 3726) pour permettre le mouvement distal de la poignée (202, 302, 402, 2502, 2702, 3702) par rapport à la gaine (242, 342, 442, 2512, 2708, 3708, 3708') ; et
un dispositif d'entraînement destiné à avancer le support d'élément tranchant vers la position proximale lorsque le support d'élément tranchant atteint la position distale ; et
dans lesquels l'ensemble intégré de capteur de glucose in vivo et d'électronique corporelle (122, 322) comprend une électronique corporelle (1100) configurée pour traiter un ou plusieurs signaux indiquant des niveaux de glucose in vivo obtenus à partir du capteur de glucose in vivo (14).

2. Procédé comprenant :
la fourniture d'un système d'insertion (200, 300, 400, 2500, 2700, 3700, 3700') et d'un ensemble intégré de capteur de glucose in vivo et d'électronique corporelle (122, 322), dans lequel le système d'insertion (200, 300, 400, 2500, 2700, 3700, 3700') comprend une gaine (242, 342, 442, 2512, 2708, 3708, 3708') définissant une surface distale, la gaine (242, 342, 442, 2512, 2708, 3708, 3708') ayant au moins une caractéristique de retenue sollicitée (2726, 3726), un support de dispositif (230, 330, 430, 2516, 2730, 3730, 3730') conçu pour supporter l'ensemble intégré de capteur de glucose in vivo et d'électronique corporelle (122, 322), un support d'élément tranchant conçu pour supporter un élément tranchant (224, 324, 424, 2550) s'étendant à travers une partie du support de dispositif, dans lequel le support de dispositif inclut au moins une caractéristique conçue pour entrer en prise de manière fonctionnelle avec le support d'élément tranchant, une poignée (202, 302, 402, 2502, 2702, 3702) capable de mouvement par rapport à la gaine (242, 342, 442, 2512, 2708, 3708, 3708'), et un dispositif d'entraînement destiné à déplacer le support d'élément tranchant et l'élément tranchant (224, 324, 424, 2550), et dans lequel l'ensemble intégré de capteur de glucose in vivo et d'électronique corporelle (122, 322) comprend une électronique corporelle (1100) configurée pour traiter un ou plusieurs signaux indiquant des niveaux de glucose in vivo obtenus à partir du capteur de glucose in vivo (14) ;
la disposition de la surface distale de la gaine (242, 342, 442, 2512, 2708, 3708, 3708') sur une surface cutanée du sujet ;
le déplacement de la poignée (202, 302, 402, 2502, 2702, 3702) dans une première direction longitudinale pour pousser le support de dispositif, l'ensemble intégré de capteur de glucose in vivo et d'électronique corporelle (122, 322), le support d'élément tranchant, et l'élément tranchant (224, 324, 424, 2550) dans la première direction longitudinale pour insérer l'élément tranchant (224, 324, 424, 2550) et la partie du capteur de glucose in vivo (14) sous la surface cutanée, dans lequel la première direction longitudinale est d'une position proximale à distale, et dans lequel le déplacement de la poignée (202, 302, 402, 2502, 2702, 3702) comprend l'application d'une force minimale pour outrepasser l'au moins une caractéristique de retenue sollicitée (2726, 3726) pour permettre un mouvement distal de la poignée (202, 302, 402, 2502, 2702, 3702) par rapport à la gaine (242, 342, 442, 2512, 2708, 3708, 3708') ;
la libération du dispositif d'entraînement ; et
le déplacement du support d'élément tranchant dans une seconde direction longitudinale par le dispositif d'entraînement, dans lequel la seconde direction longitudinale est d'une position distale à proximale.

3. Système d'insertion et ensemble intégré de capteur de glucose in vivo et d'électronique corporelle selon la revendication 1, ou procédé selon la revendication 2, comprenant en outre un dispositif d'entraînement d'insertion destiné à déplacer le support de dispositif (230, 330, 430, 2516, 2730, 3730, 3730'), l'ensemble intégré de capteur de glucose in vivo et d'électronique corporelle (122, 322), le support d'élément tranchant, et l'élément tranchant (224, 324, 424, 2550) dans la première direction longitudinale pour insérer l'élément tranchant (224, 324, 424, 2550) et la partie du capteur de glucose in vivo (14) sous la surface cutanée.

4. Système d'insertion et ensemble intégré de capteur de glucose in vivo et d'électronique corporelle selon la revendication 1 ou 3, ou procédé selon la revendication 2 ou 3, dans lesquels l'ensemble intégré de capteur de glucose in vivo et d'électronique corporelle (122, 322) comprend un logement, et facultativement, dans lesquels l'électronique corporelle (1100) et une partie du capteur de glucose in vivo (14) sont reçues dans le logement de l'ensemble intégré de capteur de glucose in vivo et d'électronique corporelle (122, 322).

5. Système d'insertion et ensemble intégré de capteur de glucose in vivo et d'électronique corporelle selon la revendication 4, ou procédé selon la revendication 4, dans lesquels le logement de l'ensemble intégré de capteur de glucose in vivo et d'électronique corporelle (122, 322) comprend un surmoulage Macromelt.

6. Système d'insertion et ensemble intégré de capteur de glucose in vivo et d'électronique corporelle selon l'une quelconque des revendications 1 ou 3 à 5, ou procédé selon l'une quelconque des revendications 2 à 5, dans lesquels l'élément tranchant (224, 324, 424, 2550) s'étend à travers une ouverture (168) dans l'ensemble intégré de capteur de glucose in vivo et d'électronique corporelle (122, 322).

7. Système d'insertion et ensemble intégré de capteur de glucose in vivo et d'électronique corporelle selon l'une quelconque des revendications 1 ou 3 à 6, ou procédé selon l'une quelconque des revendications 2 à 6, dans lesquels l'ensemble intégré de capteur de glucose in vivo et d'électronique corporelle (122, 322) comprend une couche adhésive (218, 318, 418, 2718, 118) pour une fixation à la peau du sujet.

8. Système d'insertion et ensemble intégré de capteur de glucose in vivo et d'électronique corporelle selon l'une quelconque des revendications 1 ou 3 à 7, ou procédé selon l'une quelconque des revendications 2 à 7, dans lesquels l'ensemble intégré de capteur de glucose in vivo et d'électronique corporelle (122, 322) comprend une alimentation en puissance (82).

9. Système d'insertion et ensemble intégré de capteur de glucose in vivo et d'électronique corporelle selon l'une quelconque des revendications 1 ou 3 à 8, ou procédé selon l'une quelconque des revendications 2 à 8, dans lesquels l'électronique corporelle (1100) comprend une ou plusieurs cartes de circuit imprimé, facultativement, dans lesquels les une ou plusieurs cartes de circuit imprimé comprennent une logique de commande mise en œuvre dans un ASIC, un microprocesseur, et une mémoire.

10. Système d'insertion et ensemble intégré de capteur de glucose in vivo et d'électronique corporelle selon l'une quelconque des revendications 1 ou 3 à 9, ou procédé selon l'une quelconque des revendications 2 à 9, dans lesquels l'ensemble intégré de capteur de glucose in vivo et d'électronique corporelle (122, 322) est configuré pour communiquer des informations sur le glucose à une unité de moniteur (1200), facultativement dans lesquels l'ensemble intégré de capteur de glucose in vivo et d'électronique corporelle (122, 322) est configuré pour fournir des informations de température à l'unité de moniteur (1200).

11. Système d'insertion et ensemble intégré de capteur de glucose in vivo et d'électronique corporelle selon la revendication 10, ou procédé selon la revendication 10, dans lesquels l'ensemble intégré de capteur de glucose in vivo et d'électronique corporelle (122, 322) est configuré pour communiquer les informations sur le glucose à l'unité de moniteur (1200) lorsque l'ensemble intégré de capteur de glucose in vivo et d'électronique corporelle (122, 322) reçoit une demande de l'unité de moniteur (1200).

12. Système d'insertion et ensemble intégré de capteur de glucose in vivo et d'électronique corporelle selon l'une quelconque des revendications 1 ou 3 à 11, ou procédé selon l'une quelconque des revendications 2 à 11, comprenant en outre l'activation de l'électronique corporelle (1100) par le système d'insertion (200, 300, 400, 2500, 2700, 3700, 3700').

13. Système d'insertion et ensemble intégré de capteur de glucose in vivo et d'électronique corporelle selon l'une quelconque des revendications 1 ou 3 à 12, ou procédé selon l'une quelconque des revendications 2 à 12, dans lesquels l'avancée de l'élément tranchant (224, 324, 424, 2550) vers la position proximale comprend la rétraction de l'élément tranchant (224, 324, 424, 2550) pendant que l'ensemble intégré de capteur de glucose in vivo et d'électronique corporelle (122, 322) reste sur le sujet.

14. Système d'insertion et ensemble intégré de capteur de glucose in vivo et d'électronique corporelle selon l'une quelconque des revendications 1 ou 3 à 13, ou procédé selon l'une quelconque des revendications 2 à 13, dans lesquels l'avancée de l'élément tranchant (224, 324, 424, 2550) vers la position proximale comprend la rétraction automatique de l'élément tranchant (224, 324, 424, 2550).

15. Système d'insertion et ensemble intégré de capteur de glucose in vivo et d'électronique corporelle selon l'une quelconque des revendications 1 ou 3 à 14, ou procédé selon l'une quelconque des revendications 2 à 14, dans lesquels l'avancée de l'élément tranchant (224, 324, 424, 2550) vers la position proximale comprend la rétraction automatique de l'élément tranchant (224, 324, 424, 2550) lorsque l'élément tranchant (224, 324, 424, 2550) et le support de dispositif (230, 330, 430, 2516, 2730, 3730, 3730') avancent d'une quantité prédéterminée.

16. Système d'insertion et ensemble intégré de capteur de glucose in vivo et d'électronique corporelle selon l'une quelconque des revendications 1 ou 3 à 15, ou procédé selon l'une quelconque des revendications 2 à 15, dans lesquels le dispositif d'entraînement comprend un ressort (246, 346, 446, 2544, 2746, 3746).

17. Système d'insertion et ensemble intégré de capteur de glucose in vivo et d'électronique corporelle selon l'une quelconque des revendications 1 ou 3 à 16, ou procédé selon l'une quelconque des revendications 2 à 16, dans lesquels le support de dispositif (230, 330, 430, 2516, 2730, 3730, 3730') comprend un ou plusieurs bras capables de mouvement (2542, 3762, 3762') pour entrer en prise avec l'ensemble intégré de capteur de glucose in vivo et d'électronique corporelle (122, 322), facultativement dans lesquels le système d'insertion (200, 300, 400, 2500, 2700, 3700, 3700') est configuré pour déplacer les un ou plusieurs bras capables de mouvement (2542, 3762, 3762') de manière radiale vers l'extérieur pour libérer l'ensemble intégré de capteur de glucose in vivo et d'électronique corporelle (122, 322) du support de dispositif (230, 330, 430, 2516, 2730, 3730, 3730').

18. Système d'insertion et ensemble intégré de capteur de glucose in vivo et d'électronique corporelle selon la revendication 17, ou procédé selon la revendication 17, dans lesquels les un ou plusieurs bras capables de mouvement (2542, 3762, 3762') comprennent trois bras.

19. Système d'insertion et ensemble intégré de capteur de glucose in vivo et d'électronique corporelle selon la revendication 17 ou la revendication 18 lorsqu'elle est dépendante de la revendication 4, ou procédé selon la revendication 17 ou 18 lorsqu'elle est dépendante de la revendication 4, dans lesquels chacun des bras de préhension comprennent un bossage d'entrée en prise configuré pour entrer en prise avec un évidement correspondant (3766, 3766'), les évidements (3766, 3766') fournis sur des parois latérales du logement de l'ensemble intégré de capteur de glucose in vivo et d'électronique corporelle (122, 322).

20. Système d'insertion et ensemble intégré de capteur de glucose in vivo et d'électronique corporelle selon l'une quelconque des revendications 1 ou 3 à 19, ou procédé selon l'une quelconque des revendications 2 à 19, comprenant en outre un capuchon distal amovible (204, 304, 404, 2504, 2704, 3704) fixé de manière détachable à la poignée (202, 302, 402, 2502, 2702, 3702), facultativement dans lesquels le capuchon distal amovible (204, 304, 404, 2504, 2704, 3704) est fixé à la poignée (202, 302, 402, 2502, 2702, 3702) par des fils (210, 310, 311, 410, 411, 2506, 2706, 3706).

21. Système d'insertion et ensemble intégré de capteur de glucose in vivo et d'électronique corporelle selon l'une quelconque des revendications 1 ou 3 à 20, ou procédé selon l'une quelconque des revendications 2 à 20, dans lesquels l'élément tranchant (224, 324, 424, 2550) s'étend de manière longitudinale au sein du système d'insertion (200, 300, 400, 2500, 2700, 3700, 3700') et la partie du capteur de glucose in vivo (14) qui est insérée sous la surface cutanée est sensiblement alignée avec un axe longitudinal du système d'insertion (200, 300, 400, 2500, 2700, 3700, 3700') et sensiblement perpendiculaire à la surface cutanée avant l'insertion.

22. Système d'insertion et ensemble intégré de capteur de glucose in vivo et d'électronique corporelle selon l'une quelconque des revendications 1 ou 3 à 21, ou procédé selon l'une quelconque des revendications 2 à 21, dans lesquels le capteur de glucose in vivo (14) est configuré pour plier de sorte qu'il est positionné dans deux plans formant une intersection, sensiblement perpendiculaires, et dans lesquels la partie du capteur de glucose in vivo (14) qui est insérée sous la surface cutanée est alignée sensiblement verticalement avec un axe longitudinal du système d'insertion (200, 300, 400, 2500, 2700, 3700, 3700') et sensiblement perpendiculaire à la surface cutanée avant l'insertion, et dans lesquels une partie de contact est orientée dans une configuration sensiblement horizontale et en contact électrique avec l'électronique corporelle (1100).

23. Système d'insertion et ensemble intégré de capteur de glucose in vivo et d'électronique corporelle selon l'une quelconque des revendications 1 ou 3 à 22, ou procédé selon l'une quelconque des revendications 2 à 22, dans lesquels la caractéristique de retenue sollicitée (2726, 3726) est sollicitée contre la poignée (202, 302, 402, 2502, 2702, 3702).
